(19) Europäisches Patentamt / European Patent Office / Office européen des brevets

(11) **EP 1 613 420 B1**

(12) **EUROPÄISCHE PATENTSCHRIFT**

(45) Veröffentlichungstag und Bekanntmachung des Hinweises auf die Patenterteilung:
**13.05.2015 Patentblatt 2015/20**

(21) Anmeldenummer: **04722855.6**

(22) Anmeldetag: **24.03.2004**

(51) Int Cl.:
***G01N 33/543*** $^{(2006.01)}$ ***B01J 20/32*** $^{(2006.01)}$

(86) Internationale Anmeldenummer:
**PCT/EP2004/003138**

(87) Internationale Veröffentlichungsnummer:
**WO 2004/085046 (07.10.2004 Gazette 2004/41)**

(54) **VERFAHREN ZUR SELEKTIVEN BINDUNG EINES SUBSTRATS AN SORBENTIEN DURCH MINDESTENS BIVALENTE BINDUNG**

METHOD FOR SELECTIVELY BINDING A SUBSTRATE TO SORBENTS BY WAY OF AT LEAST BIVALENT BONDS

PROCEDE DE LIAISON SELECTIVE D'UN SUBSTRAT A DES SORBANTS A L'AIDE DE LIAISON AU MOINS BIVALENTES

(84) Benannte Vertragsstaaten:
**AT BE BG CH CY CZ DE DK EE ES FI FR GB GR HU IE IT LI LU MC NL PL PT RO SE SI SK TR**
Benannte Erstreckungsstaaten:
**LV**

(30) Priorität: **25.03.2003 DE 10313324**
**15.12.2003 DE 10358696**

(43) Veröffentlichungstag der Anmeldung:
**11.01.2006 Patentblatt 2006/02**

(60) Teilanmeldung:
**09004411.6**

(73) Patentinhaber: **InstrAction GmbH**
**68199 Mannheim (DE)**

(72) Erfinder: **GOTTSCHALL, Klaus**
**68542 Heddesheim (DE)**

(74) Vertreter: **Wetzel, Fritz et al**
**Stolmár Scheele & Partner**
**Blumenstraße 17**
**80331 München (DE)**

(56) Entgegenhaltungen:
**WO-A-93/19844**
**WO-A-98/59360**
**WO-A-03/074990**
**DE-A- 19 855 173**
**US-A1- 2001 050 254**

- **HÄNDEL H., ET AL.: "Application of HRMAS 1H NMR Spectroscopy to Investigate Interactions between Ligands and Synthetic Receptors" ANGEW. CHEM. INT. ED., Bd. 42, Nr. 4, 27. Januar 2003 (2003-01-27), Seiten 438-442, XP002301536**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 613 420 B1

Printed by Jouve, 75001 PARIS (FR)

**Beschreibung**

Die Erfindung betrifft ein Verfahren zur selektiven bivalenten Bindung eines Substrats mit mindestens zwei unterschiedlichen zur Bindung befähigten Gruppen an ein Sorbens. Dieses wird aus einer Kollektion von Sorbentien ermittelt, auf deren Oberfläche sich jeweils mindestens zwei unterschiedliche zur Bindung befähigte Gruppen befinden, die durch Zerlegung synthetischer oder natürlicher Substrate in Bausteine, die diese Gruppen enthalten, gewonnen werden. Das Verfahren der selektiven bivalenten Bindung eignet sich insbesondere zur Isolierung synthetischer oder auch natürlicher Wirkstoffe sowie zur Charakterisierung und Identifizierung der Funktion und Eigenschaften dieser Wirkstoffe. Das Verfahren der selektiven bivalenten Bindung kann zur Detektion von Substrat-Rezeptor-Wechselwirkungen, zum Wirkstoff-Screening, zur selektiven Trennung isomerer Verbindungen, zur selektiven Abtrennung sowie zur Reinigung von Substraten verwendet werden.

Aus der Bioaffinitätschromatographie ist bereits bekannt, an der Oberfläche eines hochmolekularen unlöslichen Trägers Substanzen chemisch zu immobilisieren, die zu bestimmten Biomolekülen eine besonders hohe Affinität besitzen. Sie vermögen dann diese zu binden bzw. zu retinieren.

Zumeist handelt es sich bei diesen auf dem Träger immobilisierten Stoffen um Biopolymere. Andererseits ist es aber auch möglich, auf der Oberfläche niedermolekulare Stoffe zu verankern, mit denen die Bindung beziehungsweise Retinierung von Biopolymeren möglich ist.

Dabei kommt es im Allgemeinen nur dann zu einer ausreichend hohen Affinität, wenn Sorbens und Substrat zueinander komplementäre Gruppen aufweisen, die miteinander in Bindung treten können. Komplementäre Gruppen sind beispiels-weise hydrophile Gruppen, die über Wasserstoffbrücken oder Dipole oder Polypole miteinander wechselwirken können, wobei die Bindung erfolgt.

Es ist bereits bekannt, dass biologische Systeme über mehrere molekulare Kontaktstellen gleichzeitig miteinander wechselwirken können (M. Whitesides et al., Angew. Chem. 1998, 110, 2908 - 2953).

Des Weiteren sind aus der WO 00/32649 Polymere als Sorbentien zur Abtrennung von Substraten sowie Verfahren zur Abtrennung von Substraten mit Hilfe dieser Sorbentien bekannt. Hierbei wird die Abtrennung über mindestens zwei verschiedene Arten von Wechselwirkungen ermöglicht. Bei der zur Bindung befähigten Gruppe des Sorbens, die als Rezeptor wirkt, kann es sich dabei um eine einzige Gattung von Gruppen, jedoch auch um zwei oder mehr verschiedene Gattungen von Gruppen handeln.

Ferner werden in den Patentschriften WO 00/32648, WO 01/38009 sowie WO 00/78825 Sorbens/Substrat-Wechselwirkungen offenbart, die für eine mindestens bivalente Bindung gute Voraussetzungen liefern.

Bei diesen Verfahren muss für die gezielte Bindung eines Substrats auch das dafür geeignete Biopolymere bekannt und herstellbar sein, wenn es als Teil des Sorbens verwendet werden soll. Sollen umgekehrt Biopolymere auf dem Sorbens mittels niedermolekularer Stoffe gebunden werden, so müssen letztere gleichfalls bekannt und ohne Veränderung der Bindungseigenschaften auf dem Träger immobilisierbar sein.

Bekannt ist auch ein Verfahren zur Bereitstellung von synthetischen Gruppen auf einer polymeren Verbindung zur Bindung von biologisch oder pharmakologisch aktiven Substanzen. Dabei werden Templat-Moleküle, welche biologisch oder pharmakologisch wirksame Substanzen sind, an der polymeren Verbindung fixiert. Nach Ankopplung reaktiver funktioneller Gruppen an die polymere Verbindung zur Bindung von Substraten werden die Templat-Moleküle wieder gelöst (WO 00/13016).

Ferner ist auch ein Verfahren zur selektiven Abtrennung einer ausgewählten organischen Verbindung bekannt. Dabei werden auf der Oberfläche eines Trägers Gruppen aufgebracht, die zu denen der abzutrennenden Verbindung komplementär sind. Vorzugsweise handelt es sich bei der abzutrennenden Verbindung um Makromoleküle, die ionisierbare Gruppen besitzen. Die bindenden Gruppen auf der Oberfläche des Trägers sind zu denen des Makromoleküls invers geladen. Auf dem Sorbens befindet sich allerdings nur eine Art von Gruppen, über die die Bindung erfolgt (WO 93/19844).

Weiterhin offenbart auch die US 2002/0155509 A1 ein Verfahren, das letztendlich zur selektiven Abtrennung eines Substrats aus einem Substratgemisch verwendet werden kann. Dabei wird das Substratgemisch mit verschiedenen Sorbentien und Elutionsmitteln in Kontakt gebracht. Durch Desorptionsspektrometrie kann dann festgestellt werden, ob und wie stark unter den gewählten Sorbens/Elutionsmittel-Kombinationen Substrate an die Sorbentien gebunden wurden. Sorbentien und Elutionsmittel können solange variiert werden, bis eine geeignete Sor-bens/Elutionsmittel-Kombination gefunden wird, die die selektive Abtrennung eines Substrats erlaubt (Die Begriffe Sorbens und Substrat werden dabei abweichend von der in der US 2002/0155509 A1 verwendeten Definition in der Definition verwendet, die der vorliegenden Patentanmeldung zugrunde liegt und weiter unten ausgeführt ist).

Auch ist bereits bekannt, polare Gruppen zusammen mit langkettigen Alkylresten auf der Oberfläche des Trägers zu immobilisieren, wobei Sorbentien mit mindestens zwei unterschiedlichen zur Bindung befähigten Gruppen entstehen. Hierbei werden in einer ersten Reaktionsstufe Chlorsilane, die mit einem vorzugsweise mittel- oder langkettigen Alkylrest substituiert sind, beispielsweise einem $C_8$-oder $C_{18}$-Rest, mit OH-Gruppen der Oberfläche des Trägers, beispielsweise Silanolgruppen des Kieselgels, umgesetzt, wobei besagte Alkylreste auf der Oberfläche des Trägers

immobilisiert werden. Im zweiten Schritt wird dann die Oberfläche des Trägers mit Trimethoxy- oder Triethoxysilanen umgesetzt, dem sich ein Hydrolyseschritt unter Abspaltung von Alkohol unter Bildung einer Silanolgruppe anschließt. Weiterhin ist es auch möglich, Siliciumverbindungen wie Alkyltrialkoxysilane, wie beispielsweise Octadecyltrimethoxysilan, mit OH-Gruppen der Oberfläche des Trägers zu reagieren, wobei zunächst der Alkylrest immobilisiert wird. Nicht reagierte Alkoxyreste können dann unter Bildung von Silanolgruppen hydrolisiert werden, wobei die zweite, zur Bindung befähigte Gruppe generiert wird. Diese Sorbentien sollen insbesonders zur Bindung von Substraten aus wässerigen Lösungen einsetzbar sein (Column Watch, LC*GC Europe, December 2002, Seite 780 - 786).

Falls Substrate mit noch unbekannter Struktur und/oder Bindungseigenschaften mittels Sorbentien abgetrennt werden sollen, erlauben es diese beim Stand der Technik beschriebenen Verfahren im Allgemeinen nicht, gezielt voraus zu sagen, ob und wie gut ein bestimmtes Sorbens zur selektiven Bindung des Substrats geeignet ist oder nicht. Hier muss dann in zumeist aufwändigen Versuchen untersucht werden, ob bekannte Sorbentien zur selektiven Bindung des besagten Substrats geeignet sind oder nicht. Die Auffindung eines geeigneten Sorbens ist dann eher zufällig.

Der Erfindung lag somit die Aufgabe zugrunde, ein Verfahren zur Herstellung eines Sorbens zur Verfügung zu stellen, mit dem ein Substrat oder auch verschiedene Substrate selektiv durch bivalente Bindung gebunden werden können.

Diese Aufgaben konnten mit Hilfe mindestens eines Sorbens gelöst werden, das ein auf einen Träger aufgebrachtes, nicht-kovalent gebundenes Polymer aufweist, welches mit mindestens zwei unterschiedlichen zur Bindung befähigten Gruppen versehen ist, die mit mindestens zwei Gruppen am Substrat komplementär bivalent in Wechselwirkung treten können. Hierdurch tritt gegenüber der monovalenten Wechselwirkung, die wenig bis nicht-selektiv ist, eine Verstärkung auf. In der Folge wird die Zielverbindung um ein Vielfaches stärker vom Sorbens zurückgehalten als nur monovalent bindende Konkurrenten, womit gegenüber diesen Konkurrenten die selektive Bindung erreicht wird. Gegenüber anderen polyvalenten konkurrierenden Substraten kann die selektive Bindung durch ein optimiertes Sorbens erreicht werden, dessen dazu notwendige Eigenschaften an Hand einer Kollektion von Sorbentien ermittelt werden können.

Gegenstand der Erfindung ist somit ein Verfahren zur selektiven bivalenten Bindung eines synthetischen oder natürlichen Substrats mit mindestens zwei unterschiedlichen zur Bindung befähigten Gruppen an ein Sorbens, welches einen festen Träger und mindestens zwei unterschiedliche zur Bindung mit dem synthetischen oder natürlichen Substrat befähigte Gruppen umfasst, wobei das Verfahren die Stufen (i) bis (iv) umfasst:

(i) Ermittlung von mindestens zwei zur Bindung mit einem Sorbens befähigten Gruppen aus einem synthetischen oder natürlichen Substrat,

(ia) Bindung eines underivatisierten Polymers über nicht-kovalente Wechselwirkungen an den Träger,

(ii) Einführen jeweils mindestens zweier unterschiedlicher zur Bindung mit dem synthetischen oder natürlichen Substrat befähigten Gruppen über mindestens zwei gleiche oder unterschiedliche funktionelle Gruppen des Polymers in dieses Polymer unter Bildung mindestens eines Sorbens, wobei es sich bei den Gruppen um Gruppen handelt, die komplementär zu den in Stufe (i) ermittelten Gruppen sind, wobei ein mit mindestens zwei unterschiedlichen zur Bindung befähigten Gruppen derivatisiertes Polymer entsteht, und die jeweils mindestens zwei unterschiedlichen Gruppen zur Bindung befähigten Gruppen kovalent gebunden am Polymer vorliegen, wobei

> (a) die Gruppen derart ermittelt werden, dass die Beiträge der Gibbs-Energien der einzelnen Gruppen für die nicht-kovalente Bindung mit dem Substrat einen negativen Wert der Gibbs-Energie $\Delta G$ ergeben, derart, dass eine Bindungsverstärkung eintritt, die sich in einer verbesserten Trennselektivität $\alpha$, mit der das selektiv zu bindende Substrat mit den mindestens zwei unterschiedlichen zur Bindung an das mindestens eine Sorbens befähigter Gruppen unter Verwendung des mindestens einen Sorbens aus einem Substratgemisch abgetrennt wird, größer als 1,4 auswirkt,

(iii) Inkontaktbringen des Substrats mit dem Sorbens der Stufe (ii),

(iv) Prüfung der Bindungsstärke des Substrats an das Sorbens der Stufe (iii).

Die Erfindung erlaubt es somit, die Bindung zwischen Sorbentien und Substraten gezielt zu verstärken oder auch gezielt zu lockern, wodurch die Selektivität der Bindung eines Sorbens gegenüber einem Substrat, das aus einem Substratgemisch abgetrennt werden soll, auch gezielt verbessert werden kann.

Demzufolge liegt der Erfindung ein neues Abtrennungsprinzip für ein Substrat aus einem Substratgemisch zugrunde, das sich grundlegend von den Abtrennungsprinzipien der Verfahren des Standes des Technik unterscheidet, weil es die erfolgversprechende Trennselektivität für ein beliebiges zu trennendes Substratpaar entwirft und realisiert.

Das Abtrennungsprinzip der vorliegenden Erfindung beruht auf der Vorhersage, der quantifizierbaren Abschätzung oder auf der Messung der Stärke der durch Wechselwirkung zwischen jeweils mindestens zwei unterschiedlichen zur Bindung befähigten Gruppen aus Sorbens und Substrat gebildeten nicht-kovalenten Bindung. Die Abtrennungsprinzipien der Verfahren des Standes der Technik beruhen darauf, dass die Abtrennung durch grob in die Kategorien polar/unpolar bzw. hydrophil/hydrophob eingeteilte empirische Methoden erfolgt und damit zufallsabhängig ist. Dies wird auch durch den bislang häufig nicht ausreichenden Trennerfolg belegt.

Erfindungsgemäß sind die Gruppen in Stufe (ii) zu den Gruppen der Stufe (i) komplementär.

Im Sinne der Erfindung fallen unter die Bezeichnung Substrat alle Stoffe natürlichen oder synthetischen Ursprungs, die selektiv gebunden werden können. Vorzugsweise sind dies Wirkstoffe, also Verbindungen mit physiologischer und/oder biologischer Wirksamkeit in lebenden pflanzlichen oder tierischen Organismen. Prinzipiell sind dies alle natürlichen und synthetisch chemischen und/oder biologischen Verbindungen, die zwei oder mehr zur Bindung befähigte Gruppen aufweisen. Vorzugsweise sind dies Aminosäuren, Oligopeptide, Nukleotide, Nukleoside, Proteine, Glukoproteine, Antigene, Antigen-Determinanten, Antikörper, Kohlenhydrate, Enzyme, Co-Enzyme, Fermente, Hormone, Alkaloide, Glykoside, Steroide, Vitamine, Metabolite, Viren, Mikroorganismen, Inhaltstoffe pflanzlicher und tierischer Gewebe, Zellen, Zellfragmente, Zellkompartimente, Zellaufschlüsse, Lectine, Flavyliumverbindungen, Flavone und Isoflavone, sowie synthetische Wirkstoffe, wie Pharmazeutika und Pflanzenschutzmittel.

Sind die zu bindenden Wirkstoffe niedermolekular, so werden sie in der Literatur auch häufig als Liganden bezeichnet. Bindende Stoffe, die proteinartig und hochmolekular sind, werden häufig als Rezeptor bezeichnet.

Unter den Substratbegriff fallen auch Vorstufen, die sich gegebenenfalls nach weiterer Modifikation als Wirkstoff eignen können. Solche potenziellen Wirkstoffe werden oft als Hits oder Leads bezeichnet, wenn sie von den für ihre Bestimmung verwendeten screening verfahren abgeleitet sind, oder als Scaffolds, Needles oder Pharmakophore, wenn sie von Strukturmerkmalen abgeleitet sind.

Unter den Begriff Substrat fallen außerdem Wertstoffe, deren Isolierung, Entfernung oder Gewinnung aus Gemischen von wirtschaftlichem Nutzen sein kann. Hierzu zählen auch niedrig konzentrierte Wertstoffe und Nebenprodukte, z. B. aus Prozess- oder Abfallströmen. Die Wertstoffe können auch organisch sein, wie Peptide, oder Stoffwechselprodukte aus Körperflüssigkeiten.

Unter Träger sind Materialien zu verstehen, die als Unterlage oder Gerüst für die bindenden Gruppen dienen. Beim Aufbringen dieser Gruppen auf den Träger wird das Sorbens gebildet. Für chromatographische Anwendungen wird das Sorbens auch als stationäre Phase bezeichnet.

Der Begriff Sorbens umfasst jede Kombination aus Träger und mindestens zwei unterschiedliche zur Bindung mit einem Substrat befähigten Gruppen.

Der Begriff Baustein bezeichnet Teile bzw. Fragmente von Substraten, vorzugsweise Wirkstoffe, die jeweils mindestens eine zur Bindung befähigte Gruppe aufweisen. Beispiele für derartige Bausteine sind Epitope. Der Begriff Baustein kann auch identisch mit dem Begriff zur Bindung befähigte Gruppe sein. Die räumliche Anordnung der Bausteine innerhalb eines Substrats wird im Folgenden öfters als Bindungsstelle bezeichnet. Beispielsweise ist Histidin ein Baustein, der als zur Bindung befähigte Gruppe einen Imidazol-Rest trägt, der wiederum Amidin- oder Imin-Gruppen als zur Bindung befähigte Gruppen enthält.

Der Begriff Epitop bezeichnet molekulare Bereiche von Substraten. Beispielsweise wird mit dem Begriff Epitop ein molekularer Bereich eines Antigens bezeichnet, der einen Antikörper zu binden vermag. Solche Bindungsstellen eines Antikörpers auf einem Antigen werden auch als Antigen-Determinante bezeichnet.

Der Begriff (unterschiedliche) zur Bindung befähigte Gruppe umfasst alle Gruppen, die in der Lage sind, mit dem Sorbens und/oder Substrat über kovalente oder nicht-kovalente Wechselwirkungen zu binden. In der angelsächsischen Literatur wird dieser Begriff auch mit binding site residue bezeichnet. Im Übrigen sind dies alle Verbindungen oder die Reste von Verbindungen, die in der Literatur als geeignet zur Ausbildung nicht-kovalenter Bindungen beschrieben werden. Der Be-griff nicht-kovalente Bindung wird weiter unten erläutert.

Zur Bindung befähigte Gruppen sind vorzugsweise Hydroxyl-, Carboxyl-, Amid-, Amino-, i-Butyl-, Phenyl-, Nitrophenyl-, Naphthyl-, aber auch Diol-, Hydroxyphenyl-, Carbonyl, Imin, Alkylen, Alkinyl, Indoyl- und Imidazolylreste. Eine zur Bindung befähigte Gruppe kann somit auch mindestens eine funktionelle Gruppe enthalten. Die zur Bindung befähigten Gruppen sind aber nicht auf funktionelle Gruppen begrenzt.

Eine zur Bindung befähigte Gruppe kann auch mehr als eine Form der energetischen Wechselwirkung ausüben, d. h. sie kann mehr als eine Art von nicht-kovalenter Bindung eingehen. Beispielsweise ist der Indolrest grundsätzlich in der Lage, gleichzeitig mit geeigneten zu bindenden Substanzen ionische, van der Waals-, π-π- und dispersive Wechselwirkungen auszuüben. Dem Indenrest fehlt hingegen die ionische Wechselwirkungsfähigkeit, und die dispersive Wechselwirkung ist schwächer ausgeprägt.

Die einzelnen Beiträge zur Bindung sind dabei auch vom Lösungsmittel abhängig. Sie können durch die Wahl der Lösungsmittelzusammensetzung, des pH-Wertes und der Temperatur gezielt beeinflusst werden. Im Allgemeinen sind die van der Waals-Wechselwirkungen in organischen Lösungsmitteln weniger ausgeprägt als in wässrigen Lösungsmittelgemischen. Demgegenüber werden die in aprotischen Lösungsmitteln dominanten Wasserstoffbrücken-Wechselwirkungen mit zunehmendem Wassergehalt in der Regel stark abgeschwächt.

Der Begriff unterschiedlich bedeutet, dass die Gruppen entweder eine unterschiedliche elementare Zusammensetzung besitzen, oder dass bei gleicher elementarer Zusammensetzung die Elemente in den Gruppen unterschiedlich verknüpft oder die Gruppen unterschiedlich chemisch gebunden sind. Die Unterschiedlichkeit bezüglich mindestens zweier zur Bindung befähigter Gruppen schließt auch die sterische Anordnung gegenüber einem zu bindenden Stoff ein. Eine diesbezügliche Anordnung betrifft beispielsweise die Unterscheidung von Stereoisomeren, insbesondere Diastereomeren und Enantiomeren. Beispielsweise sind cisständige Hydroxylgruppen unterschiedlich zu trans-ständigen oder Hydroxylgruppen einer R-Form unterschiedlich zu denen einer S-Form. Diese Unterschiede können durch physikalische Verfahren detektiert werden, beispielsweise durch NMR-Spektroskopie, da solche Gruppen magnetisch nichtäquivalent sind und im NMR-Spektrum unterschiedliche Resonanzsignale ergeben. Die Detektion kann auch durch Röntgenstrukturanalyse erfolgen. Auch zeichnen sich solche Gruppen dadurch aus, dass sie eine unterschiedliche Reaktivität gegenüber angreifenden Reagenzien besitzen können.

Unterschiedliche zur Bindung befähigte Gruppen sind somit insbesondere solche Gruppen, die gegenüber der zu bindenden Substanz (Substrat) jeweils unterschiedliche Beiträge zur Wechselwirkungs-Energie beisteuern. Diese Wechselwirkungsenergie wird auch als Wechselwirkungs-Gibbs Energie $\Delta G$ bezeichnet. Solche Gruppen können in ihrer Konstitution, Konfiguration und Konformation durchaus gleich sein, aber in ihrem Wechselwirkungsanteil unterschiedlich. Beispielsweise können Carboxylgruppen in Glutaminsäurederivaten einen unterschiedlichen Wechselwirkungsanteil aufweisen. Auch können unterschiedlich gebundene Rhamnose-Reste einen unterschiedlichen Wechselwirkungsanteil aufweisen, der beispiels-weise zur Trennung von Naringin und Rutin ausgenützt werden kann.

Unterschiedlichen Beiträge zur Wechselwirkungs-Gibbs Energie $\Delta G$ können jeweils wiederum unterschiedlich große Enthalpie- und Entropiekomponenten aufweisen. So ist es denkbar, dass zwei ionische Wechselwirkungen der im zu bindenden Stoff enthaltenen Carboxylgruppen zwar bezüglich der Enthalpie-$\Delta H$-Wechselwirkung nahezu gleiche Beiträge beisteuern, die zweite Bindungsstelle jedoch einen relativ größeren negativen Entropiebeitrag $\Delta S$ aufweist.

Umgekehrt kommt es auch vor, dass in einem Substrat mindestens zwei zur Bindung befähigte Gruppen unmittelbar nebeneinander stehen, die chemisch gleich oder äquivalent sind. Ihre Beiträge zur Wechselwirkung unterscheiden sich gegebenenfalls nur graduell oder sind innerhalb der Messgenauigkeit nicht mehr unterscheidbar. Das stöchiometrische Verhältnis solcher Gruppen untereinander oder in Hinsicht auf weitere zur Bindung befähigte Gruppen wird bei der Herstellung des Sorbens durch den Derivatisierungsgrad berücksichtigt. Dieser Derivatisierungsgrad ist für Lösungen oder Suspensionen des Sorbens auch das Maß für Konzentrationsangaben.

Ein Beispiel für eine Anhäufung gleicher oder energetisch angenähert äquivalenter zur Bindung befähigter Gruppen stellen die Steroidrezeptoren dar. Diese enthalten für den Bindungskontakt zu Estradiol oder Progesteron bis zu sieben Leucinreste, die mit ihren Alkylgruppen den Liganden unpolar binden. Hinzu kommen noch bis zu drei polare Bindungsstellen, die aus Arginin, Glutamin(säure) und Histidin bestehen. Erfindungsgemäß können diese natürlichen Rezeptoren in vereinfachter Weise simuliert werden, indem man im geeigneten Konzentrationsverhältnis i-Pentylreste aus Methylvaleriansäure und polare Gruppen wie Bernsteinsäureamid sowie basische Gruppen wie Amin oder Imidazol einführt.

Derartige Sorbentien vermögen in brauchbarer Weise nicht nur das Zielmolekül Estradiol stark zu binden, sondern auch eine Reihe von synthetischen und natürli-chen Stoffen, die im physiologischen Test und in vivo estrogenartige Wirkung entfalten. Hierzu gehören beispielsweise Diethylstilbestrol sowie Genistein.

Das Sorbens wird dabei vorzugsweise als synthetischer polymerer Rezeptor mit derartigen Wirkstoffen kalibriert, aber auch mit dazu strukturell verwandten, jedoch unwirksamen Substanzen, wie Tamoxifen, Testosteron oder Catechin. Der praktische Nutzen ist gegeben, wenn die am natürlichen Rezeptor gut bindenden Stoffe auch am Sorbens starke Bindung zeigen, im Gegensatz zu Stoffen, die am Vorbild bereits schwach oder unspezifisch binden. Bei der Strukturoptimierung wird neben dem Verhältnis der zur Bindung befähigten Gruppen noch der Vernetzungsgrad eingestellt, der die Größe und räumliche Beschaffenheit der Bindungsstellen steuert.

Derartige Sorbentien binden aus gelösten Substanzgemischen überwiegend oder sogar ausschließlich solche Substanzen, die auch am biologischen Protein-Vorbild stark gebunden werden. Somit können aus Stoffgemischen natürlicher oder synthetischer Herkunft auf schnelle und einfache Weise potenzielle Wirkstoffe in reiner Form isoliert werden.

Ein wichtiger Aspekt der Erfindung ist die weitgehend freie Lösungsmittelwahl bei dem erfindungsgemäßen Verfahren bzw. Anwendung. Die Rangfolge und die Größenordnungen der Bindungsenergie-Unterschiede zwischen den stark und den schwach bindenden Substanzen bleibt überraschenderweise weitgehend unverändert, wenn man zum wässrigen Eluens größere Mengen Alkohol und zusätzlich Säuren oder Puffer zugibt. Der Zusatz von Methanol lockert vorzugsweise die Bindung für alle bei der Kalibrierung verwendeten Substanzen erheblich, ohne dass die Aufteilung in die Gruppen der stark und schwach bindende Stoffe beeinträchtigt wird. Die Folge ist eine erheblich frühere Elution unter Chromatographiebedingungen. Somit lassen sich die interessierenden Substanzen in einer vertretbaren Zeit testen oder isolieren, da durch den Zusatz von organischen Lösungsmitteln die Bindungskonstanten im Verhältnis zu reinem Wasser oder physiologischem Puffer um Zehnerpotenzen verringert werden.

Der Begriff nicht-kovalente Bindung bedeutet, dass die zur Bindung befähigten Gruppen vorzugsweise über Ionenpaare, über Wasserstoffbrückenbindungen, über Dipol-Dipol-Wechselwirkungen, über Charge-Transfer-Wechselwirkungen, über π-π-Wechselwirkungen, über Kation-π-Elektronen-Wechselwirkungen, über van der Waals-Wechselwirkungen und disperse Wechselwirkungen, über hydropobe (lipophile) Wechselwirkungen, über Komplexbildung, vorzugsweise Komplexbildung von Übergangsmetallkationen, sowie die Kombinationen dieser Wechselwirkungen einander zu binden vermögen.

Unter dem Begriff komplementär wird verstanden, dass nur solche Gruppen miteinander in Bindung treten können, die zueinander passen. Die die Bindung verursachende Wechselwirkung muss dabei energetisch günstig sein. Je ausgeprägter die nicht-kovalente Bindung besagter Gruppen miteinander ist, desto stärker wird das Substrat an das mindestens eine Sorbens gebunden. Dabei ist es auch möglich, dass zu einer Gruppe mehrere Gruppen komplementär sein können. Beispielsweise können zur Hydroxyl-Gruppe die Carboxyl-, die Amin- und die Amid-Gruppe komplementär sein.

Der Begriff komplementäre Gruppen beinhaltet auch, dass derartige Gruppen durch Gruppen ersetzt werden können, die den komplementären Gruppen strukturell ähnlich oder mit diesen strukturell verwandt sind. Beispielsweise ist es möglich, in einer nicht-kovalenten Bindung, die auf der π-π-Wechselwirkung beruht, einen Naphthyl-Rest durch einen Anthracen-Rest zu ersetzen, wodurch der Beitrag des Aromaten zur Bindungsstärke der nicht-kovalenten Bindung weiter moduliert bzw. gesteigert werden kann. In dazu analoger Weise ist es möglich, den Beitrag eines Indol-Restes in einer dispersiven nicht-kovalenten Bindung durch Ersatz durch einen Acridin-Rest weiter zu erhöhen.

Die Stärke der Wechselwirkung zwischen komplementären Gruppen, die z. B. als Bindungskonstante gemessen und ausgedrückt werden kann, ergibt sich aus den Beiträgen der einzelnen zur Bindung befähigten Gruppen. Diese Einzelbeiträge zur Bindungskonstanten hängen nicht nur von der Art der nicht-kovalenten Wechselwirkung ab, sondern von den Abständen und der Orientierung (Winkel) der miteinander in Wechselwirkung tretenden Gruppen sowie von der Zusammensetzung des Lösungsmittels. Die einzelnen Wechselwirkungsarten unterscheiden sich energetisch erheblich, wobei die Bindung und somit die Gibbs-Energie in unterschiedlichem Maße mit dem Abstand zwischen diesen Gruppen abnimmt.

Zueinander komplementäre Gruppen zeichnen sich auch dadurch aus, dass die Beiträge der Gibbs-Energien der einzelnen Gruppen für die nicht-kovalente Bindung in einer Änderung der Gibbs-Energie $\Delta G$ resultiert, die einen (entsprechend großen) negativen Wert annimmt. Dabei werden erfindungsgemäß die Gruppen derart ausgewählt, dass die Änderung der Gibbs-Energie $\Delta G$ zu einer Bindungsverstärkung führt, derart, dass eine verbesserte Trennselektivität gegenüber den abzutrennenden Substanzen resultiert.

Eine verbesserte Trennselektivität tritt ganz generell dann ein, wenn der $\Delta G$-Wert für die Bindung zwischen den ausgewählten komplementären Gruppen des hergestellten Sorbens und dem Substrat (der Zielsubstanz) in hinreichendem Maße negativer ist (oder wird) als der $\Delta G$-Wert zwischen diesem Sorbens und einer abzutrennenden Substanz. In der Chromatographie eluiert die abzutrennende Substanz in diesem Fall früher, sie wird schwächer gebunden. Eine verbesserte Trennselektivität tritt aber auch dann ein, wenn man die abzutrennende Substanz gezielt durch Einbringung anderer komplementärer Gruppen, also auf Grund der damit verbundenen Veränderung des $\Delta G$-Werts stärker bindet als das Substrat (Zielsubstanz).

Das Ziel der Trennung aufgrund ausreichender Trennselektivität wird erfindungsgemäß immer erreicht, wenn im Sorbens-Substrat-Komplex mindestens eine komplementäre Gruppe mehr oder stärker (z. B. bei Stereoisomeren) an der Bindung mit dem Substrat beteiligt ist als im Komplex zwischen dem Sorbens und der mindestens einen abzutrennenden Substanz.

Beispiele für typische Werte für besagte Wechselwirkungs-Gibbs Energie $\Delta G$ (kJ/ mol), die abhängig vom Lösungsmittel ist, sind

- für die ionische Wechselwirkung -4 bis -6, wobei die Stärke bzw. Reichweite dieser Wechselwirkung reziprok zum Abstand abnimmt. Ein Beispiel für eine derartige Wechselwirkung ist die Wechselwirkung zwischen einer Carbonsäure und einem quartärem aminischen Stickstoff in Wasser;

- für die Ion-Quadrupolwechselwirkung -1, wobei die Stärke bzw. Reichweite zur dritten Potenz des Abstandes abnimmt. Ein Beispiel ist die Wechselwirkung zwischen quartärem Stickstoff in Ammoniumverbindungen und einer Aren-Gruppe in Wasser;

- für die dispersive Wechselwirkung (induzierte Dipole) -1,75, wobei die Reichweite bzw. Stärke mit der 6. Potenz des Abstandes abnimmt. Ein Beispiel ist die Wechselwirkung zwischen zwei Aren-Gruppen in Chloroform;

- für Wasserstoffbrücken -4 bis -6. Ein Beispiel ist die Wechselwirkung zwischen zwei Amid-Gruppen in Chloroform. In Tetrachlorkohlenstoff beträgt die Wechselwirkungsenergie zwischen solchen Gruppen etwa -10;

- für den hydrophober Effekt -2,3, wie er sich zwischen Alkan und MethylenRest in Wasser ergibt.

Werden erfindungsgemäß Hydantoine bivalent an Ammoniumgruppen in Chloroform gebunden, so werden $\Delta$G-Werte von bis zu -22 kJ/mol gemessen. Im Falle der monovalenten Bindung eines Succinimidderivates hingegen betragen die $\Delta$G-Werte durchschnittlich lediglich -9 kJ/mol. Die Differenz der beiden $\Delta$G-Werte beträgt somit ca. 13 kJ/mol, der zugehörige Wert für die Trennselektivität ist rund 200. Diese Daten lassen auf Wasserstoffbrückenbindungen schließen und auf eine entropische Verstärkung vor allem der bivalenten Wechselwirkung.

Für jede Art nicht-kovalenter Wechselwirkungen können sich in einem gegebenen Lösungsmittelsystem die abstandsabhängigen Gibbs-Energien in unterschiedlicher Weise aus einem Enthalpie- und einem Entropiebeitrag zusammensetzen.

Erfindungsgemäß werden diese Einzelbeiträge bestimmt durch die Prüfung der Bindungsstärke eines ersten Substrats, das eine, zwei, drei, ...n zur Bindung befähigte Gruppen enthält, mit z. B. einer Menge von zweiten Substraten, deren zur Bindung befähigte Gruppen so gewählt sind, dass Aussagen bezüglich einer bestimmten Art von Wechselwirkung möglich werden. So können erste Substrate verwendet werden, die vorzugsweise Amino-, Acetyl-, Benzyl-, Nitrophenyl- und Isopentylreste enthalten, sowie Zweier- und Dreierkombinationen hiervon. Die zweiten Substrate bestehen dann aus Derivaten von vorzugsweise Alanin, Asparaginsäure und Glutaminsäure. Die N-terminalen Schutzgruppen dieser Derivate sind vorzugsweise entweder aliphatisch oder aromatisch.

Die Bindungsenergien können vorzugsweise als k'-Wert aus isokratischen HPLC-Versuchen ermittelt werden. Wenn die Konzentration der zur Bindung befähigten Gruppen am ersten Substrat bekannt ist und das Phasenvolumenverhältnis zwischen der immobilisierten (stationären) Phase und der mobilen Phase, lässt sich aus dem k'-Wert die Bindungskonstante $K_A$ bestimmen und aus dieser wiederum die Änderung der Gibbs-Energie $\Delta$G. Die Enthalpieänderung $\Delta$H und die Entropieänderung $\Delta$S können z. B. mikrocalorimetrisch bestimmt werden oder durch temperaturabhängige Messung der Gleichgewichtskonstanten, die auch als van't Hoff-Plot bezeichnet wird. Anschließend ist durch Vergleich der jeweiligen Wechselwirkungsenergien zwischen ausgewählten Rezeptorvarianten und Liganden nachprüfbar, inwieweit sich Wechselwirkungsbeiträge addieren, verstärken oder abschwächen. Selbstverständlich sind die Methoden für die Bindungsbestimmung nicht auf die oben genannten beschränkt. Außerdem lassen sich alle gängigen Bestimmungsmethoden verwenden wie kompetitive Assays, Surface Plasmon Resonanz oder NMR-Titration. Die Bestimmung der Wechselwirkungsenergien kann in Form von miniaturisierten Assays und in paralleler Bestimmung erfolgen.

Unter sterisch günstigen Bedingungen addieren sich die Anteile der Gibbs-Energie für die zur Bindung befähigten Gruppen. Daraus folgt, dass sich die Anteile an der Bindungskonstanten multiplizieren. Darüber hinaus sind kooperative Effekte möglich, die zur weiteren Bindungsverstärkung beitragen. Auch unter sterisch weniger günstigen Bedingungen kann man meistens eine mindestens bivalente Bindungsverstärkung erreichen. Dies ist für die praktische Anwendung von großem Nutzen, da sich die Bindungsverstärkung bei geeigneter Wahl der zur Bindung befähigten Reste nahezu vollständig in einer verbesserten Trennselektivität gegenüber den abzutrennenden Substanzen (Begleitsubstanz/Nebenkomponenten) auswirkt.

Unter dem Begriff nicht-komplementär wird verstanden, dass Gruppen zwar miteinander in Bindung treten können, diese Gruppen jedoch schwächere Beiträge zur nicht-kovalenten Bindung liefern als es komplementäre Gruppen tun. Die Bindungsstärke zwischen nicht-komplementären Gruppen ist demzufolge schwächer ausgeprägt als die Bindung zwischen komplementären Gruppen. Zueinander nicht-komplementäre Gruppen führen im Sinne der Erfindung zu einer Lockerung der zwischen diesen Gruppen gebildeten nicht-kovalenten Bindung oder zu einer Schwächung der betreffenden gesamten Bindungsstelle, oder sie sind nichtbindend. Sie zeichnen sich vorzugsweise dadurch aus, dass die Beiträge der Gibbs-Energien der einzelnen Gruppen für die nicht-kovalente Bindung in einer Änderung der Gibbs-Energie $\Delta$G resultiert, die null ist oder einen positiven Wert annimmt.

Unter Ermittlung wird eine gezielte Auswahl verstanden, beispielsweise eine gezielte Auswahl von zur Bindung befähigten Gruppen.

Das nach diesem Verfahren hergestellte mindestens eine Sorbens kann für die Erkennung von Sorbens-Substrat-Wechselwirkungen verwendet werden. Als Methode für die Erkennung eignet sich insbesondere das erfindungsgemäße Verfahren zur selektiven bivalenten Bindung besagten Substrats an das mindestens eine besagte Sorbens. Als Maß für die Erkennung kann die Bindungsstärke herangezogen werden. Kommt es zu einer ausreichend starken Bindung zwischen Sorbens und Substrat, erhält man eine Aussage, welche Gruppen des Substrats und welche Gruppen des Sorbens einander zu binden vermögen.

Sind die Gruppen des Substrats unbekannt, so kann im Bindungsfall darauf geschlossen werden, welche zur Bindung befähigte Gruppen im Substrat an der Bindungsstelle vorliegen können.

Es ist aber auch möglich, molekulare Bereiche eines Substrats unbekannter Struktur in geeignete Bausteine, z.B. Epitope, zu zerlegen und diese Struktur oder eine dazu komplementäre Struktur durch geeignete Anordnungen der Bausteine auf dem Sorbens nachzustellen.

Die Zerlegung kann dabei sowohl nach chemischen, physikalischen oder chemisch-physikalischen Methoden erfolgen, wie beispielsweise durch chemische Abbaureaktionen oder durch Ultraschall, aber auch durch virtuelle Experimente. Für diese virtuellen Experimente können auch Computer-gestützte Methoden herangezogen werden, mit deren Hilfe Informationen über die in den Bausteinen der Substrate vorliegenden Bindungsmöglichkeiten erhalten werden können.

Diese Zerlegung geht davon aus, dass die Menge aller wechselwirkungsfähigen Bausteine und die Anzahl von zur Bindung befähigten Gruppen endlich und begrenzt ist sowie für eine konkrete Problemstellung darüber hinaus zweckentsprechend abgrenzbar ist. Aus jeder willkürlich wählbaren Teilmenge solcher Gruppen kann man beliebige Klassen von Kombinationen mit jeweils m Elementen (m = 2, 3, 4,...) herstellen. Ein Beispiel wäre die Klasse 3 mit allen möglichen Kombinationen von jeweils drei zur Bindung befähigten Gruppen aus einer Auswahl n = 5 mit beispielsweise Phenyl-, Alkyl-, Amino-, Carboxyl- und Amid-Gruppen.

In dieser Weise kann man jedes Protein in 20 Bausteine, nämlich die Aminosäuren, zerlegen, von denen wiederum n = 6 bis n = 9 zur Bindung befähigte Gruppen in erster Näherung relevant sind für die nicht-kovalente Wechselwirkung mit einem zweiten Substrat. Diese Reduktion kommt dadurch zustande, dass dieselbe oder eine gleichwertige zur Bindung befähigte Gruppe in mehreren Aminosäuren enthalten ist, wie z. B. die Hydroxyl-, Carboxyl- und Amid-Gruppe, und auch eine basische Funktion, wenn es auf graduelle Abstufungen zwischen Lysin, Arginin, Tryptophan oder Histidin nicht ankommt.

In vergleichbarer Weise kann man die 8 isomeren Ketohexosen oder die 16 stereoisomeren Aldohexosen und die daraus abgeleiteten Pyranoside und Furanoside als Bausteine einsetzen, die Oligosaccharide repräsentieren.

In diesem Sinne besteht jedes beliebige unbekannte Substrat aus einer abzählbaren Anzahl von Bausteinen, die wiederum jeweils eine definierte Anzahl zur Bindung befähigter Gruppen enthalten. Die Bausteine und die zur Bindung befähigten Gruppen entstammen dem chemischen Wissen und sind nach Art und Eigenschaften in der Regel bekannt. Dies gilt vor allem dann, wenn sie der organischen Chemie oder Komplexchemie zuzuordnen sind.

Da man zu jeder Kombination der bekannten Bausteine und zur Bindung befähigten Gruppen im Vorhinein Bibliotheken beliebigen Umfangs von dazu komplementären und identischen Sorbentien herstellen kann, kann grundsätzlich jeder Baustein aus einem molekularen Bereich oder aus einer Bindungsstelle eines Substrats unbekannter Struktur in einer solchen Sorbensbibliothek enthalten sein bzw. einbezogen werden. Gleiches gilt für die Kombinationen der zur Bindung befähigten Gruppen.

Bei der erfindungsgemäßen Vorgehensweise können auch mehrere Sorbentien, also eine Kollektion von Sorbentien, erhalten werden. Man kann nun ein bekanntes oder unbekanntes zweites Substrat, das verschieden vom ersten Substrat ist, und dessen zur Bindung befähigten Gruppen bekannt sind, in Kontakt mit dieser Kollektion von Sorbentien bringen und die Bindungsstärken bestimmen. Hierdurch erhält man eine Aussage, wie die Bausteine an der Bindungsstelle des zweiten Substrats angeordnet sind, und wie die Raumstruktur der Bindungsstelle beschaffen ist. Somit kann das neue Verfahren auch zur Strukturaufklärung verwendet werden.

Weiterhin ist das neue Verfahren zur selektiven bivalenten Bindung besagten Substrats auch für die Wirkstoffentwicklung, vorzugsweise für die Entwicklung von Arzneimitteln, außerordentlich wertvoll. Bekanntlich beruht die Wirkungsweise eines Arzneimittels darauf, dass es unter physiologischen Bedingungen an einen natürlichen Rezeptor, der beispielsweise ein Hormon oder ein Enzym sein kann, gebunden wird. Man kann nun die Bindungsstelle des natürlichen Rezeptors in vorstehend beschriebener Weise zerlegen und eine Kollektion von Sorbentien erstellen. Jedes Sorbens aus der Kollektion dieser Sorbentien enthält dann definierte Bausteine (Teile) dieser Bindungsstellen. Vorzugsweise wird dabei auch die räumliche Anordnung der Bausteine, weiter bevorzugt die räumliche Anordnung der Bausteine der gesamten Bindungsstelle, nachgestellt. Wenn man nun die Stärke der Bindung eines beliebigen Substrates, beispielsweise eines Arzneimittels, gegen jedes dieser synthetischen Rezeptorteile ermittelt, von denen nun jedes ein anderes Strukturteil des natürlichen Rezeptors darstellt, erhält man aus den Bindungsdaten eine Information, ob dieses Substrat überhaupt mit dem natürlichen Rezeptor gut in Wechselwirkung treten kann, und wenn ja, mit welchen der räumlich angeordneten Rezeptorgruppen. Durch entsprechende chemische Modifizierung kann dann das Substrat, also das zu entwickelnde Arzneimittel, solange optimiert werden, bis die maximale Bindung an den Rezeptor gegeben ist.

Das Verfahren eignet sich dazu, bisher unbekannte oder nur wegen einer bestimmten Funktion postulierte Biopolymere, vorzugsweise Proteine oder Glykoproteine, zu isolieren, und bezüglich ihrer Eigenschaften zu validieren.

In vergleichbarer Weise ist es denkbar, zu Peptiden aus Phagen-Displays, zu Oligonukleotiden oder zu anderen Matritzen eine komplementäre Sorbensstruktur zu synthetisieren, die für die Isolierung von Wirkstoffmolekülen direkt aus Gemischen eingesetzt werden kann.

Durch Darstellung der für Wirkstoffe typischen Strukturteile auf der Sorbensoberfläche ist es umgekehrt denkbar, aus Substratgemischen das jeweilig zugehörige Substrat zu binden und zu charakterisieren. Beispielsweise ist ein derartiges Substrat ein Rezeptor.

In Stufe (i) erfolgt die Auswahl von mindestens zwei unterschiedlichen Gruppen, die zur Bindung eines synthetischen oder natürlichen Substrats an ein Sorbens befähigt sind, durch Ermittlung besagter Gruppen aus einem synthetischen oder natürlichen Substrat. Die Ermittlung von mindestens zwei unterschiedlichen Gruppen, die zur Bindung eines synthetischen oder natürlichen Substrats an ein Sorbens befähigt sind, kann in jeder erdenklichen Weise erfolgen, d. h. es können beliebige Gruppen durch beliebige Methoden ausgewählt werden, sofern diese zur Bindung befähigt

sind. Die Auswahl erfolgt in einer bevorzugten Ausführungsform entsprechend der mit dem Substrat zu erwartenden nicht-kovalenten Wechselwirkungen.

In dieser Ausführungsform der Erfindung umfasst die Ermittlung nach Stufe (i) vorzugsweise das Zerlegen eines synthetischen oder natürlichen Substrats in mindestens zwei Bausteine mit mindestens zwei zur Bindung mit einem Sorbens befähigten Gruppen.

Im Rahmen der Erfindung ist es in einer Ausführungform möglich, in Stufe (i) das synthetische oder natürliche Substrat lediglich in zwei Bausteine mit jeweils einer zur Bindung befähigten Gruppe zu zerlegen, wobei in Stufe (ii) lediglich ein Sorbens erhalten wird.

Es ist aber auch möglich, das synthetische oder natürliche Substrat in drei Bausteine zu zerlegen, deren paarweise Kombination in Stufe (ii) zu drei Sorbentien führt.

Bei Zerlegung in vier Bausteine werden durch paarweise Kombination in Stufe (ii) sechs Sorbentien erhalten.

Es ist aber auch möglich, dass beim Vorliegen von drei unterschiedlichen Bausteinen neben der paarweisen Kombination in Stufe (ii) diese drei Bausteine zusammen als Triplett auf ein Sorbens aufgebracht werden können. Neben den vorstehend erwähnten drei Sorbentien erhält man dann noch ein viertes Sorbens. Analog dazu ist es auch möglich, dass beim Vorliegen von vier unterschiedlichen Bausteinen neben der paarweisen Kombination in Stufe (ii), die zu sechs Sorbentien führt, zusätzlich vier Sorbentien erhalten werden können, die jeweils drei unterschiedliche Bausteine enthalten, und ein weiteres Sorbens, das alle vier Bausteine als Quartett enthält.

Demzufolge ist die Erfindung auch dadurch gekennzeichnet, dass die Auswahl mindestens zweier zur Bindung mit einem Sorbens befähigten Gruppen aus einem synthetischen oder natürlichen Substrat in Stufe (i) zu zwei Bausteinen mit jeweils mindestens einer zur Bindung mit dem Sorbens befähigten Gruppe führt und in Stufe (ii) ein Sorbens erhalten wird; oder die Auswahl mindestens zweier zur Bindung mit einem Sorbens befähigten Gruppen aus einem synthetischen oder natürlichen Substrat in Stufe (i) zu drei Bausteinen mit jeweils mindestens einer zur Bindung mit dem Sorbens befähigten Gruppe führt und in Stufe (ii) mindestens drei Sorbentien erhalten werden; oder die Auswahl mindestens zweier zur Bindung mit einem Sorbens befähigten Gruppen aus einem synthetischen oder natürlichen Substrat in Stufe (i) zu vier Bausteinen mit jeweils mindestens einer zur Bindung mit dem Sorbens befähigten Gruppe führt und in Stufe (ii) mindestens sechs Sorbentien erhalten werden.

In ähnlicher Weise ist es auch denkbar, aus einer größeren Anzahl von i Bausteinen n Bausteine auszuwählen und daraus Multipletts aus jeweils m zur Bindung befähigter Gruppen zu kombinieren. Beispielsweise kann man aus der Menge der natürliche Aminosäuren die Bausteine Phenylalanin, Tyrosin, Isoleucin, Asparaginsäure, Asparagin, Serin, Lysin, Tryptophan und Histidin ($n = 9$) auswählen, womit die wichtigsten Arten von nicht-kovalenter Wechselwirkung abgedeckt werden können. Die Kombination von jeweils $m = 4$ unterschiedlicher, zur Bindung befähigter Gruppen aus dieser Auswahl liefert 126 unterschiedliche Varianten von Sorbentien, die auch in kombinatorischer Weise oder als Assay zu Bindungszwecken und Bindungsstudien eingesetzt werden können.

Jeder dieser m nicht-kovalenten Wechselwirkungsbeiträge liefert für jedes einzelne Sorbens einen charakteristischen Wert zur Gesamtwechselwirkung mit einer zu bindenden Substanz. Diese Einzelbeiträge jeder zur Bindung befähigten Gruppe ($m = 1$) können lösungsmittelabhängig innerhalb eines für die Anwendung zu vernachlässigenden Schwankungsbereiches für eine beliebige zu bindende Substanz experimentell ermittelt werden. Ebenso kann man die Messdaten für die Dublettwechselwirkungen mit $m = 2$, für die Triplettwechselwirkungen mit $m = 3$, usw., erhalten.

Hierdurch wird ein umfassender Satz von Energieinkrementen für die unterschiedlichen Formen und Kombinationen von nicht-kovalenten Wechselwirkungen erhalten, der dann Vorhersagen für die Bindungsstärke zwischen zwei beliebigen Substraten oder Bausteinen ermöglicht. Dabei wird auch die Tatsache genützt, dass die unterschiedlichen nicht-kovalenten Wechselwirkungen vom Lösungsmittel und vom pH-Wert abhängen. So wirken sich Wasserstoffbrückenwechselwirkungen in aprotisch unpolaren organischen Lösungsmitteln stark aus, aber in polar protischen oder Wasser kaum. Carboxylgruppen ergeben mit basischen Resten in organischen Lösungsmitteln starke Salzbrücken, in Wasser wird hingegen in der Regel eine vergleichsweise geringere entropiegetriebene Wechselwirkung festgestellt.

Diese Zusammenhänge sind anhand der Bindung von Aminosäurederivaten an unterschiedliche Sorbentien beispielhaft dargestellt. Dabei kann man wie bereits dargelegt bei bekannter Konzentration der am Sorbens angebrachten Bausteine oder zur Bindung befähigten Gruppen aus dem k'-Wert der chromatographischen Messung auf die Bindungskonstante $K_A$ schließen. Damit ist ein schnelles und parallelisierbares Verfahren verfügbar, um Bindungskonstanten von miteinander um die Bindungsstelle konkurrierenden Substraten zu erhalten, auch wenn diese in einem komplexen Gemisch vorliegen.

Aus den für die Kombinationen multivalenter Wechselwirkungen erhältlichen Werten der Bindungskonstanten und Bindungsenergien ist es in der beschriebenen Weise möglich, auf die Art und die Anzahl der zur Bindung befähigten Gruppen einer strukturell nicht bekannten zu bindenden Substanz zu schließen bzw. das Fehlen anderer Gruppen zu postulieren. So lassen sich z. B. Aussagen machen über die Anzahl von Carboxylgruppen, basischen Gruppen oder aliphatischen oder aromatischen Resten in einem gebundenen Aminosäurederivat oder Peptid.

In ähnlicher Weise kann man Aussagen machen über das strukturabhängige voraussichtliche oder mögliche Bindungsverhalten zwischen zwei Substraten mit unbekannter Struktur, sobald ihre zur Bindung befähigten Gruppen

bekannt sind. Dies kann auf Peptide oder Proteinfragmente zutreffen, wenn man lediglich die Aminosäurezusammensetzung kennt.

Es ist auch denkbar, das Bindungsverhalten zwischen zwei Substraten unbekannter Struktur vorherzusagen oder zu beschreiben, wenn sie im gewählten Lösungsmittelsystem eine stabile Raumstruktur einnehmen. Zwei Proteine oder Glykoproteine mit definierter Tertiärstruktur, die miteinander an mindestens einer Bindungsstelle in Wechselwirkung treten können, werden mit den jeweils zueinander komplementären Vertretern einer Bibliothek von Sorbentien Wechselwirkungen ähnlicher Stärke oder Rangfolge eingehen.

Ein weiterer wichtiger Anwendungsfall beschreibt die Herstellung von Sorbentien, die einen vollständigen Satz aller Kombinationen aus zur Bindung befähigten Gruppen repräsentieren, die zu einer Bindungsstelle an einem Protein oder Glykoprotein komplementär sind. Diese Bibliothek von Sorbentien wird dann getestet mit einem vollständigen Satz von Liganden, der z. B. alle Zweier-, Dreier- und Viererkombinationen genau der zur Bindung befähigten Gruppen an der Proteinbindungsstelle repräsentiert. An den Sorbentien mit der jeweils stärksten Bindung befinden sich dann diejenigen zur Bindung befähigten Gruppen, die in einem zu entwickelnden Wirkstoff vorzugsweise enthalten sein sollten. Selbstverständlich lassen sich an diesen Sorbentien auch die Proteine binden, die als Muster für die komplementären Gruppen gedient haben.

Auf analoge Weise kann man aus dem Bindungsmuster eines über Phagenassay erhaltenen cyclischen Peptids Schlüsse auf die Bindungsstelle im zugehörigen Proteintarget ziehen. Darüber hinaus ist es denkbar, durch komplementäre Abbildung eines solchen Peptids eine sorbensgestützte Matritze zur Entdeckung von neuen Wirkstoffen mit geeigneter, nämlichen diesem Peptid entsprechender Konfiguration und Konformation zu erzeugen.

Somit lässt sich das Verfahren auch zur Bindung, Charakterisierung und Validierung von unbekannten Protein-Targets und von Bindungsstellen für nichtkompetitive oder modulatorisch wirkende Wirkstoffe einsetzen. Außerdem ist es möglich, flexible und instabile Wirkstoffe wie Peptide in rigide Strukturen mit befriedigender Darreichungsmöglichkeit umzusetzen.

In allen genannten Fällen wird die Strukturprognose dadurch ermöglicht, dass die Substrate mit einer geeigneten Auswahl an Sorbentien in Kontakt gebracht werden unter Messung der Bindungsdaten. Dabei sind fehlende oder schwache Wechselwirkungen für die Ableitung einer komplementären Substratstruktur genauso wichtig wie eine starke Bindung. Wenn ein Substrat beispielsweise eine Aminogruppe enthält, wird die Bindung zum carboxylgruppenhaltigen Sorbens um einen charakteristischen Betrag größer ausfallen als die Bindung des gleichen Substrats zu einem Sorbens, das Hydroxylgruppen oder selbst Aminogruppen trägt.

Ein wesentlicher praktischer Wert dieser Vorgehensweise liegt im Ausschluss der Mehrzahl von denkbaren Möglichkeiten der Bindung, wobei zumindest eine Eingrenzung des Aufwandes auf eine weiter untersuchbare Anzahl von wahrscheinlichen Bindungskombinationen erfolgt. Das gleiche Prinzip wird beim Screening benutzt, bei der Prüfung eines Substanzgemisches auf darin enthaltene Substanzen mit vorgegebenen Strukturmerkmalen. Der große praktische Nutzen ist dabei der ohne zusätzlichen Aufwand erreichte Ausschluss der großen Mehrzahl an nicht brauchbaren Substanzen.

Vorzugsweise erfolgt die Zerlegung in Bausteine so, dass Bausteine erhalten werden, die in der Bindungsstelle des natürlichen oder synthetischen Substrats unmittelbar räumlich benachbart sind. Die räumliche Anordnung der Bindungsstelle kann dann bei Zerlegung in zwei Bausteine durch eine lineare Anordnung dieser Bausteine, bei drei Bausteinen durch ein Dreieck und bei vier Bausteinen durch einen (verzerrten) Tetraeder charakterisiert werden.

Ist besagte Bindungsstelle so gestaltet, dass in ihr vorzugsweise drei oder vier Bausteine mit mindestens jeweils einer zur Bindung befähigten Gruppe vorliegen, werden an besagter Bindungsstelle stereoisomere Substrate, wie sie etwa in racemischen Gemischen vorliegen, im Allgemeinen unterschiedlich stark gebunden.

Demzufolge können auch stereoisomere Substrate nach dem erfindungsgemäßen Verfahren mit Hilfe des mindestens einen Sorbens unterschiedlich stark gebunden werden. Diese Eigenschaft kann für die Wirkstoffentwicklung herangezogen werden, da bekanntlich stereoisomere Verbindungen unterschiedliche physiologische Wirkungen besitzen können.

Das neue Verfahren stellt somit eine wertvolle Methode zur selektiven Abtrennung einer oder mehrerer stereoisomerer Verbindungen aus einem Gemisch von stereoisomeren Verbindungen dar. Beispielsweise kann es zur Racematspaltung verwendet werden.

Als weitere stereoisomere Verbindungen, die selektiv gebunden werden können, können Diastereomere, Konformere, geometrische Isomere, wie cis- und trans-Isomere, Epimere, sowie Anomere, wie $\alpha$- und $\beta$-glykosidische Zucker, genannt werden.

Aber nicht nur stereoisomere Verbindungen können mit Hilfe des neuen Verfahrens selektiv gebunden werden, sondern auch Konstitutionsisomere, d. h. Verbindungen, die die gleiche elementare Zusammensetzung besitzen, in denen aber die Elemente relativ zueinander unterschiedlich angeordnet sind.

Denkbar ist beispielsweise die Trennung annellierter Aromaten, die bei sonst gleicher Summenformel sich durch die Art der Verknüpfung der Kohlenstoff-Ringe unterscheiden.

Beim Einführen von jeweils mindestens zwei unterschiedlichen zur Bindung mit dem Substrat befähigten Gruppen gemäß Stufe (ii) des erfindungsgemäßen Verfahrens, wie nachfolgend beschrieben, lässt es sich im Allgemeinen nicht vermeiden, dass in dem mindestens einen gebildeten Sorbens nicht nur bindende Bereiche entstehen, in denen die gewünschten mindestens zwei unterschiedlichen zur Bindung befähigten Gruppen in statistischer Verteilung nebeneinander vorliegen, sondern dass auch Bereiche entstehen, in denen im Wesentlichen nur gleiche Gruppen vorliegen, oder Bereiche, in denen diese Gruppen angereichert sind. Solche Bereiche stören jedoch bei der selektiven Abtrennung besagten Substrates nicht, da ein solcher Bereich im Substrat im Allgemeinen schwächer bindet als ein Bereich, der die mindestens zwei unterschiedlichen Gruppen enthält. Zumeist stößt ein derartiger Bereich, der im Wesentlichen nur eine zur Bindung befähigte Art von Gruppen enthält, besagtes Substrat sogar ab. Insbesondere wirkt dieser Bereich dann abstoßend, wenn sich nicht-komplementäre Gruppen gegenüberstehen.

Generell werden gegenüberliegende nicht-komplementäre Gruppen die Bindung insgesamt schwächen. Diese Wirkung tritt bereits bei bivalenten Bindungen ein. Wenn als zur Bindung befähigte Gruppen beispielsweise der Carboxyl-Rest einerseits und der Amin-Rest andererseits sowie Phenyl-Rest einerseits und Fluorenyl-Rest andererseits ausgewählt wurden, dann ist jede räumliche Anordnung energetisch relativ ungünstiger, bei der mindestens einer der polaren Reste einem unpolaren gegenübersteht. Wegen der beweglichen Anordnung der Polymerketten wird sich das am Sorbens zu bindende Substrat spontan in der Weise anlagern, dass die maximal mögliche Gibbs-Energie gewonnen wird.

Diesen Sachverhalt kann man allgemein auch so ausdrücken, dass einem Paar an zur Bindung befähigten Gruppen im Sorbens ein Paar komplementäre Gruppen gegenüberstehen muss. Eine Bindung zwischen einem Sorbens und einem Liganden erreicht ihre maximale Stärke, wenn sich alle beteiligten Gruppen jeweils paarweise bzw. multiplettweise komplementär anordnen können.

Bereits bei der bivalenten Paarung zweier Substrate wird die Richtungsabhängigkeit deutlich. Diese sterische Lenkung verstärkt sich erheblich beim Übergang zu trivalenten und tetravalenten Wechselwirkungen. Für eine hohe Ausbeute an energetisch optimalen Bindungsplätzen braucht man Polymerderivate mit besonders hoher konformativer Beweglichkeit. Dabei sind Copolymere denkbar, bei denen zwischen den gebundenen zur Wechselwirkung befähigten Gruppen Teilbereiche mit hoher konformativer Beweglichkeit eingebaut sind, z. B. Alkylketten.

Das molare Verhältnis bzw. das lokale Konzentrationsverhälnis der mindestens zwei unterschiedlichen zur Bindung befähigten Gruppen, die auf das mindestens eine Sorbens aufgebracht werden, ist für die selektive Bindung eines Substrats außerordentlich wichtig. Vorzugsweise muss jede Gruppe am zu bindenden Substrat auch am Sorbens eine zur Bindung geeignete Gruppe vorfinden.

Vorzugsweise werden daher auf das Sorbens die mindestens zwei unterschiedlichen zur Bindung befähigten Gruppen in einem molaren Verhältnis aufgebracht, wie es den strukturellen Anforderungen des zu bindenden Substrats optimal entspricht.

Vorzugsweise werden auf das Sorbens die mindestens zwei unterschiedlichen zur Bindung befähigten Gruppen, die komplementär zu den Gruppen des Substrats sind, in einem molaren Verhältnis aufgebracht, wie es auch im zu bindenden Substrat vorliegt. Die dazu vorzugsweise verwendeten präparativen Methoden werden weiter unten beschrieben.

Die synthetischen oder natürlichen Substrate der Stufe (i) können niedermolekular sein, vorzugsweise mit einem Molekulargewicht unter 1000 Da. Es kann sich dabei aber auch um Oligomere oder Polymere, vorzugsweise Biopolymere, handeln.

Das zur Bindung von vorzugsweise biologischen Substraten geeignete mindestens eine Sorbens weist vorzugsweise zur Bindung befähigte Gruppen auf, die auch für die Bindung der in der Natur vorkommenden Strukturen oder für die Bindung maßgebender Teile derartiger Strukturen verantwortlich sind, und die mit dem vorzugsweise biologischen Substrat in Wechselwirkung treten können. Die Gruppen werden im Folgenden auch als Rezeptoren oder Rezeptorgruppen bezeichnet.

Die mindestens zwei zur Bindung befähigten Gruppen sind vorzugsweise Bestandteile von Bausteinen oder Teile oder Fragmente von Substraten mit funktionellen Gruppen. Dabei sind hier insbesondere Enzym-, Aminosäure-, Peptid-, Zucker-, Aminozucker-, Zuckersäuren- sowie Oligosaccharid-Gruppen bzw. Derivate davon, sowie Nukleoside und Nukleotide zu nennen. Weitere geeignete Substrate sind Pyrimidin- und Purinbasen, wie Cytosin, Uracil, Thymin, Purin, Adenin, Guanin, Harnsäure, Hypoxanthin, 6-Purinthiol, 6-Thioguanin, Xanthin. Fragmente von Molekülen sind beispielsweise Phenyl-, Phenol- oder Indol-Reste aus Phenylalanin, Tyrosin oder Tryptophan, sowie Hydroxyl-, Carboxyl-, Amino- und Amidgruppen. Essentiell für die genannten Gruppen ist ausschließlich, dass das in der Natur vorkommende Bindungsprinzip eines Rezeptors mit einem Substrat beibehalten bzw. angenähert wird, so dass mittels des neuen Verfahrens z. B. synthetische Enzyme, bindende Domänen von Antikörpern oder sonstige physiologische Epitope, also molekulare Bereiche, fertige Hosts, Peptide, Glykopeptide, Epitope von Proteinen, Glykoproteine, sowie Oligonukleotide eingesetzt werden können.

Als Aminosäuren sind vorzugsweise zu nennen:

- Aminosäuren mit aliphatischen Resten wie z.B. Glycin, Alanin, Valin, Leucin, Isoleucin;
- Aminosäuren mit einer aliphatischen Seitenkette, die eine oder mehrere Hydroxylgruppen umfasst, wie z. B. Serin,

Threonin;

- Aminosäuren, die eine aromatische Seitenkette aufweisen, wie z. B. Phenylalanin, Tyrosin, Tryptophan;
- Aminosäuren, die basische Seitenketten umfassen, wie z. B. Lysin, Arginin, Histidin;
- Aminosäuren, die saure Seitenketten aufweisen, wie z. B. Asparaginsäure, Glutaminsäure;
- Aminosäuren, die Amidseitenketten aufweisen, wie z. B. Asparagin, Glutamin;
- Aminosäuren, die schwefelhaltige Seitenketten aufweisen, wie z. B. Cystein, Methionin;
- Modifizierte Aminosäuren, wie z.B. Hydroxyprolin, $\gamma$-Carboxylglutamat, O-Phosphoserin;
- Derivate der genannten oder von gegebenenfalls weiteren Aminosäuren, beispielsweise an der oder gegebenfalls den Carboxylgruppen mit beispielsweise Alkyl- oder Arylresten, die gegebenenfalls geeignet substituiert sein können, veresterte Aminosäuren.

Statt der Aminosäuren ist auch die Verwendung eines oder mehrerer Di- oder Oligopeptide denkbar, beispielsweise können beta-, gamma- oder sonstige strukturisomeren Aminosäuren und davon abgeleitete Peptide wie z.B. Depsipeptide verwendet werden.

Dabei ist es auch möglich, dass mit einem Baustein mindestens zwei unterschiedliche, zur Bindung befähigte Gruppen gleichzeitig eingeführt werden.

Demzufolge ist das erfindungsgemäße Verfahren auch dadurch gekennzeichnet, dass ein Baustein mindestens zwei unterschiedliche zur Bindung befähigte Gruppen trägt.

Wenn mehr als vier zur Bindung befähigte Gruppen im selben Sorbens angebracht werde sollen, dann besteht eine bevorzugte Ausführung darin, jeweils mindestens zwei dieser zur Bindung befähigten Gruppen gemeinsam mittels eines schon fertigen Bausteins in definierter räumlicher Anordnung einzuführen. Dabei werden bevorzugt diejenigen zur Bindung befähigten Gruppen in einem Baustein angebracht, die bereits im ersten Substrat in gleicher Weise benachbart waren.

Selbstverständlich ist es denkbar, mehrere solcher mindestens bivalenten Bausteine nacheinander oder gleichzeitig in ein Sorbens einzuführen und zudem mit monovalenten Bausteinen zu kombinieren.

Ein einfaches Beispiel für einen bivalenten Baustein ist Fluorenylmethoxycarbonyl-Glutamin, auch als Fmoc-Glutamin bezeichnet. Hier wird die Carboxylgruppe zur Bindung an das Sorbens verwendet, wobei der Amidrest zur polaren Bindung eines Liganden fähig ist, und die Fluorenylgruppe zur $\pi$-$\pi$-Wechselwirkung. In ähnlicher Weise lassen sich Oligopeptide einsetzen, aber auch kammförmige Oligomerderivate.

Vorzugsweise erfolgt die Bindung besagter Substrate an das mindestens eine Sorbens über Reste oder Gruppen aus Aminosäuren, Zuckern, Nukleotiden und Nukleosiden sowie Pyrimidin- und Purinbasen, welche sich auf dem Sorbens befinden.

Demzufolge ist die Erfindung auch dadurch gekennzeichnet, dass die mindestens zwei unterschiedlichen zur Bindung befähigten Gruppen des mindestens einen Sorbens ausgewählt werden unter Gruppen, die Bestandteil von Aminosäuren, Zucker, Nukleotide, Nukleoside, Pyrimidin- oder Purinbasen sind.

In einer weiteren Ausführungsform werden die mindestens zwei unterschiedlichen zur Bindung befähigten Gruppen des Substrats ausgewählt unter Gruppen, die Bestandteil von Aminosäuren, Zucker, Nukleotide, Nukleoside, Pyrimidin- oder Purinbasen sind.

Durch das Einfügen von weiteren Gruppen natürlichen oder synthetischen Ursprungs, insbesondere synthetischen Ursprungs, kann die nicht-kovalente Bindungsfähigkeit des Sorbens gezielt variiert, insbesondere verstärkt werden.

Beispielsweise können Aminosäuren, die mit synthetischen Schutzgruppen versehen sind, in das neue Verfahren eingesetzt werden. Beispielsweise können Aminosäuren, die mit dem Fluorenyl-Rest geschützt sind, eingesetzt werden. Außer dem Fluorenyl- können auch Reste wie der Anthracenyl- oder der Naphthylrest verwendet werden. Hierbei kann durch Ausbildung weiterer nicht-kovalenter Bindungen zwischen den aromatischen Ringen der Schutzgruppen und der bindenden Gruppen des Substrats eine Verstärkung der bindenden Wirkung erreicht werden. Als weitere Beispiele seien Nitrophenyl- und Oligofluorphenylreste und andere elektronenreiche oder elektronenarme Aromaten genannt, die $\pi$-$\pi$-Wechselwirkungen ausbilden können.

Das Sorbens der Stufe (ii) umfasst einen Träger, der aus anorganischen oder organischen Materialien oder anorganischen und organischen Materialien aufgebaut sein kann. Als Trägermaterialien sind sämtliche Materialien geeignet, auf die sich die mindestens zwei unterschiedlichen Gruppen aus Stufe (ii) durch geeignete Verfahren einführen lassen.

Das Trägermaterial ist ein Feststoff, daher kann seine Oberfläche eben sein, wie beispielsweise Platten aus Glas oder Metall, oder auch gekrümmt oder in poröse Körper eingebettet, beispielweise röhren- oder schwammartig, wie beispielsweise Zeolithe, Kieselgel oder Cellulose-Beads. Weiter können die Trägermaterialien natürlichen Ursprungs oder synthetischer Natur sein. Als Beispiele seien unter anderem Gelatine, Kollagen oder Agarose genannt. Auch können poröse und nicht-poröse Harze sowie Kunststoff- oder Keramikoberflächen verwendet werden.

Erfindungsgemäß liegen die jeweils mindestens zwei unterschiedlichen Gruppen der Stufe (ii) auf dem Träger

in kovalent gebundener Form an ein Polymeres vor.

Dabei fallen unter den Begriff "Polymeres" auch höhermolekulare Verbindungen, die in der Polymerchemie als "Oligomere" bezeichnet werden. Dabei können sowohl ein Polymeres wie auch Gemische von Polymeren verwendet werden. Ohne auf bestimmte Polymere beschränken zu wollen, seien als mögliche Polymere u.a. genannt:

- Polysaccharide, wie z. B. Cellulose, Amylose und Dextrane;
- Oligosaccharide wie z. B. Cyclodextrine;
- Chitosan;
- Polyvinylalkohol, Poly-Threonin, Poly-Serin;
- Polyethylenimin, Polyallylamin, Polyvinylamin, Polyvinylimidazol, Poly-anilin, Polypyrrol, Poly-Lysin;
- Poly(meth)acrylsäure(ester), Polyitaconsäure, Poly-Asparagin;
- Poly-Cystein.

Ebenso sind nicht nur Homopolymere, sondern auch Copolymere und insbesondere Block-Copolymere und statistische Copolymere prinzipiell geeignet, um im vorliegenden Verfahren eingesetzt zu werden. Dabei sind sowohl Copolymere mit nicht-funktionalisierbaren Anteilen wie etwa Co-Styrol oder Co-Ethylen oder auch Copolymere wie etwa Co-Pyrrolidon zu nennen.

Besagte Polymere weisen dabei mindestens zwei gleiche oder unterschiedliche funktionelle Gruppen auf, über die die mindestens zwei unterschiedlichen zur Bindung befähigte Gruppen aus Stufe (ii) an das Polymere kovalent gebunden werden können.

Demzufolge ist die Erfindung dadurch gekennzeichnet, dass die jeweils mindestens zwei unterschiedlichen Gruppen in Stufe (ii) kovalent gebunden an ein Polymeres vorliegen.

Als unter anderem bevorzugte funktionelle Gruppen des mindestens zwei gleiche oder unterschiedliche funktionelle Gruppen aufweisenden Polymeren sind u.a. OH-Gruppen, gegebenenfalls substituierte Amingruppen, SH-Gruppen, $OSO_3H$-Gruppen, $SO_3H$-Gruppen, $OPO_3H_2$-Gruppen, $OPO_3HR$-Gruppen, $PO_3H_2$-Gruppen, $PO_3HR$-Gruppen, COOH-Gruppen und Gemische aus zwei oder mehr davon zu nennen, wobei R vorzugsweise einen Alkylrest bedeutet. Ebenso können die mindestens zwei gleiche oder verschiedenen funktionelle Gruppen aufweisenden Polymere auch weitere polare Gruppen, wie beispielsweise -CN, enthalten.

Erfindungsgemäß werden in Stufe (ii) die mindestens zwei unterschiedlichen zur Bindung befähigten Gruppen zunächst über die mindestens zwei gleichen oder unterschiedlichen funktionellen Gruppen des Polymeren in dieses eingeführt, wobei ein mit besagten Gruppen derivatisiertes Polymeres entsteht.

Die Derivatisierung des funktionalisierten Polymeren mit den mindestens zwei Gruppen kann dabei nach bekannten Methoden sowohl in homogener wie auch heterogener Phase erfolgen.

Bei Derivatisierung in heterogener Phase kann die Festphasenreaktion verwendet werden.

Werden die mindestens zwei gleichen oder unterschiedlichen funktionellen Gruppen aufweisenden Polymere in homogener flüssiger Phase mit besagten mindestens zwei unterschiedlichen zur Bindung befähigten Gruppen derivatisiert, so werden, um eine optimale Löslichkeit zu erreichen, vorzugsweise gemischtfunktionale Polymere eingesetzt. Als Beispiele hierfür sind etwa zu nennen:

- partiell oder vollständig silylierte, alkylierte oder acylierte Cellulose;
- Polyvinylacetat / Polyvinylalkohol;
- Polyvinylether / Polyvinylalkohol;
- N-Butylpolyvinylamin / Polyvinylamin.

Ebenso können auch Polymer/Copolymer-Gemische verwendet werden. Dabei können alle geeigneten Polymer/Copolymer-Gemische eingesetzt werden, beispielsweise Gemische aus den oben bereits genannten Polymeren und Copolymeren, wobei unter anderem hierbei etwa zu nennen ist:

- Poly(acrylsäure)-co-Vinylacetat;
- Polyvinylalkohol-co-Ethylen;
- Polyoxymethylen-co-Ethylen;
- modifizierte Polystyrole, wie z.B. Copolymere des Styrols mit (Meth)acrylsäure(estern);
- Polyvinylpyrrolidon und dessen Copolymere mit Poly(meth)acrylaten.

Das mindestens zwei gleiche oder unterschiedliche funktionelle Gruppen aufweisende Polymere kann vor der Derivatisierung mit den mindestens zwei unterschiedlichen Gruppen mit einem Aktivierungsreagens umgesetzt werden. Solche Reagenzien und Verfahren zu deren Verwendung sind beispielsweise in der WO 00/32649 beschrieben.

Beispielsweise können als Aktivierungsreagenzien Verbindungen verwendet werden, die sich vom Strukturelement des Succinimids ableiten, wobei das am Stickstoff befindliche Wasserstoffatom durch eine -OCO-Cl-Gruppe ersetzt ist. Ein Beispiel ist die nachstehend aufgeführte Verbindung.

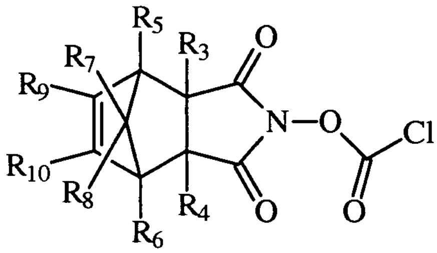

$R_3$ bis $R_{10}$ können dabei vorzugsweise für Wasserstoff, Alkyl-, Aryl-, Cycloalkyl- und heterocyclische Reste stehen. Stehen die Reste $R_3$ bis $R_{10}$ für Wasserstoff, so wird die Verbindung im Folgenden auch als ONB-Cl bezeichnet.

Wird das mindestens zwei gleiche oder verschiedene funktionelle Gruppen aufweisende Polymere mit einem Aktivierungsreagens umgesetzt, so kann dieses Umsetzungsprodukt mit geeigneten Verbindungen, die die zur Bindung besagten Substrats erforderlichen Gruppen besitzen, umgesetzt werden.

Es ist auch denkbar, das mindestens zwei gleiche oder verschiedenene funktionelle Gruppen aufweisende Polymere mit einem Gemisch aus zwei oder mehr geeigneten Aktivierungsreagenzien umzusetzen. Diese können gleichzeitig mit dem Polymeren umgesetzt werden. Ebenso können die zwei oder mehr Aktivierungsreagenzien auch nacheinander mit dem Polymeren umgesetzt werden.

Hierbei können prinzipiell alle Verbindungen, die mit dem aktivierten Polymeren reagieren können und unmittelbar oder mittelbar zum gewünschten Polymeren führen, das dann derivatisiert ist, verwendet werden. Unter anderem können zur Derivatisierung Verbindungen eingesetzt werden, die mindestens eine nucleophile Gruppe aufweisen.

Eine weitere Möglichkeit besteht darin, das aktivierte Polymere mit einem aminogruppenhaltigen ein- oder mehrwertigen Alkohol bzw. Thiol umzusetzen. Wird das mindestens zwei funktionelle Gruppen enthaltende Polymer beispielsweise mit ONB-Cl aktiviert, so reagiert der aminogruppenhaltige ein- oder mehrwertige Alkohol oder das aminogruppenhaltige ein- oder mehrwertige Thiol selektiv mit der Aminogruppe. Die somit in das Polymer eingeführten OH- oder SH-Gruppen lassen sich dann in einem weiteren Schritt wieder mit beispielsweise einem der oben beschriebenen Aktivierungsreagenzien aktivieren, wodurch Kettenverlängerungen und Verzweigungen ermöglicht werden, je nach Wertigkeit der ursprünglich eingesetzten Alkohole oder Thiole.

In einer anderen Ausführungsform ist es auch möglich, zunächst Verbindungen, die jeweils mindestens eine unterschiedliche zur Bindung befähigte Gruppe enthalten, mit einem Aktivierungsreagens umzusetzen, und dann mit besagtem Polymeren weiterzureagieren.

Vorzugsweise werden aktivierte Derivate von Aminosäuren, Zuckern, Nukleotiden, Nukleosiden, Primidin- und Purinbasen mit dem mindestens zwei gleiche oder unterschiedliche funktionelle Gruppen aufweisenden Polymeren umgesetzt. Dabei werden, wiederum in einer bevorzugten Ausführungsweise, die Verbindungen mit ONB-Cl oder einer Verbindung dieses Strukturtyps aktiviert werden.

Besagte Umsetzungen können zur Polymervernetzung, zur Polymerstabilisierung und zur Polymerverzweigung verwendet werden.

Besagte Umsetzungen ermöglichen es weiterhin, Polymerderivate herzustellen, die verschiedenste räumliche Anordnungen aufweisen und demzufolge für eine Vielzahl von Anwendungen, in denen diese räumliche Anordnung von entscheidender Bedeutung ist, verwendbar sind.

So lassen sich beispielsweise Anordnungen realisieren, die wie Hairy Rods, Kammpolymere, Netze, Körbe, Schalen, Röhren, Trichter oder Reußen aufgebaut sind.

Die Umsetzungen können dabei in aprotisch-dipolaren und/oder polar-protischen Lösungsmitteln oder Lösungsmittelgemischen wie z. B. wäßrigen Lösungsmittelgemischen erfolgen. Je nach umzusetzendem Polymertyp und verwendetem Aktivierungsreagens und/oder Verbindungen, die die mindestens zwei unterschiedlichen zur Bindung befähigten Gruppen enthalten, können in diesen Lösungsmittelgemischen neben Wasser verschiedene weitere Lösungsmittel vorliegen. Bevorzugt werden hierbei unter anderem Lösungsmittel wie z. B. aprotisch-dipolare Lösungsmittel wie z.B. DMSO, DMF, Dimethylacetamid, N-Methylpyrrolidon, Tetrahydrofuran oder Methyl-t-butylether eingesetzt.

Der pH-Wert, der bei den Umsetzungen gewählt werden kann, liegt dabei im Allgemeinen im Bereich von 4 bis 14, bevorzugt im Bereich von 8 bis 12, und besonders bevorzugt im Bereich von 8 bis 10. Zur Einstellung eines bestimmten Bereiches des pH-Wertes kann mit geeigneten Pufferlösungen gearbeitet werden.

Über Lösungsmittel und pH-Wert können gezielt die Quell- und Schrumpfeigenschaften des Netzwerks eingestellt werden, wodurch über das Netzwerk der Zugang des Substrats zum Sorbens beeinflusst werden kann.

Der Derivatisierungsgrad des Polymeren, also der Grad zu dem das funktionalisierte Polymere mit den mindestens zwei zur Bindung befähigten Gruppen derivatisiert ist, kann so beeinflusst werden, dass die bestmögliche Wechselwirkung mit dem Substrat erreicht wird.

Bevorzugt wird ein Derivatisierungsgrad im Bereich von 1 bis 70 %, besonders bevorzugt im Bereich von 3 bis 60 % und insbesondere bevorzugt im Bereich von 5 bis 50 %.

Deshalb ist es auch möglich, dass mindestens zwei der gleichen oder unterschiedlichen funktionellen Gruppen derivatisiert sind, so dass sie als Rezeptorgruppen mit dem Substrat wechselwirken und mindestens eine, nicht substratspezifisch wirkende funktionelle Gruppe und/oder eine Monomereinheit ohne funktionelle Gruppe zwischen zwei dieser derivatisierten Gruppen liegen, und wobei die funktionellen Gruppen gleich oder verschieden voneinander sind und ausgewählt werden aus den oben genannten Gruppen.

Es ist auch denkbar, dass die in underivatisierter Form im Polymeren noch vorliegenden funktionellen Gruppen einen Beitrag zur Wechselwirkung mit dem Substrat leisten.

Möglich ist auch die Verwendung eines Derivats eines mindestens zwei gleiche oder unterschiedliche funktionelle Gruppen aufweisenden Polymeren, in dem eine weitere, nicht substratspezifisch wirkende funktionelle Gruppe mit einer Endcapping-Gruppe derivatisiert ist.

Durch geeignete Wahl der Endcapping-Gruppe ist es auch möglich, die Löslichkeit des mit der Endcapping-Gruppe oder mit den Endcapping-Gruppen versehenen Polymerderivats zu beeinflussen und den Erfordernissen bei eventuellen späteren, weiteren Umsetzungen anzupassen.

Als Endcapping-Gruppe kann prinzipiell jede Gruppe gewählt werden, die eine funktionelle Gruppe inert oder weitestgehend inert gegen Wechselwirkungen mit dem Substrat macht. Der Begriff "inert" bedeutet in diesem Zusammenhang, dass die Wechselwirkungen, die das Substrat mit den Rezeptorgruppen des derivatisierten Polymers eingeht, im Vergleich zu den Wechselwirkungen, die dieses Substrat mit einer oder mehreren der durch die Endcapping-Gruppe derivatisierten funktionellen Gruppe eingeht, so stark sind, dass das Substrat im Wesentlichen nur über die Rezeptorgruppen gebunden wird.

Ist es gewünscht, über die Wechselwirkung zwischen Substrat und Rezeptorgruppe zwei oder mehr unterschiedliche Substrate, beispielsweise in einem chromatographischen Prozeß, zu trennen, so muss die Endcapping-Gruppe die funktionelle Gruppe gegenüber möglichen Wechselwirkungen, wie oben beschrieben, nicht völlig inert machen. In diesem Fall genügt es beispielsweise, wenn die Endcapping-Gruppe mit den zwei oder mehr zu trennenden Substraten genügend schwache oder unspezifische Wechselwirkungen eingeht, die für den Trennprozeß keine Rolle spielen.

Als Endcapping-Gruppe kann dabei jede geeignete Gruppe gemäß dem Stand der Technik verwendet werden. Je nach Substrat ist es beispielsweise denkbar, dass als Endcapping-Gruppe eine Gruppe gewählt wird, die kein H-Donor ist. Bevorzugt wird dabei

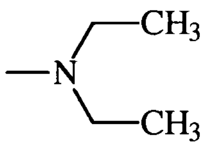

besonders bevorzugt

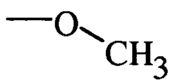

eingesetzt.

Als Rezeptor kann in einem mindestens zwei gleiche oder unterschiedliche funktionelle Gruppen aufweisenden Polymeren jeder weiter oben beschriebene Rest eingeführt werden, der durch Umsetzung des Polymeren mit mindestens zwei aktivierten Derivatisierungsreagenzien, die mindestens jeweils eine nucleophile Gruppe umfassen, oder durch Umsetzung des aktivierten Polymeren mit mindestens zwei solcher Derivatisierungsreagenzien erhalten werden kann.

Bevorzugt wird ein Derivat eines mindestens zwei gleiche oder unterschiedliche funktionelle Gruppen aufweisenden Polymeren, wie oben beschrieben, bei dem mindestens zwei Rezeptoren Reste von Verbindungen oder Gruppen, die in Verbindungen für die Bindung verantwortlich sind, umfassen, wobei die Verbindungen ausgewählt sind aus der Gruppe umfassend Aminosäuren, Zucker, Nukleotide, Nukleoside, Pyrimidin- und Purinbasen.

Um das funktionelle Gruppen-aufweisende Polymere mit den genannten Verbindungen, Derivaten dieser Verbindungen oder Gruppen, die diese Verbindungen enthalten, oder Mischungen davon zu derivatisieren, kann man nach dem oben beschriebenen Verfahren vorgehen. So ist es denkbar, zuerst die Umsetzung beispielsweise einer Aminosäureverbindungen mit einem geeigneten Aktivierungsreagens durchzuführen und dann das Umsetzungsprodukt mit

dem Polymeren umzusetzen. Ebenso ist es denkbar, zuerst das Polymere mit einem geeigneten Aktivierungsreagens zu aktivieren und dann mit der Aminosäure umzusetzen. Natürlich ist es auch denkbar, Polymeres, Aminosäure und Aktivierungsreagens direkt zusammenzugeben.

Die Einführung von Resten von Zuckern, Nukleotiden, Nukleosiden, Pyrimidin- und Purinbasen oder von bindenden Gruppen, die in besagten Verbindungen enthalten sind, oder Mischungen davon, ist in analoger Weise möglich.

Je nach Wahl der Aminosäuren, Zucker, Nukleotide, Nukleoside, Pyrimidin- und Purinbasen oder der entsprechenden Reste oder Derivate oder der bindenden Gruppen, die in diesen Verbindungen enthalten sind, kann es notwendig sein, eventuell hierin vorhandene funktionelle Gruppen bei der Derivatisierung und/oder der Aktivierung mit Schutzgruppen zu schützen. Hierbei sind alle geeigneten Schutzgruppen möglich, die aus dem Stand der Technik bekannt sind. Je nach späterer Verwendung des Polymeren können diese Schutzgruppen nach der Derivatisierung am Aminosäure-, Zucker-, Nukleotid-, Nukleosid-, Pyrimidin- und Purinbasenrest verbleiben oder wieder abgespalten werden.

Statt der Aminosäure ist auch die Verwendung eines oder mehrerer Oligopeptide denkbar.

Um die Wechselwirkung mit dem Substrat zu optimieren, kann das flüssige oder in einem Lösungsmittel bzw. Lösungsmittelgemisch gelöste Polymerderivat in Anwesenheit des Substrates, das hierbei als Templat wirkt, verformt werden.

Dabei geht man bei der Verformung beispielsweise so vor, dass man in einem geeigneten Lösungsmittel oder Lösungsmittelgemisch ein derivatisiertes Polymeres, wie oben beschrieben, mit dem Substrat zusammengibt und dem Polymeren die Möglichkeit gibt, dabei eine oder mehrere energetisch begünstigte Konformationen einzunehmen.

Dabei ist es auch denkbar, ein derivatisiertes Polymeres mit verschiedenen Substraten zusammenzugeben und zu verformen. Weiter ist es auch denkbar, sollte dies erforderlich sein, verschiedene derivatisierte Polymere mit einem oder mehreren verschiedenen Substraten zusammenzugeben und zu verformen.

Es ist auch denkbar, dass das Derivat des mindestens zwei gleiche oder unterschiedliche funktionelle Gruppen aufweisenden Polymeren ohne Templat verformt wird.

Im Anschluß an die Verformung kann die Konformation des Polymerderivates, die sich durch die Verformung in Anwesenheit des Templates gebildet hat, fixiert werden.

Hierbei ist es auch möglich, das verformte Polymere vor der Fixierung auf einen Träger aufzubringen.

Zur Fixierung sind prinzipiell alle denkbaren Verfahren einsetzbar. Insbesondere sind hierbei Temperaturänderung, Lösungsmittelwechsel, Fällung und Vernetzung zu nennen. Bevorzugt wird die Konformation durch Vernetzung fixiert.

Das Trägermaterial und die Form des Trägers sind dabei im Wesentlichen frei wählbar, wobei das Trägermaterial jedoch so beschaffen sein muß, dass das Polymer dauerhaft auf dem Träger aufgebracht werden kann. Vorzugsweise geht das Trägermaterial, nachdem das Polymere aufgebracht wurde, mit den zu trennenden Stoffen keine oder nur eine oder mehrere unspezifische Wechselwirkungen ein.

Je nach späterem Einsatzgebiet kann es erforderlich sein, dass das Trägermaterial druckstabil ist. Der Begriff "druckstabil" bedeutet in diesem Zusammenhang, dass das Trägermaterial bei Drücken bis zu 100 bar formstabil ist.

Als Trägermaterialien können die vorstehend genannten Materialien verwendet werden. Die Form des Trägermaterials kann dabei den Erfordernissen des Verfahrens angepasst werden und unterliegt keinen Beschränkungen. Möglich sind beispielsweise tabletten-, kugel- oder strangförmige Träger.

Die Aufbringung auf das Trägermaterial ist weitgehend frei wählbar. Möglich sind beispielsweise das Aufbringen durch Imprägnieren, Eintauchen des Trägers in eine entsprechende Polymerlösung, Aufsprühen des Polymers oder Aufrotieren des Polymers.

Es ist auch möglich, das derivatisierte Polymere auf verschiedene geeignete Träger aufzubringen. Ebenso ist es möglich, zwei oder mehr voneinander verschiedene derivatisierte Polymere auf einen oder mehrere geeignete Träger aufzubringen.

In einer weiteren Ausführungsform des erfindungsgemäßen Verfahrens wird das derivatisierte, verformte und fixierte Polymere zu einem porösen Material verarbeitet. Es bildet dann gleichzeitig den Träger, so dass kein zusätzliches Trägermaterial benötigt wird. Dabei können z. B. Beads, unregelmäßige Partikel, Schwämme, Scheiben, Stränge oder Membranen erhalten werden.

Dabei kann eine Konformation fixiert werden, die sich aus einer Art von derivatisiertem Polymeren gebildet hat. Ebenso ist es aber auch denkbar, dass die Konformation aus zwei oder mehr voneinander unterschiedlichen Arten von derivatisierten Polymeren gebildet wurde. Der Begriff "unterschiedliche Arten von derivatisierten Polymeren" bedeutet hierbei, dass sich die Polymere beispielsweise hinsichtlich des Basispolymeren oder der Art des Aktivierungsreagens oder der Art durch Derivatisierung eingeführten Rezeptorgruppen oder des Aktivierungsgrades oder des Derivatisierungsgrades oder einer Kombination aus zwei oder mehr dieser Merkmale unterscheiden.

Die Vernetzung kann hierbei beispielsweise dadurch erreicht werden, dass zwei oder mehr Stränge von derivatisierten Polymeren direkt miteinander reagieren. Dies kann dadurch erreicht werden, dass die durch Derivatisierung eingeführten Gruppen so beschaffen sind, dass zwischen diesen Gruppen kovalente und/oder nicht-kovalente Bindungen geknüpft werden können. Ganz allgemein ist es denkbar, dass diese kovalenten und/oder nicht-kovalenten Bindungen

zwischen Gruppen ausgebildet werden, die an einem Polymerstrang hängen, und/oder zwischen Gruppen ausgebildet werden, die an zwei oder mehr Polymersträngen hängen, so dass durch die Vernetzung zwei oder mehr Polymerstränge über eine oder mehrere Stellen miteinander verknüpft sein können.

Ebenso ist es auch denkbar, zur Vernetzung ein oder mehrere geeignete Vernetzungsmittel einzusetzen, mit denen, wie vorstehend beschrieben, in kovalenter und/ oder nicht-kovalenter Weise Gruppen innerhalb eines Polymerstrangs und/oder Gruppen, die an mehreren Strängen von gegebenenfalls unterschiedlichen derivatisierten Polymeren hängen, vernetzt werden können.

Als Vernetzungsreagenzien kommen prinzipiell alle geeigneten, aus dem Stand der Technik bekannten Verbindungen in Betracht. Demgemäß kann die Vernetzung beispielsweise in kovalent-reversibler Weise, in kovalent-irreversibler Weise oder in nicht-kovalenter Weise erfolgen, wobei bei Vernetzung in nicht-kovalenter Weise beispielsweise Vernetzungen über ionische Wechselwirkung oder über Charge-Transfer-Wechselwirkung zu nennen sind.

Als Vernetzungsreagenzien, die zu kovalent-irreversibler Vernetzung führen können, sind unter anderem zwei- oder mehrfach funktionelle Verbindungen wie beispielsweise Diole, Diamine oder Dicarbonsäuren zu nennen. Dabei werden beispielsweise zweiwertige Vernetzer mit dem aktivierten Polymerderivat umgesetzt oder das mindestens zweiwertige aktivierte Vernetzungsreagens mit dem nichtaktivierten Polymerderivat.

Eine kovalent-reversible Vernetzung kann beispielsweise durch Knüpfen einer Schwefel-Schwefel-Bindung zu einer Disulfidbrücke zwischen zwei an einem oder zwei Polymersträngen hängenden Gruppen realisiert werden.

Eine Vernetzung über ionische Wechselwirkung kann beispielsweise über zwei Reste zustandekommen, von denen der eine als Struktureinheit ein quartäres Ammoniumion und der andere als Struktureinheit beispielsweise

$-COO^-$ oder $-SO_3^-$

aufweist.

Eine Vernetzung über Wasserstoffbrücken kann beispielsweise zwischen zwei komplementären Basenpaaren ausgebildet werden, beispielsweise über folgende Struktur:

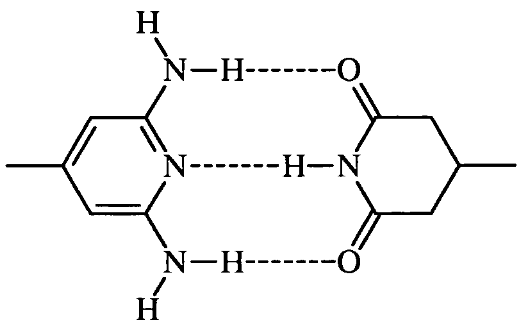

Ganz allgemein können nicht-kovalent zu vernetzende Polymerderivate bezüglich der Vernetzungsstellen komplementär aufgebaut sein, wobei zueinander komplementäre Struktureinheiten beispielsweise Säure/Triamin oder Uracil/Melamin sind. Ebenso kann bei einer nicht-kovalenten Vernetzung das Vernetzungsreagens komplemantär zu den Vernetzungsstellen am Polymerstrang sein. Als Beispiel hierfür wären etwa eine Amingruppe am Polymerstrang und eine Dicarbonsäure als Vernetzungsreagens zu nennen.

Aus dem Carboxylat lässt sich mit Hilfe der aus der Peptid-Chemie bekannten Kupplungsreagenzien eine Amidbindung zu den Aminogruppen des Polymeren herstellen. In gleicher Weise wird eine am Polymer kovalent gebundene Carboxylgruppe mit Aminogruppen im Polyvinylamin vernetzt oder umgekehrt eine gebundene Aminogruppe mit Carboxyl, z.B. von Polyacrylat.

Der Vernetzungsgrad kann im Wesentlichen beliebig gewählt werden und beispielsweise auf die nachstehend beschriebenen Einsatzgebiete zugeschnitten werden.

In Stufe (ii) kann die Umsetzung der mindestens zwei unterschiedlichen zur Bindung befähigten Gruppen mit dem mindestens zwei Gruppen enthaltendem Polymeren auch in heterogener Phase erfolgen, d. h. an der festen Oberfläche des Polymeren. Zeckmäßigerweise wird dann besagtes Polymer in einem Lösungsmittel suspendiert, das für das eingesetzte Polymere eine nur geringe Lösekraft besitzt.

Zur Derivatisierung des Polymeren sowie zum Aufbringen des erhaltenen Polymeren auf den Träger können die vorstehend beschriebenen Aktivierungs- und Derivatisierungsschritte sowie Vernetzungs- und Beschichtungsmethoden verwendet werden.

Andererseits ist es auch möglich, das vorzugsweise in heterogener Phase derivatisierte Polymere ohne weiteres Trägermaterial als Träger zu verwenden.

Die vorstehend beschriebenen und in homogener oder heterogener Phase hergestellten derivatisierten Polymere können schrittweise auf den Träger aufgebracht werden. Hierbei wird in mindestens einem Schritt mindestens eine Lage des mindestens einen Polymeren an das Trägermaterial gebunden und in mindestens einem weiteren Schritt mindestens eine weitere Lage des mindestens einen Polymeren auf die mindestens eine an das Trägermaterial gebundene Polymerlage aufgebracht. Die dazu geeigneten Verfahren sind in der WO 01/38009 beschrieben.

Das schrittweise Aufbringen des mindestens einen Polymeren kann hierbei gemäß sämtlicher geeigneter Verfahren erfolgen, die gewährleisten, dass pro Schritt mindestens eine Lage des Polymeren aufgebracht wird, so dass eine lagenförmige Polymerstruktur auf dem Trägermaterial aufgebracht wird.

Bei diesen Verfahren wird in dem mindestens einen Schritt, in dem die mindestens eine Lage des mindestens einen Polymeren auf dem Trägermaterial gebunden wird, in einer ersten Ausführungsform eine Lösung des mindestens einen Polymeren mit dem Trägermaterial bei Reaktionsbedingungen in Kontakt gebracht, bei denen das mindestens eine Polymere auf dem Trägermaterial nicht gebunden wird und anschließend werden die Reaktionsbedingungen derart variiert, dass das mindestens eine Polymere auf dem Trägermaterial gebunden wird, oder in einer zweiten Ausführungsform eine Lösung des mindestens einen Polymeren mit dem Trägermaterial bei Reaktionsbedingungen in Kontakt gebracht, bei denen die Lösung des mindestens einen Polymeren unter Theta-Bedingungen vorliegt.

Hierbei kann die Lösung, die gemäß der ersten Ausführungsform mit dem Trägermaterial in Kontakt gebracht wird, ein oder auch mehrere geeignete Lösungsmittel aufweisen, wobei das mindestens eine Polymere in dem Lösungsmittel oder Lösungsmittelgemisch gelöst oder auch kolloidal gelöst oder auch suspendiert, beispielsweise in Form einer Nanosuspension, sein kann.

Dann werden die Reaktionsbedingungen so gewählt, dass beim Inkontaktbringen der Lösung mit dem Trägermaterial zunächst keine Bindung des mindestens einen Polymeren an das Trägermaterial erfolgt. Diese Reaktionsbedingungen werden beispielsweise durch ein oder mehrere geeignete Lösungsmittel eingestellt. Bevorzugt werden diesbezüglich Lösungsmittel eingesetzt, in denen das mindestens eine Polymere so gut löslich ist, dass die Bindung an das Trägermaterial unterbleibt.

Im Sinne der vorliegenden Erfindung bedeutet der Begriff "das Polymer wird nicht an das Trägermaterial gebunden", dass mittels Messung des Verteilungskoeffizienten im Wesentlichen keine Bindung festgestellt werden kann.

Ebenso können diese Reaktionsbedingungen durch geeignete Temperaturwahl erreicht werden, in dem beispielsweise die Lösung mit dem Trägermaterial bei so hohen Temperaturen in Kontakt gebracht wird, dass die Bindung des mindestens einen Polymers an das Trägermaterial unterbleibt.

Weiter können diese Reaktionsbedingungen durch geeignete Einstellung des pH-Wertes der Polymerlösung erreicht werden, falls die Bindung des mindestens einen Polymeren an das Trägermaterial vom pH-Wert abhängig ist.

Ebenso ist auch denkbar, durch eine geeignete Kombination von zwei oder mehr dieser Methoden die Bindung des mindestens einen Polymeren an das Trägermaterial zunächst zu verhindern.

Durch diese spezifische Art der Reaktionsführung wird unter anderem erreicht, dass Reaktionsbedingungen, unter denen das in der Lösung enthaltene mindestens eine Polymer ausfällt, vermieden werden.

Was das Inkontaktbringen der Lösung des mindestens einen Polymers mit dem mindestens einen Trägermaterial anbelangt, so sind prinzipiell alle geeigneten Verfahrensführungen denkbar.

So ist es beispielsweise möglich, eine Lösung, die das mindestens eine Polymer umfasst, mit dem Trägermaterial in Kontakt zu bringen. Ebenso ist es denkbar, das Trägermaterial zuerst mit dem mindestens einen Lösungsmittel in Kontakt zu bringen und dann in das mindestens eine Lösungsmittel das mindestens eine Polymer einzubringen. Ebenso ist es möglich, zuerst das Trägermaterial mit mindestens einem Lösungsmittel in Kontakt zu bringen und dann eine Lösung, die das mindestens eine Polymer umfasst, zuzugeben. Werden zwei oder mehr Polymere eingesetzt, so ist es denkbar, jedes separat oder zusammen mit einem oder mehreren anderen Polymeren in jeweils einem Lösungsmittel oder Lösungsmittelgemisch zu lösen und die einzelnen Lösungen, von denen jede mindestens ein Polymer umfasst, zusammen oder getrennt mit dem Trägermaterial, das gegebenenfalls bereits gelöst oder kolloidal gelöst oder suspendiert in mindestens einem Lösungsmittel vorliegt, in Kontakt zu bringen.

Prinzipiell sind die bereits vorstehend beschriebenen Trägermaterialien geeignet, auf die das mindestens eine Polymere durch Bindung aufgebracht werden kann. Werden zwei oder mehr voneinander verschiedene Polymere eingesetzt, so ist es ausreichend, wenn eines der Polymeren durch Bindung auf das Trägermaterial aufgebracht werden kann. Es ist auch denkbar, dass zwei oder mehr verschiedene Polymere durch Bindung auf das Trägermaterial aufgebracht werden können.

Werden zwei oder mehr voneinander verschiedene Polymere und zwei oder mehr voneinander verschiedene Trägermaterialien verwendet, so ist unter anderem denkbar, dass sämtliche Polymere auf sämtliche Trägermaterialien aufgebracht werden können. Ebenso ist es denkbar, dass ein oder mehrere Polymere auf einem oder mehreren Trägermaterialien und ein oder mehrere davon verschiedene Polymere auf einem oder mehreren davon verschiedenen Trägermaterialien aufgebracht werden können.

Ferner können weitere Polymere und Verbindungen, wie beispielsweise allgemein übliche Hilfsmittel aufgebracht werden, wobei die Bindung des Polymeren an das Trägermaterial auch durch andere Wechselwirkungen oder/und

Verfahren erreicht werden kann. Weiterhin können die in der Lösung vorhandenen Polymere oder/und Verbindungen nicht auf dem Träger aufgebracht werden und beispielsweise in der Lösung verbleiben. Unter anderem ist es denkbar, dass mindestens eines dieser Polymere in einem weiteren Schritt auf beispielsweise ein Trägermaterial aufgebracht wird, das vor diesem weiteren Schritt mit der Lösung, die dieses Polymer umfasst, in Kontakt gebracht wird.

Gemäß der ersten Ausführungsform werden nach dem Inkontaktbringen die Reaktionsbedingungen derart geändert, dass nun die Bindung des mindestens einen Polymeren an das Trägermaterial erfolgt. Wie vorstehend beschrieben, ist es denkbar, dass im Falle, dass zwei oder mehr verschiedene Polymere oder/und zwei oder mehr verschiedene Trägermaterialien eingesetzt werden, ein Polymeres an ein Trägermaterial gebunden wird.

Was die Variation der Reaktionsbedingungen anbelangt, so sind sämtliche Änderungen denkbar, die dazu geeignet sind, die Bindung mindestens eines der Polymere an das Trägermaterial zu ermöglichen.

Im Falle, dass die Bindung von der Temperatur abhängig ist, ist es beispielsweise denkbar, die Temperatur entweder zu erhöhen oder zu erniedrigen, je nachdem, welche Änderung die Bindung begünstigt. In ebenfalls bevorzugten Ausführungsformen wird die Zuammensetzung der Lösung, die das mindestens eine Polymere enthält, geändert oder diese Lösung langsam eingeengt.

Was die Änderung der Zusammensetzung der Lösung, die das mindestens eine Polymere enthält, anbelangt, so sind prinzipiell sämtliche Verfahren denkbar, die geeignet sind, durch diese Änderung die Bindung zu ermöglichen.

In einer bevorzugten Ausführungsform wird der Lösung, in der das mindestens eine Polymere enthalten ist, mindestens ein weiteres Lösungsmittel zugegeben, das hinsichtlich mindestens eines der Polymeren schlechtere Lösungsmitteleigenschaften aufweist.

In einer weiteren Ausführungsform wird die Zusammensetzung der Lösung derart geändert, dass mindestens eine saure oder mindestens eine basische Verbindung oder ein Gemisch aus zwei oder mehr davon zugegeben werden, durch die der pH-Wert der Lösung so geändert wird, dass die Bindung mindestens eines der Polymeren ermöglicht wird. Selbstverständlich ist es auch möglich, eine oder mehrere Pufferlösungen zuzugeben, durch die der pH-Wert der Lösung so geändert wird, dass die Bindung mindestens eines der Polymeren ermöglicht wird.

Ferner können geeignete Verbindungen wie beispielsweise Salze, umfassend beispielsweise Metallkationen, oder geeignete organische Verbindungen zugegeben werden, durch deren Zusatz die Bindung mindestens eines der Polymeren erfolgt.

Die Lösung, die das mindestens eine Polymere enthält, kann auch derart eingeengt werden, dass die Konzentration des mindestens einen Polymeren, das an das Trägermaterial gebunden werden soll, in der Lösung weitgehend konstant bleibt. Dieses Einengen der Lösung erfolgt dann durch eine entsprechend langsame Verfahrensführung, durch die die Polymerkonzentration weitgehend konstant gehalten wird.

Ferner können zwei oder mehr der oben genannten Methoden unter Einschluß der Änderung der Temperatur in geeigneter Weise kombiniert werden. So ist es beispielsweise denkbar, sowohl die Zusammensetzung der Lösung wie beschrieben zu variieren und unterstützend die Lösung langsam einzuengen oder/und die Temperatur in geeigneter Weise zu variieren.

Je nach gewählten Reaktionsbedingungen ist es denkbar, dass ein Polymeres oder mehrere, voneinander verschiedene Polymere auf das Trägermaterial aufgebracht werden. Unter anderem ist es denkbar, die Reaktionsbedingungen derart zu wählen, dass zwei oder mehr voneinander verschiedene Polymere gleichzeitig auf das Trägermaterial aufgebracht werden, wobei eine Lage auf dem Trägermaterial entsteht, die die zwei oder mehr voneinander verschiedenen Polymeren umfasst. Werden zwei oder mehr voneinander verschiedene Trägermaterialien verwendet, so ist es denkbar, dass auf jedem Trägermaterial eine Lage eines Polymeres aufgebracht wird, die ein Polymeres oder zwei oder mehr voneinander verschiedene Polymere umfassen kann.

Weiterhin ist es auch möglich, dass in einem Schritt zwei oder mehr Lagen mindestens eines Polymeren auf das Trägermaterial aufgebracht werden, wobei die erste Lage des Polymeren an das Trägermaterial gebunden ist, die zweite Lage des Polymeren an die erste Lage gebunden ist und gegebenfalls jede weitere Lage des Polymeren an die jeweils vorhergehende Lage gebunden ist. Dabei kann jede Lage prinzipiell eine einzige Polymerart oder zwei oder mehr voneinander verschiedene Polymere umfassen.

Ferner kann gemäß der zweiten Ausführungsform eine Lösung des mindestens einen Polymeren mit dem Trägermaterial bei Reaktionsbedingungen in Kontakt gebracht, bei denen die Lösung des mindestens einen Polymers unter Theta-Bedingungen vorliegt. Hinsichtlich dieser Ausführungsform erfolgt das Aufbringen des mindestens einen Polymeren auf das Trägermaterial ganz besonders bevorzugt während des Inkontaktbringens der Lösung mit dem Trägermaterial.

Bevorzugt wird gemäß dem vorstehend beschriebenen Verfahren in einem ersten Schritt eine Lage mindestens eines Polymeren auf das Trägermaterial aufgebracht und auf diese erste Lage in einem zweiten Schritt eine zweite Lage und auf die zweite Lage gegebenfalls in einem dritten Schritt eine dritte Lage und so fort. Bezüglich der geeigneten Methoden des Aufbringens sei auf obenstehende ausführliche Diskussion verwiesen.

Unter dem Begriff Bindung des Polymeren an den Träger werden sämtliche nicht-kovalenten Wechselwirkungen verstanden, über die das mindestens eine Polymer mit dem Trägermaterial oder/und mit einer gegebenfalls bereits auf

das Trägermaterial aufgebrachten oder gegebenenfalls auf einer Polymerlage aufgebrachten Polymerlage wechselwirken kann.

Demgemäß können im Wesentlichen sämtliche Polymere eingesetzt werden, die zur Ausbildung solcher nichtkovalenten Wechselwirkungen in der Lage sind. Hierbei ist es unter anderem denkbar, dass mindestens eine funktionelle Gruppe, über die das Polymer mindestens eine dieser Wechselwirkungen ausbildet, im Polymerstrang selbst oder/und in mindestens einer Seitenkette des Polymerstrangs liegt.

Wechselwirkungen können jedoch auch beispielsweise über Kohlenwasserstoffketten und weitere Struktureinheiten, über die van der Waals-Wechselwirkungen aufgebaut werden können, erfolgen.

Erfindungsgemäß werden sämtliche der vorstehend beschriebenen Polymere oder/und Copolymere oder deren Gemische in underivatisierter Form auf dem Träger aufgebracht, wobei gewährleistet sein muss, dass sie, wie vorstehend beschrieben, zu mindestens einem Trägermaterial nicht-kovalente Wechselwirkungen ausbilden können.

Zur Derivatisierung des Polymeren, das auf den Träger aufgebracht ist, können dann die vorstehend beschriebenen Aktivierungs- und Derivatisierungsschritte verwendet werden, an die sich noch Vernetzungsschritte anschließen können, wie sie in der WO 00/32649 und WO 00/78825 beschrieben sind.

Das erfindungsgemäße Verfahren ist dadurch gekennzeichnet, dass vor der kovalenten Bindung der mindestens zwei unterschiedlichen Gruppen an das mindestens zwei gleiche oder verschiedenene funktionelle Gruppen aufweisende Polymere besagtes Polymere auf einen Träger aufgebracht wird.

In einer weiteren besonderen Ausführungsform des Verfahrens kann das mindestens zwei gleiche oder verschiedenene funktionelle Gruppen aufweisende Polymere auch durch Polymerisation oder Polykondensation mindestens zweier gleich oder verschieden funktionalisierter Monomerer direkt auf dem Träger erzeugt werden.

Dabei können vorzugsweise olefinisch ungesättigte Momomere, die vorzugsweise OH-Gruppen, gegebenenfalls substituierte Amingruppen, SH-Gruppen, $OSO_3H$-Gruppen, $SO_3H$-Gruppen, $OPO_3H_2$-Gruppen, $OPO_3HR$-Gruppen, $PO_3H_2$-Gruppen, $PO_3HR$-Gruppen, COOH-Gruppen und Gemische aus zwei oder mehr enthalten, wobei R vorzugsweise einen Alkylrest bedeutet, nach den bekannten Methoden miteinander in Anwesenheit des Trägermaterials polymerisiert werden. Auch können die Monomeren weitere polare Gruppen, wie beispielsweise -CN, enthalten. Weitere geeignete Monomere sind beispielsweise Ethylenimin, Allylamin oder Vinylpyrrolidon.

Als Polymerisationstechniken seien vorzugsweise die Emulsions-, Suspensions-, Dispersions- und Fällungspolymerisation genannt, wobei die Polymerisation in Gegenwart des Träger bzw. des Trägermaterials durchgeführt wird. Die Polymerisation kann durch die üblichen Methoden, beispielsweise durch Radikalstarter, wie Azoverbindungen oder Peroxide, durch kationische oder anionische Starter oder durch energiereiche Strahlung initiiert werden.

Dabei wird die Polymerisation so durchgeführt, dass erfindungsgemäß keine Reaktion zwischen den entstehenden Polymerketten und der Oberfläche des Trägers stattfindet. Vorzugsweise ist eines der mindestens zwei Monomeren ein hydrophiles Monomeres, wie beispielsweise Ethylenimin, Allylamin oder Vinylpyrrolidon. In Gegenwart eines hydrophilen Trägers, wie beispielsweise Kieselgel, wird das entstehende Polymere gewöhnlich stark auf der Trägeroberfläche adsorbiert.

Zur Erhöhung der Stabilität der gebildeten stationären Phase kann die Polymerisation der zwei gleich oder verschieden funktionalisierten Monomeren auch in Gegenwart eines oder mehrerer Vernetzer durchgeführt werden. Vernetzend wirkende Substanzen sind beispielsweise bifunktionelle Verbindungen, wie beispielsweise Divinylbenzol oder Ethylenglykoldiacrylat.

Auch können mindestens zwei gleich oder unterschiedlich funktionalisierte Monomerbausteine, die vorzugsweise die vorstehend genannten Gruppen tragen, nach den bekannten Methoden miteinander in Anwesenheit des Trägermaterials polykondensiert werden.

Dabei können auch die Methoden und Reagenzien auf ONB-Cl-Basis, wie sie in der WO 00/32649 und WO 00/78825 beschrieben sind, zum Einsatz kommen. Die erhaltenen funktionalisierten Polykondensate können vorzugsweise vom Polyphenylen-, Polyester-, Polyamid-, Polyether-, Polyetherketon-, Polyethersulfon-, Polyurethan- oder Polysiloxylsilan-Typ sein. Es können bei diesem Reaktionstyp auch gemischte Polykondensate entstehen. Die Polykondensation kann dabei in Lösung wie auch in der Schmelze durchgeführt werden.

Bevorzugt werden Polykondensate vom Polyester-Typ verwendet. Diese können zur Erhöhung der Stabilität auch durch Zusatz weiterer polyfunktioneller Verbindungen, wie beispielsweise mehrwertiger Alkohole, beispielsweise Trimethylolpropan, Pentaerythrit oder Zucker, weiter vernetzt werden. Auch ist die Vernetzung über polyfunktionelle Isocyanate möglich, sofern die Polyester Gruppen aufweisen, die mit den Isocyanat-Gruppen reaktiv sind. Beispielsweise können Hydroxylgruppen-enthaltende Polyester mit Polyisocyanaten umgesetzt werden, wobei in die Polyester Urethan-Einheiten eingebaut werden.

Das erhaltene beschichtete Trägermaterial kann dann beispielsweise aus der bei Polymerisation bzw. Polykondensation erhaltenen Reaktionsmischung durch Abfiltrieren isoliert und durch Auswaschen mit geeigneten Lösungsmitteln von nicht auf der Oberfläche des Trägermaterials gebundenen Polymer- bzw. Polykondensationspartikeln befreit werden.

In einer weiteren Ausführungsform der Erfindung ist es demnach möglich, dass das mindestens zwei gleiche

oder unterschiedliche funktionelle Gruppen enthaltende Polymere direkt auf dem Träger durch Polymerisation oder Polykondensation von mindestens zwei gleich oder verschieden funktionalisierten Monomeren erzeugt wird.

Es auch möglich, vorstehend beschriebene Polymerisation, die zur Beschichtung des Trägers führt, analog zur bekannten "imprinting-Technik" in Anwesenheit des später zu erkennenden Substrats durchzuführen. Im Sprachgebrauch dieser Technik wird für den Begriff Substrat auch häufig der Begriff Templat verwendet.

Vorausetzung für diese Polymerisation ist, dass die mindestens zwei gleich oder verschieden funktionalisierten Monomeren bereits die zur Bindung befähigten Gruppen tragen. Vorzugsweise trägt dabei jedes Monomere eine dieser Gruppen, wobei die Gruppen unterschiedlich sind.

Es ist aber auch möglich, Monomere einzusetzen, die bereits mindestens zwei unterschiedliche zur Bindung befähigte Gruppen aufweisen.

Vorzugsweise wird die Polymerisation in Gegenwart porenbildender Substanzen durchgeführt.

Zur Durchführung der Polymerisation können die weiter oben beschriebenen Polymerisatinstechniken verwendet werden.

Nach Herauslösen oder Herausspülen des Substrats mit geeigneten Lösungsmitteln wird in Stufe (ii) mindestens ein Sorbens mit einem vorgebildeten Wechselwirkungsraum für das Substrat erhalten.

Vorzugsweise werden für diese Ausführungsform die für die Polymerisation verwendeten Monomeren so ausgewählt, dass das auf dem Träger gebildete Polymere ein möglichst steifes und hochvernetztes Gerüst besitzt, damit der Wechselwirkungsraum möglichst stabil ist. Vorzugsweise werden daher als mindestens eines der funktionalisierten Monomere Acrylsäure oder Methacrylsäure oder deren Derivate oder Mischungen davon eingesetzt, die bekanntlich die Herstellung von Polymerisaten oder Copolymerisaten mit hohen Glasübergangstemperaturen erlauben. Als besonders gut für diesen Zweck geeignete Monomere seien beispielsweise Methacrylsäure und Ethylenglykoldimethacrylat genannt.

Als weiteres Beispiel sei die Polymerisation von Methacrylsäure mit Hydroxyethylacrylat genannt, wobei ein Polymeres erhalten wird, das als zur Bindung befähigte Gruppen Carboxyl- und Hydroxylgruppen enthält.

Es ist aber auch möglich, die weiter oben beschriebene Polykondensation, die zur Beschichtung des Trägers führt, in Gegenwart des später zu erkennenden Substrats durchzuführen, wobei als Monomere solche Verbindungen eingesetzt werden, die bereits unterschiedliche zur Bindung befähigte Gruppen aufweisen. Vorzugsweise weist dabei jedes Monomere eine dieser Gruppen auf, wobei die Gruppen unterschiedlich sind.

Es ist aber auch möglich, Monomere einzusetzen, die bereits mindestens zwei unterschiedliche zur Bindung befähigte Gruppen aufweisen.

Demzufolge ist diese Ausführungsform auch dadurch gekennzeichnet, dass das Polymere direkt auf dem Träger durch Polymerisation oder Polykondensation mindestens eines Monomeren, das mindestens zwei unterschiedliche zur Bindung befähigten Gruppen aufweist, oder von mindestens zwei Monomeren, die jeweils mindestens eine zur Bindung befähigte Gruppen aufweisen, wobei besagte Gruppen unterschiedlich sind, hergestellt wird, und die Polymerisation oder Polykondensation in Gegenwart des später zu bindenden Substrats stattfindet. Vorzugsweise erfolgt in den Ausführungsformen, in denen die Polymerisation oder Polykondensation besagter Monomerer direkt in Gegenwart des Trägers durchgeführt wird, in Gegenwart mindestens eines zweiten oder dritten Monomeren, das keine zur Bindung befähigte Gruppe besitzt. Das mindestens eine zweite oder dritte Monomere hat dabei die Funktion eines Spacers.

Es ist nicht unbedingt erforderlich, dass die zur Bindung des mindestens bivalenten Substrats an das mindestens eine Sorbens benötigten mindestens zwei unterschiedlichen Gruppen gebundenen an ein Polymeres vorliegen. Es ist auch möglich, die Gruppen ohne Verwendung eines Polymeren in Stufe (ii) direkt auf der Oberfläche des Trägers zu immobilisieren.

Vorzugsweise wird die Immobilisierung direkt auf dem Träger dann durchgeführt, wenn dieser aus einem anorganischen Material aufgebaut ist. Als anorganisches Material sind vorzugsweise Kieselgel oder Aluminiumoxid zu nennen.

Vorzugsweise erfolgt die Immobilisierung über Aktivierungs- und/oder Silanisierungsreagenzien. Die Verküpfung mit der Oberfläche des Trägers kann auch unter Verwendung eines Spacers durchgeführt werden.

Vorzugsweise können als AktivierungsReagenzien die in der WO 00/32648 beschriebenen eingesetzt werden.

Silanisierungsreagenzien umfassen auch vorzugsweise solche Siliciumverbindungen, die eine Hydrosilylierungreaktion eingehen können.

Vorzugsweise werden als Silanisierungsreagenzien Halogensilane, bevorzugt Chlorsilane, Alkoxysilane und Silazane eingesetzt.

Hierbei kann in einer Ausführungsform eine Verbindung, die die zur Bindung des Substrats aufzubringende benötigte Gruppe besitzt, zunächst mit einer geeigneten Siliciumverbindung umgesetzt werden. Das Produkt kann anschließend mit Hydroxyl-Gruppen, die sich auf der Oberfläche des Trägers befinden, unter Ausbildung einer kovalenten Sauerstoff-Silicium-Bindung immobilisiert werden. Beispielsweise können Alkylreste, die auch gegebenenfalls substituiert sein können, beispielsweise mit Amino-, Harnstoff-, Ether-, Amid- und Carbamatgruppen, unter Verwendung alkylierter Silane auf diese Weise auf der Oberfläche immobilisiert werden.

Beispielsweise ist es auf diese Weise möglich, den 3-Aminopropylrest über ein Siliciumatom auf der Oberfläche des Trägers zu immobilisieren. Die Aminogruppe kann dann weiter umgesetzt werden, beispielsweise mit Säurechloriden zu Amiden. Es können aliphatische, vorzugsweise aber aromatische Säurechloride verwendet werden, sowie aktivierte Bausteine, insbesondere ONB-aktivierte Bausteine, wie sie beschrieben sind in der WO 00/32649 und WO 00/78825.

Beispiele für Siliciumverbindungen, mit denen Alkylreste auf den Träger aufgebracht werden können, sind Methyl- und Octyltrichlorsilan, mit denen relativ kurze bzw. mittlere Alkylreste eingeführt werden können, sowie Octadecyl-, Docosyl- und Tricontyltrichlorsilan, mit denen relativ lange Ketten eingeführt werden können. Mit 3-Aminopropyltriethoxysilan ist beispielsweise die Einführung eines eine Aminogruppe enthaltenden kurzkettigen Alkylrests möglich.

Ferner ist auch die Verwendung von Silylglycidylethern möglich, die nach Hydrolyse Diole bilden, die auch als Diolphasen bezeichnet werden.

Andererseits ist es auch möglich, zunächst die Oberfläche des Trägers mit einer Siliciumverbindung umzusetzen, die noch eine oder mehrere funktionelle Gruppen besitzt. Anschließend können die zur Bindung ausgewählten oder ermittelten Gruppen, die auf dem Träger immobilisiert werden sollen, über geeignete Verbindungen über die eine oder mehrere funktionellen Gruppen eingeführt werden.

Beispielsweise können zum Aufbringen auf die Oberfläche des Trägers Siliciumverbindungen verwendet werden, die noch eine Doppelbindung besitzen. Über besagte Doppelbindung können dann die die zur Bindung vorgesehenen Gruppen eingeführt werden können. Beispiele für geeignete Siliciumverbindungen sind Vinylsilan oder (Meth)Acryloxypropyltrimethoxysilan.

Die beschriebenen Methoden können auch in Kombination verwendet werden.

Gegebenenfalls kann die Anbindung der zur Bindung vorgesehenen Gruppen auch über einen Spacer erfolgen, wobei vorzugsweise eine kurzkettige Kohlenstoffkette zwischen der zu immobilisierenden Gruppe und dem Träger eingebaut wird. Die Verknüpfung von Träger und zu immobilisierender Gruppe kann vorzugsweise über geeignete Carbodiimide, wie beispielsweise Dicyclocarbodiimid, Diisopropylcarbodiimid, N-Cyclohexyl-N'-2-(N-methylmorpholino)-ethylcarbodiimid-p-toluolsulfonat, N-Ethyl-N'-(3-dimethylaminopropyl)carbo-diimid-hydrochlorid, Chloroformiate, Carbonyldiimidazole oder Diisocyanate, wie Hexamethylendiisocyanat, erfolgen. Auch homo- oder heterotelechele Polyethylenglykole können verwendet werden.

Bei Verwendung eines Spacers entsteht vorzugsweise eine bürstenförmige Phase, bei der die mindestens zwei unterschiedlichen zur Bindung befähigten Gruppen vorzugsweise entweder am Ende des Spacers und/oder seitlich am Spacer gebunden sind.

In einer weiteren Ausführungsform erfolgt in Stufe (ii) das Aufbringen der mindestens zwei unterschiedlichen zur Bindung mit einem zweiten Substrat befähigten Gruppen über ein Reagenz auf einen polymerbeschichteten Träger aufgebracht wird, das ausgewählt wird aus der Gruppe umfassend AktivierungsReagenzien, SilanisierungsReagenzien und Spacer, oder Gemische aus zwei oder mehreren dieser Reagenzien.

Es hat sich für die substratspezifische Bindung als ungünstig erwiesen, Sorbentien einzusetzen, die als die mindestens zwei unterschiedlichen zur Bindung befähigten Gruppen die beim Stand der Technik beschriebenen Gruppen aufweisen, nämlich Hydroxylgruppen aus dem Kieselgelgerüst bzw. Silanol- und Alkylgruppen, die über das Silanisierungsreagenz eingebracht werden. Somit ist eine Kombination aus den Gruppen Hydroxyl, Silanol und Alkyl oder Hydroxyl und Alkyl oder Silanol und Alkyl, wobei die Gruppen jeweils an Kieselgel immobilisiert sind, von der Erfindung ausgeschlossen.

Besonders gut geeignete Gruppen im Sinne der Erfindung sind hingegen Gruppen wie der Phenyl-, der Hydroxyphenyl-, der Carboxyl-, der Amin- und der Amid-Rest sowie der Hydroxyl-, Indol-, Imidazol- und Guanidin-Rest. Vorzugsweise werden diese Reste über einen Spacer unter Ausbildung einer bürstenförmigen Phase an die Oberfläche des Trägers gebunden.

Eine besonders bevorzugte Ausführungsform ist demzufolge dadurch gekennzeichnet, dass in Stufe (ii) die mindestens zwei unterschiedlichen zur Bindung mit einem zweiten Substrat befähigten Gruppen ausgewählt sind aus der Gruppe bestehend aus Phenyl-, Hydroxyphenyl-, Carboxyl-, Amin-, Amid-, Hydroxyl-, Indol-, Imidazol- und Guanidin-Rest.

Das nach den vorstehend beschriebenen Methoden hergestellte mindestens eine Sorbens kann nach den üblichen Methoden zu vorzugsweise Folien, Filme, Mikrotiterplatten oder Nanobeads verarbeitet werden. Vorzugsweise wird das mindestens eine Sorbens der Stufe (ii) im Nanoformat hergestellt und verwendet.

Das zu bindende Substrat bzw. das aus einem Substratgemisch selektiv zu bindende Substrat wird nun in Stufe (iii) mit dem Sorbens der Stufe (ii) in Kontakt gebracht. Dabei kann das Substrat bzw. das Substratgemisch in fester, flüssiger oder gasförmiger Phase vorliegen, oder auch in Mischungen aus zwei oder mehr dieser Phasen.

Vorzugsweise liegen Substrat bzw. Substratgemisch in flüssiger Phase vor. Einsetzbar sind dabei Lösungen wie auch Suspensionen oder Dispersionen des Substrats bzw. Substratgemischs. Als Flüssigkeiten können sowohl Wasser wie auch organische Lösungsmittel, Gemische organischer Lösungsmittel und Gemische umfassend Wasser und organische Lösungsmittel verwendet werden. In allen Fällen können Puffer, Salze, Säuren, Basen oder Modifier, wie beispielsweise Ionenpaarreagenzien, in beliebiger Konzentration in der Flüssigkeit vorhanden sein. Vorzugsweise

ist die Konzentration 10 mmolar bis 2 molar bezogen auf einen Liter Flüssigkeit. Vorzugsweise liegt das zu bindende Substrat in wässeriger Form vor, beispielsweise als Körperflüssigkeit.

Zur Prüfung der Bindung bzw. des Bindungsverhaltens des Substrats an das Sorbens können die bekannten Verfahren und Methoden herangezogen werden. Unter Bindung zwischen Sorbens und Substrat wird dabei vorzugsweise die nicht-kovalente Bindung verstanden.

Unter nicht-kovalenter Bindung sind dabei vorzugsweise die eingangs beschriebenen Wechselwirkungen zu verstehen.

Es ist jedoch auch möglich, dass das mindestens eine Substrat kovalent-reversibel oder kovalent-irreversibel an das mindestens eine Sorbens gebunden wird.

Vorzugsweise eignen sich in Stufe (iv) zur Prüfung der Bindungsstärke des Substrats an das Sorbens der Stufe (iii) chromatographische Verfahren und Auswertungsmethoden. Insbesondere zu nennen sind säulenchromatographische Verfahren, beispielsweise die bekannte HPLC-Methode. Das mindestens eine Sorbens wird hierbei als stationäre Phase der Säule verwendet. Aus der Reihenfolge der eluierten Substrate kann unmittelbar auf deren Bindungsstärke an das jeweils verwendete Sorbens geschlossen werden. Das am stärksten gebundene Substrat wird zuletzt eluiert.

Es können Frontalanalysen durchgeführt werden, bei der verdünnte Lösungen des zu trennenden Substratgemischs kontinuierlich auf die stationäre Phase aufgegeben werden. Das am stärksten gebundene Substrat kann so von den weniger stark gebundenen unterschieden werden, da letztere zuerst im Eluat erscheinen.

Aber auch die bekannten Elutionstechniken sind durchführbar, wobei relativ konzentrierte Lösungen des Substratgemischs auf den Säulenkopf aufgegeben und dann mit einem Fließmittel eluiert werden. Die schwach gebundenen Substrate erscheinen zuerst im Eluat. Das am stärksten gebundene Substrat kann dann gegebenenfalls auch durch Verwendung eines stärker eluierenden Fließmittels vom Sorbens desorbiert werden.

Vorteilhaft kann auch die Mikrocalorimetrie eingesetzt werden. Hierbei wird die bei der Bindung des Substrats an das Sorbens frei werdende Adsorptionswärme gemessen.

Eine weitere vorteilhaft anwendbare Methode ist die Surface Plasmon Resonance-Methode, bei der die Resonanzfrequenz anregbarer Elektronen bestimmt wird, die von den physikalischen Eigenschaften der Grenzschicht von Substrat und Sorbens, also auch der Bindungsstärke, abhängig ist.

Vorteilhaft kann auch als Prüfmethode Fluoreszenz-Labeling zum Einsatz kommen, wobei die mit einem fluoreszierende Farbstoff markierten Substrate nur dann fluoreszieren, wenn sie mit dem komplementären Rezeptor wechselwirken.

Eine weitere Methode ist die Enzyme Linked Immunosorbent Assay-Methode (Elisa), bei der beispielsweise Antigene, die an das Sorbens gebunden sind, durch Behandeln mit Immunreagenzien nachgewiesen werden können. Auch kompetitive und nicht-kompetitive Assays sind einsetzbar, darunter Radioassays.

Demgemäß ist diese Ausführungsform der Erfindung dadurch gekennzeichnet, dass zur Prüfung der Bindungsstärke des Substrats an das Sorbens in Stufe (iv) ein Verfahren ausgewählt aus der Gruppe umfassend Chromatographie, Mikrocalorimetrie, Surface Plasmon Resonance, Fluoreszenz-Labeling, kompetitive und nicht-kompetitive Assays, darunter Radioassay und Elisa verwendet wird.

Aus der Bindungsstärke kann eine Aussage getroffen werden, welches der Sorbentien beziehungsweise welche der darauf aufgebrachten Gruppen für die Bindung des Substrats verantwortlich sind. Diese Methode erlaubt somit, das betreffende Substrat zu isolieren, zu identifizieren und zu charakterisieren. Damit ist die Validierung von Funktion und Eigenschaften des Substrats möglich.

Demzufolge ist das Verfahren zur selektiven bivalenten Bindung besagten Substrats auch dadurch gekennzeichnet, dass es zusätzlich die Stufe (v) umfasst:

(v) Isolierung des Substrats.

Weiter ist das Verfahren zur selektiven bivalenten Bindung besagten Substrats auch dadurch gekennzeichnet, dass es zusätzlich die Stufe (vi) umfasst:

(vi) Charakterisierung und Identifizierung des Substrats.

Das neue Verfahren eigenet sich insbesondere zur selektiven Bindung von natürlichen Substraten oder natürlichen Wirkstoffen sowie synthetischer Wirkstoffe. Diesen Substraten und Wirkstoffen ist gemeinsam, dass sie einen Pharmakophor besitzen, also eine räumliche Anordnung von Gruppen, die die Grundlage für die biologische Wirkung in lebenden Organismen bilden. Der Pharmakophor verankert den Wirkstoff in der Bindetasche des natürlichen Rezeptors. Der Pharmakophor ist an ein Gerüst, das in der angelsächsischen Literatur auch als <u>scaffold</u> bezeichnet ist, verankert.

Natürliche Substrate und Wirkstoffe umfassen vorzugsweise Aminosäuren, Oligopeptide, Nukleotide, Nukleoside, Proteine, Glukoproteine, Antigene, Antigen-Determinanten, Antikörper, Kohlenhydrate, Enzyme, Co-Enzyme, Fermente, Hormone, Alkaloide, Glykoside, Steroide, Vitamine, Metabolite, Viren, Mikroorganismen, Inhaltstoffe pflanzlicher und tierischer Gewebe, Zellen, Zellfragmente, Zellkompartimente, Zellaufschlüsse, Lectine, Flavyliumverbindungen, Flavone und Isoflavone.

Im Rahmen der Erfindung ist es besonders interessant, natürliche Rezeptoren und Enzyme oder sonstige Proteine mit pharmakologischer Wirksamkeit zu zerlegen, erfindungsgemäß mit ihrer Hilfe eine Kollektion von Sorbentien zu erstellen und erfindungsgemäß zu verwenden. Derartige Rezeptoren sind vorzugsweise intrazelluläre oder membranständige Proteine, die synthetische oder natürliche Wirkstoffe zu binden vermögen.

Intrazelluläre Rezeptoren können aus Zytoplasma und aus Zellkernen erhalten werden. Solche Rezeptoren bzw. Sorbentien, die mindestens zwei bindende Gruppen dieser Rezeptoren enthalten, können zur selektiven Bindung von Steroidhormonen, wie Glucocorticoiden, Mineralocorticoiden, Androgenen, Estrogenen, Gestagenen, Vitamin-D-Hormonen, sowie von Retinoiden oder Schilddrüsenhormonen verwendet werden.

Membranständige Rezeptoren, deren bindende Gruppen erfindungsgemäß auf Sorbentien aufgebracht werden können, sind Guanin-Nukleotid-Proteingekoppelte Rezeptoren, Ionenkanal-Rezeptoren sowie Enzym-assoziierte Rezeptoren.

Zu der Gruppe der Guanin-Nukleotid-Protein-gekoppelten Rezeptoren gehören für die medikamentöse Therapie besonders wichtige Neurotransmitter-Rezeptoren, wie Adenosin- und adrenergene Rezeptoren, ATP-(P2Y-)-, Dopamin-, $GABA_B$-, (metabotrope) Glutamat-, Histamin-, Muscarin-, Opioid- und Serotonin-Rezeptoren. Auch Hormon- und Mediator-Rezeptoren, z. B. von Adiuretin, Glucagon, Somatostatin und Prostaglandine, zählen zu dieser Gruppe.

Ionenkanal-Rezepzoren umfassen ATP-(P2X-), $GABA_A$-, (ionotrope) Glutamat-, Glycin-, 5-$HT_3$- und Nicotin-Rezeptoren.

Zu den Enzym-assoziierten Rezeptoren zählen Rezeptoren mit TyrosinkinaseAktivität, mit assoziierten Tyrosinkinasen, mit Guanylatcyclase-Aktivität sowie Rezeptor-Serin-/Threoninkinasen.

Synthetische Wirkstoffe umfassen vorzugsweise Pharmazeutika und Pflanzenschutzmittel.

Pharmazeutika sind beispielsweise Substanzen mit Wirkung auf das Nervensystem (Psychopharmaka, Schlafmittel, Analeptika, Analgetika, Lokal- und Allgemeinanästhetika, Muskelrelaxantien, Antikonvulsiva, Antiparkinsonmittel, Antimetika, ganglionär angreifende Substanzen, am Symphatikus angreifende Substanzen, am Parasympatikus angreifende Substanzen); mit Wirkung auf das Hormonsystem (Hypothalmus-, Hypophysen-, Schilddrüsen-, Nebenschilddrüsen- und Nierenhormone, Thymushormone, das Inselorgan des Pankreas-, der Nebennieren-, der Gonaden-beeinflussende Substanzen); mit Wirkung auf Mediatoren (Histamin, Serotonin, Eicosanoide, Plättchen-aktivierende Faktoren, Kinine); mit Wirkung auf das Herz-Kreislauf-System; mit Wirkung auf den Respirationstrakt (Antiasthmatika, Antitussiva, Expetorantien, Surfactant); mit Wirkung auf den Magen-Darm-Kanal (Verdauungsenzyme, Hepatika); mit Wirkung auf die Niere und ableitende Harnwege (Diuretika); mit Wirkung auf das Auge (Ophtalmika); mit Wirkung auf die Haut (Dermatotherapeutika); Substanzen zur Prophylaxe und Therapie von Infektionserkrankungen (antibakteriell wirksame Pharmaka, Antimykotika, Chemotherapeutika für Viren- und Protozoenerkrankungen, Anthelminthika); mit Wirkung auf maligne Tumoren (Antimetaboliten, Zytostatika, Topoisomerase-Hemmstoffe, Mitosehemmstoffe, zytostatisch wirksame Antibiotika, Hormone und Hormonantagonisten); mit Wirkung auf das Immunsystem und immunologisch wirksame Stoffe (Seren, Immunmodulatoren, Immunsuppressiva).

Pflanzenschutzmittel sind beispielsweise Insektizide, Herbizide, Pestizide und Fungizide.

Als beispielhafte Verbindungen und Verbindungsklassen synthetischer Wirkstoffe seien genannt Phenothiazine und Phenothiazinanaloge, Butyrophenone und Diphenylbutylpiperidine, Benzamide, Benzodiazepine, Hydoxytryptophane, Coffeine, Amphetamine, Opioide und Morphine, Phetidine und Methadone, Salicyl-und Acetylsalicylsäurederivate, Arylpropionsäurederivate, Anthranilsäurederivate, Anilinderivate, Pyrazolderivate, Sulfapyridine, Hydroxychloroquin und Chloroquin, Penicillamin, N-methylierte Barbiturate und Thiobarbiturate, Dipropylessigsäuren, Hydantoine, Dopamine, Noradrenolin und Adrenolin, Mutterkornalkaloide, Carbaminsäure-Derivate, Phosphorsäureester, Belladonna-Alkaloide, Hypophthalmushormone, HVL-Hormone, Hypophysenhinterlappenhormone, Thiouracile und Mercaptoimidazole, Sulfonylharnstoffe, Histamine, Triptane, Prostaglandine, Dipyradimole, Hirudin und Hirudinderivate, Thiazide, Psoralene, Benzoylperoxid und Azeleinsäure, Vitamin A, Vitamin K, Vitamin $B_1$, $B_2$, $B_6$, Nicotinsäureamid, Biotin, Vitamin $B_{12}$, Vitamin C, Halogenverbindungen, Aldehyde, Alkohole, Phenole, N-haltige Heterocyclen, Pyrethrine und Pyrethroide, Phosphorsäureester, Thiophosphorsäureester, Carbaminsäureester, $\beta$-Lactame, Aminogylcoside, Tetracycline, Fluorchinolone, Oxazolidinone, Diaminobenzylpyrimidine, Pyrazinamide, Griseofulvin, Aziridine, Actinomycine, Anthracycline, Zytokine, monoklonale und polyklonale Antikörper. Ferner können Antigen-Determinanten, Lectine, Flavyliumverbindungen, Flavone und Isoflavone sowie Mono- und Oligosaccharide genannt werden.

Die synthetischen Wirkstoffe können auch unter Verwendung natürlicher Wirkstoffe aufbereitet sein. Ferner umfasst der Begriff auch potenzielle Wirkstoffe sowie Substanzen, die Pharmakophore tragen, sowie das Gerüst (scaffold), an dem besagte Pharmakophore verankert sind.

Wie eingangs bereits erwähnt, ist das neue Verfahren zur selektiven bivalenten Abtrennung besagten Substrats insbesondere dazu geeignet, Informationen darüber zu erhalten, ob ein beliebiges Substrat mit einem natürlichen Rezeptor überhaupt in Wechselwirkung treten kann. Umgekehrt ist es auch möglich, unter Verwendung von z. B. allen für

die Substraterkennung relevanten Gruppen Bibliotheken von synthetischen molekularen Bereichen, also Epitopen, herzustellen, deren Bestandteile jeweils zwei, drei oder auch mehr unterschiedliche Wechselwirkungstellen enthalten. Wenn man z. B. einen bekannten Wirkstoff in Kontakt mit diesen synthetischen Rezeptorbibliotheken bringt, wird eine Wahrscheinlichkeitsaussage erhalten über die Art der Bindungsstelle am natürlichen Rezeptor.

Bei der Erfindung wird somit ein neues Komplementaritätsprinzip angewendet, das auf Seiten des Rezeptors bzw. Sorbens und auf Seiten des Substrats jeweils mindestens zwei unterschiedliche Reste von Verbindungen oder Gruppen, die in Verbindungen für die Bindung verantwortlich sind, umfassen. Vorzugsweise werden dabei die Verbindungen ausgewählt aus der Gruppe umfassend Aminosäuren, Zucker, Nukleotide, Nukleoside, Pyrimidin- und Purinbasen.

Von allen möglichen Kombinationen dieser bivalenten molekularen Bereiche untereinander ist allerdings nur eine kleine Auswahl kompatibel komplementär, d. h. in ihrer Wechselwirkung energetisch günstig. Die Mehrzahl der Kombinationen ist energetisch ungünstig, z. B. alle Paarungen von hydrophoben Resten einerseits und hydrophilen Resten andererseits, oder alle Reste, die sich abstoßen.

Kompatibel sind beispielsweise die Kombinationen der zur Bindung befähigten paarweisen Gruppen OH-/Phenyl- mit Amino-/Alkyl-Rest, aber nicht OH-/Phenyl- mit Alkyl-/Amino-Rest, da nur die hydrophilen OH- und Amino-Reste sowie die hydrophoben Phenyl- und Alkylreste einander binden. Weitere kompatible Kombinationen sind beispielsweise Carboxyl-/Amino- mit Amino-/Carboxylrest sowie Imidazol-/Hydroxyl- mit Amid-/Amid-Rest. Nichtkompatibel im Sinne dieser Betrachtung ist die Kombination Hydroxyl-/Phenyl-mit Alkyl-/Amino-Rest, da ein hydrophiler keinen hydrophoben Rest zu binden vermag.

Bei zwanzig natürlichen Aminosäuren ergeben sich für Dublette von Bausteinen mit jeweils mindestens einer zur Bindung befähigten Gruppe insgesamt 380 Varianten. Für eine Bibliothek, die lediglich die sinnvollen Strukturvarianten beinhaltet, braucht man allerdings wesentlich weniger dieser synthetischen Dublette von Bausteinen, die man auch als Dublett-Rezeptoren bezeichnen kann, weil bei einer Reihe von Aminosäuren die Funktionalität gleich ist, wie z. B. bei Threonin und Serin, bei Glutamin und Asparagin, bei Valin, Isoleucin und Leucin, usw.. Es ist daher im Allgemeinen ausreichend, von diesen zwanzig Aminosäuren vorzugsweise lediglich bis zu sieben zu verwenden.

Da die beweglich angebrachten Rezeptorgruppen im synthetischen Rezeptor ihre Raumkoordinaten entsprechend der Anforderungen des Substrats verändern können, werden häufig für den gewünschten Bindungszweck nicht die Aminosäuren selbst mit ihren unterschiedlich langen Ketten benötigt, sondern nur das für die Wechselwirkung benötigte Prinzip. In diesem Sinn sind oftmals die Funktionen von beispielsweise Arginin, Lysin, Tryptophan und Histidin einfach durch Aminogruppen darstellbar, vorausgesetzt, es wird nur die Basenfunktion benötigt.

Werden beispielsweise von sieben Aminosäuren lediglich vier Aminosäuren oder das Prinzip dieser Aminosäuren im Sinne der Erfindung verwendet, so ergeben sich nach Permutation insgesamt lediglich 35 verschiedene Kombinationen an Dublett-Rezeptoren.

Denkbar ist somit eine kombinatorische Bibliothek umfassend eine Kollektion von Sorbentien mit jeweils mindestens zwei unterschiedlichen Gruppen, die zur Bindung mindestens eines Substrats mit mindestens zwei unterschiedlichen Gruppen geeignet sind, wobei die jeweils mindestens zwei unterschiedlichen Gruppen der Sorbentien und die des mindestens einen Substrats zueinander komplementär sind,wobei die mindestens zwei unterschiedlichen Gruppen der Sorbentien und die mindestens zwei unterschiedlichen Gruppen des mindestens einen Substrats ausgewählt sein können unter Gruppen, die Bestandteile unterschiedlicher Aminosäuren, Zucker, Nukleotide, Nukleoside, Pyrimidin- oder Purinbasen sind.

Die kombinatorische Bibliothek ist dadurch gekennzeichnet, dass die Herstellung der Sorbentien die Stufen (i) und (ii) umfasst:

(i) Ermittlung von mindestens zwei unterschiedlichen Gruppen, die zur Bindung eines ersten synthetischen oder natürlichen Substrats an ein Sorbens befähigt sind,

(ii) Aufbringen jeweils mindestens zweier unterschiedlicher zur Bindung mit einem zweiten synthetischen oder natürlichen Substrat befähigten Gruppen auf jeweils einen Träger unter Bildung mindestens eines Sorbens, wobei es sich bei den Gruppen um Gruppen handelt, die zu denen der Stufe (i) gleich oder komplementär sind, und das zweite Substrat der Stufe (ii) gleich oder verschieden vom ersten Substrat gemäß Stufe (i) ist.

Bei der selektiven Abtrennung des Substrats wird ein Sorbens-Substrat-Komplex erhalten, der mindestens ein Sorbens mit mindestens zwei unterschiedlichen zur Bindung befähigten Gruppen und mindestens ein Substrat mit mindestens zwei unterschiedlichen zur Bindung befähigten Gruppen umfasst, wobei die zur Bindung befähigten Gruppen des mindestens einen Sorbens und die des mindestens einen Substrats zueinander komplementär sind, und wobei die mindestens zwei unterschiedlichen Gruppen des mindestens einen Sorbens und die mindestens zwei unterschiedlichen Gruppen des mindestens einen Substrats unterschiedliche Gruppen, die Bestandteil von Aminosäuren, Zuckern, Nukleotiden, Nukleosiden, Pyrimidin- oder Purinbasen sind, umfasse.

Im Sorbens-Substrat-Komplex besteht die Bindung zwischen dem mindestens einen Sorbens und Substrat in einer nicht-kovalenten, kovalent-reversiblen oder kovalent-irreversiblen Bindung. Vorzugsweise ist die Bindung nicht-kovalent reversibel.

Denkbar ist auch die Verwendung des neuen Verfahrens zur selektiven Bindung eines Substrats an Sorbentien durch mindestens bivalente Bindung und die Verwendung der kombinatorischen Bibliothek.

Eine Verwendungsmöglichkeit besteht in der Detektion von Rezeptor-Wirkstoff-Wechselwirkungen sowie dem Wirkstoff-Screening.

Für die Detektion von Rezeptor-Wirkstoff-Wechselwirkungen sowie für das Wirkstoff-Screening können die oben aufgeführten Wirkstoffe bzw. Wirkstoffklassen eingesetzt werden.

Auch für die Entwicklung neuer Wirkstoffkandidaten (Leadsubstanzen) kann die Erfindung vorteilhaft verwendet werden. Diese Leadsubstanzen können bezüglich ihrer Wirkung, Selektivität, Bioverfügbarkeit, Pharmakokinetik und Toxizität unter Verwendung des neuen Verfahrens bzw. der kombinatorischen Bibliothek optimiert werden.

Dabei ist es auch denkbar, dass Wirkstoffkandidaten nur mit einem Abschnitt der biologischen Bindungsstelle in Wechselwirkung treten. Durch Kombination und Verbindung von mindestens zwei solchen Wirkstoffkandidaten, die an wenigstens zwei Abschnitten der biologischen Bindungsstelle binden, kann man auf einfache Weise neue Wirkstoffe finden. Diese Wirkstoffsuche funktioniert auch unter Verwendung einer hochparallelisierter Verfahrensdurchführung.

Eine weitere Anwendungsmöglichkeit liegt in der Trennung stereoisomerer und strukturisomerer Verbindungen.

Ferner ist die Reinigung und/oder Trennung von Substraten und Substratgemischen möglich.

Die Reinigung und/oder Trennung kann durch chromatographische Methoden erfolgen. Als weitere geeignete Methoden können Elektrophorese, Elektrofokusierung, Flachgelelektrophorese, Parallelchromatographie, parallele Flashchromatographie und Kapillartechniken genannt werden. Bei ausreichend hoher Selektivität kann ein Substrat auch direkt aus dem gelösten Gemisch durch Zugabe des Sorbens adsorbiert, ausgerührt und in Form eines Sorbens-Substrat-Kompexes abfiltriert werden.

Weitere Anwendungsmöglichkeiten bestehen in der Entfernung von Schadstoffen und Abbauprodukten aus Stoffgemischen, wobei die Substanzen auch in sehr niedriger Konzentration vorliegen können.

Ferner können Schadstoffe und Abbauprodukte aus Körperflüssigkeiten, wie Blut, abgetrennt werden. Solche Schadstoffe und Abbauprodukte liegen beispielsweise bei Vergiftungen, als Stoffwechselprodukte oder Metabolite vor. Sie können biogener Natur sein und im Körper selbst gebildet, diesem aber auch von außen zugeführt worden sein, beispielsweise über die Haut, über die Mundschleimhäute oder durch Injektion, beispielsweise in die Blutbahn. Zu Schadstoffen und Abbauprodukten sind auch Schlangengifte und Rauschmittel zu zählen.

Zudem können die Sorbentien in Dialyseeinrichtungen eingesetzt werden.

Ferner ist die Entfernung von Schadstoffen aus Lösungsmitteln, aus Prozesswässern und aus Prozessen zur Lebensmittelherstellung möglich.

Mit Hilfe der Erfindung können auch pharmakokinetische Untersuchungen, insbesondere zur Metabolisierung und Bioverfügbarkeit durchgeführt werden.

Das neue Verfahren zur selektiven bivalenten Bindung kann auch vorteilhaft zur Abreicherung dynamisch kombinatorischer Bibliotheken verwendet werden. Hierbei wird zweckmäßigerweise so vorgegangen, dass aus einer Mischung, die neben einer Vielzahl von Edukten auch gewünschtes Substrat, vorzugsweise einen Wirkstoff, enthält, letzteres im Sinne der Erfindung abgetrennt wird. Daraufhin wird sich in der Mischung das Gleichgewicht unter Bildung weiteren Substrats neu einstellen. Der Vorgang der Abtrennung wird dann so lange wiederholt, bis kein Substrat mehr gebildet wird.

Wie vorstehend erläutert, wird das neue Verfahren zur gezielten und selektiven Abtrennung eines Substrats aus einem Gemisch mit wenigstens einem weiteren Substrat verwendet.

Unter dem Begriff selektive Bindung wird somit erfindungsgemäß verstanden, dass ein Substrat aus einem Gemisch mit mindestens einem weiteren begleitenden Substrat dadurch abgetrennt wird, dass das mindestens zwei unterschiedliche Gruppen aufweisende Substrat mit den auf dem Sorbens befindlichen mindestens zwei unterschiedlichen Gruppen eine stärkere Bindung eingeht als das begleitende Substrat.

Mit der vorliegenden Erfindung ist erstmals der Wechselwirkungsort und die Wechselwirkungsart u. a. durch die nachfolgenden Vorgehensweisen exakt definierbar, wie an Hand der Beispiele ersichtlich ist:

- durch gezieltes Einbringen von Bindungsstellen am Rezeptor in der gewünschten Konzentration und Kombination,
- durch Weglassen, Hinzufügen, Variieren oder Blockieren einzelner Bindungsstellen sowohl am Rezeptor als auch an Testsubstraten, wobei die Auswirkung auf die Bindungsstärke jeweils genau (=energetisch) bestimmt wird,
- durch spektroskopische Untersuchungen und durch Bestimmung der Adsorptionsisothermen.

Beispielsweise kann durch Vergleich mit den literaturbekannten Einzelbeiträgen der jeweiligen nicht- kovalenten

Bindungsarten die Gesamtwechselwirkung einer multivalenten Bindung überraschend gut vorhergesagt werden. Wenn die jeweilige Bindungsenergie in der erwarteten Höhe bestimmt wird, ist umgekehrt der Schluss auf die an der Bindung beteiligten Gruppen möglich.

Damit kann erstmals gezielt Selektivität erzeugt werden hinsichtlich eines beliebig ausgewählten Trennproblems.

Die neue Lehre beinhaltet bezüglich der zu isolierenden Zielverbindung (Substrats) das Konstruieren einer zweckgerichteten nicht-kovalent multivalenten Wechselwirkung, die sich hinreichend unterscheidet von den nicht-kovalenten Wechselwirkungen mit den konkurrierenden Substraten (Begleitstoffen).

Die Verfahren der vorliegenden Erfindung zeigen hohe Werte für die Trennselektivität, die auch einfach als Selektivität bezeichnet wird. Die Trennselektivität $\alpha$ ist dabei definiert als Quotient der jeweiligen Bindungskonstanten bzw. Kapazitätsfaktoren der Bindung des selektiv abzutrennenden Substrats an das Sorbens und der Bindungskonstanten der Bindung des begleitenden Substrats an das Sorbens.

Die Trennselektivität erreicht bei Weglassen beispielsweise einer einzigen Carboxylgruppe in einem Substrat einen Wert von über 35. Beim Tausch eines aromatischen Ein-Ring-Systems in ein Drei-Ring-System wird ein Wert von 10 erhalten.

Mit dem neuen Verfahren können Trennselektivitäten im technischen Maßstab erzielt werden, die im Vergleich zum Stand der Technik überraschend hoch sind und nicht selten Trennungen ermöglichen, die bislang chromatographisch nicht möglich waren.

Erfindungsgemäß ist die Trennselektivität $\alpha$, mit der das selektiv zu bindende Substrat mit den mindestens zwei unterschiedlichen zur Bindung an mindestens ein Sorbens befähigten Gruppen unter Verwendung des mindestens einen Sorbens aus einem Substratgemisch abgetrennt wird, größer als 1,4.

Bevorzugt ist eine Trennselektivität $\alpha$ von größer als 2, weiter bevorzugt von größer als 4, noch weiter bevorzugt von größer als 8.

Weitere bevorzugte Trennselektivitäten sind größer als 10, weiter bevorzugt größer als 35.

Da außerdem die Bindungskonstanten direkt in die dem Fachmann bekannten Bestimmung der Gibbs-Energie eingehen, ist auch ein Zusammenhang zwischen Gibbs-Energie und der Trennselektivität gegeben. Je negativer die Änderung der Gibbs-Energie $\Delta G$ für die nicht-kovalente Bindung ist, d.h. je stärker der komplementäre Charakter der einander bindenden Gruppen ausgeprägt ist, desto größer ist auch die Trennselektivität gegenüber Begleitstoffen, die sich durch die Einführung der hier in Rede stehenden zur Bindung (mit den Zielsubstanzen) befähigten Gruppen nicht nennenswert bzgl. der Gibbs-Energien (zu diesen Gruppen bzw. den Sorbens) verändern.

Darüber hinaus reicht es zur Erzeugung von Selektivität bezüglich eines beliebigen Substratpaares aus, eine einzige zur Bindung befähigte Gruppe <u>zusätzlich</u> im Sorbens anzubringen, soweit diese Gruppe bei einer der beiden zu trennenden Substraten keinen komplementären Partner hat.

Auf die beschriebene Weise kann auch nachgewiesen werden, ob und welche Bindungsarten gleichzeitig (d. i. Mehrvalenz) vorkommen. Diese Mehrvalenz, insbesondere die erzielten Sollwerte für die Bindungskonstanten und Gibbs-Energien sind jedoch nur dann möglich, wenn das Substrat durch Induced Fit zumindest teilweise räumlich eingeschlossen werden kann oder sich konformativ an den Rezeptor anpaßt.

Diese Anpassung ist <u>bevorzugt mit dem polymeren Netzwerk</u> möglich, wobei der Vernetzungsgrad des polymeren Nanofilms so gewählt wird, dass noch ausreichend konformative Beweglichkeit und somit Anpassungsfähigkeit an die Substratstruktur gegeben ist. Kleine Substrate mit Molmassen unter 1000 Dalton werden vorzugsweise komplett im Polymernetzwerk eingeschlossen. Größere Substrate wie z. B. Peptide oder Proteine binden vorzugsweise mit begrenzter Kontaktfläche in einer Vertiefung im Polymernetz, die eine mehrvalente Wechselwirkung zuläßt, aber zu starke Bindung durch Einschluss vermeidet.

Um das Konzept zur Konstruktion von selektiven multivalenten Bindungsstellen zu realisieren, ist es häufig notwendig, die benötigten Bindungspunke konformativ beweglich im Raum anzubieten. Darüber hinaus ist es notwendig, durch das Substrat eine ausreichend starke Bindungstendenz anzubieten, um die konformative Anpassung (Induced Fit) zu erreichen. Nicht zuletzt müssen die mindestens zwei notwendigen Wechselwirkungsstellen in hoher Konzentration im Raum präorganisiert sein, damit das gewünschte Bindungsereignis aufgrund der Konformationsänderung in großer Zahl realisiert wird.

Die Erfindung soll durch die nachstehenden Beispiele erläutert werden.

**Beispiel 1: Selektive Bindung N-blockierter Aminosäuren als Substrate an Sorbentien auf Basis Polyvinylamin/Kieselgel durch mindestens bivalente Bindung**

Es wurden die Retentionseigenschaften von acht verschiedenen Aminosäurederivaten (Substrate in Tabelle 1) an vier verschiedenen Sorbentien auf Basis Polyvinylamin/Kieselgel (Sorbentien in Tab. 2) untersucht, wobei als Analysenmethode die Chromatographie gewählt wurde. Die Sorbentien werden nachfolgend auch als stationäre Phasen, synthetische Rezeptoren oder auch als Rezeptoren bezeichnet, auch in den anderen Beispielen.

Bei den Aminosäurederivaten handelte es sich um Glutamin- (1-4) und Glutamat-Derivate **(5-8),** deren Aminogruppen jeweils mit den vier verschiedenen Schutzgruppen Acetyl- (Ac), tert.-Butyloxycarbonyl- (Boc), Benzyloxycarbonyl- (Z) sowie Fluorenylmethoxycarbonyl- (Fmoc) blockiert waren.

| **Tab. 1: Verwendete Substrate** | | | |
|---|---|---|---|
| **Glutaminderivate** | **Glutamatderivate** | **N-Schutzgruppe** | |
| 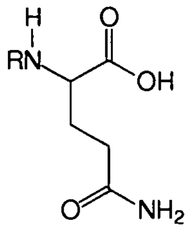 | 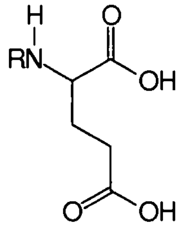 | | |
| Ac-Gln **1** | Ac-Glu **5** | Ac | 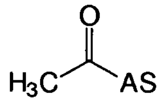 |
| Boc-Gln **2** | Boc-Glu **6** | Boc | 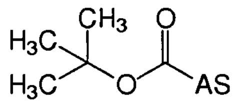 |
| Z-Gln **3** | Z-Glu **7** | Z | 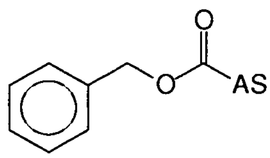 |
| Fmoc-Gln **4** | Fmoc-Glu **8** | Fmoc | 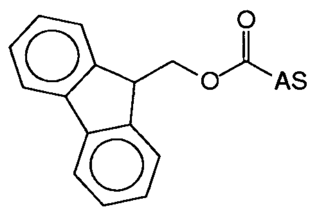 |

Bei den verwendeten Rezeptor-Phasen handelte es sich um Polyvinylaminbeschichtetes, sphärisches Kieselgel mit 20 μm Partikelgröße und 1000 Å Porendurchmesser. Beim Beschichtungsvorgang entstand zunächst die Amino-Phase **A.** Die derivatisierten Rezeptor-Phasen **B** bis **D** wurden durch Festphasensynthese nach bekannten Methoden aus der Amino-Phase **A** hergestellt.

| **Tab. 2: Strukturen der verwendeten Rezeptor-Phasen** | | |
|---|---|---|
| **Phasenbezeichnung** | **Phasenaufbau** | **Phasenstruktur** |
| **A** BV 02043 | K1000-PVA-FA-2-5-Dod Amino-Phase | 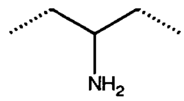 |
| **B** ND 03001#2 | K1000-PVA-FA-2-5-Dod-Ac-100 Acetyl-Phase | 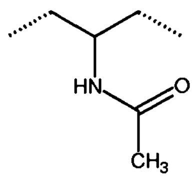 |

(fortgesetzt)

| Tab. 2: Strukturen der verwendeten Rezeptor-Phasen | | |
|---|---|---|
| **Phasenbezeichnung** | **Phasenaufbau** | **Phasenstruktur** |
| **C** ND 02031 | K1000-PVA-FA-2-10-Dod-MVS-100 4-Methylvaleryl-Phase | 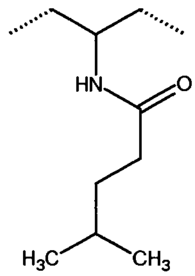 |
| **D** ND 03017 | K1000-PVA-FA-2-5-Dod-BzlO-100 Benzyloxycarbonyl-Phase | 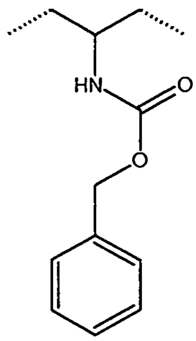 |

Alle Rezeptor-Phasen besaßen noch einen messbaren Gehalt an freien Aminogruppen, die im protonierten Zustand ionische Wechselwirkungen mit geeigneten anionischen Gruppen, z. B. Carboxylatgruppen, der Substrate eingehen konnten. Die Rezeptoren **C** und **D** enthielten zusätzlich einen für lipophile Wechselwirkungen geeigneten Rest.

Der Aminogruppengehalt der Rezeptoren wurde aus chromatographischen Durchbruchskurven mit 10 mM 4-Toluolsulfonsäure in DMF bestimmt. Die ermittelten Mengen von Aminogruppen pro Gramm Rezeptor-Phase sind in Tabelle 3 zusammengefasst.

| Tab. 3: Aminogruppengehalt der Rezeptor-Phasen | |
|---|---|
| **Synthetischer Rezeptor** | **Aminogruppen in mmol/g** |
| **A** BV 02043 | 0,60 |
| **B** ND 03001#2 | 0,03 |
| **C** ND 02031 | 0,13 |
| **D** ND 03017 | 0,16 |

Als mobile Phase für die chromatographischen Untersuchungen an den Substraten **1** bis **8** wurde wässriger Tris-HCl-Puffer von pH 7,5 verwendet. Die Elution erfolgte unter isokratischen Bedingungen bei Pufferkonzentrationen von 10 bis 500 mM.

Als Maß für die Stärke der Wechselwirkung zwischen Substrat und Rezeptor in den jeweiligen Pufferlösungen wurde die anlagenunabhängige, relative Elutionsgrösse k' (Kapazitätsfaktor) verwendet. Sie berechnet sich aus der Differenz aus Elutionsvolumen am Peakmaximum und Säulentotvolumen, dividiert durch das Säulentotvolumen, wie in nachfolgender Gleichung dargestellt:

$$k' = \frac{Elutionsvolumen - Säulentotvolumen}{Säulentotvolumen}$$

Die k'-Werte der Substrate in 10 bzw. 50 mM Tris-HCl-Puffer sind in den Tabellen 4 und 5 zusammengestellt.

| Tab. 4: k'-Werte der Substrate in 10 mM Tris-HCl-Puffer pH 7,5 | | | | | | | | |
|---|---|---|---|---|---|---|---|---|
| | **Relativer Elutionswert k' der Substrate** | | | | | | | |
| | **Ac** | | **Boc** | | **Z** | | **Fmoc** | |
| Rezeptor | **1** | **5** | **2** | **6** | **3** | **7** | **4** | **8** |
| **A** | 9,5 | 433 | 8,8 | 411 | 15 | >715 | 85 | >715 |
| **B** | 0,1 | 0,2 | 0,1 | 0,3 | 0,3 | 0,3 | 0,2 | 1,6 |
| **C** | 1,3 | 25,7 | 3,6 | 127 | 12,5 | 575 | 419 | >715 |
| **D** | 1,4 | 26,1 | 2,1 | 41,9 | **9,7** | **263** | 297 | >715 |

| Tab. 5: k'-Werte der Substrate in 50 mM Tris-HCl-Puffer pH 7,5 | | | | | | | | |
|---|---|---|---|---|---|---|---|---|
| | **Relativer Elutionswert k' der Substrate** | | | | | | | |
| | **Ac** | | **Boc** | | **Z** | | **Fmoc** | |
| Rezeptor | **1** | **5** | **2** | **6** | **3** | **7** | **4** | **8** |
| **A** | 2,3 | 33,2 | 2,2 | 34,4 | 3,6 | 62,9 | 20,3 | 478 |
| **B** | 0 | 0 | 0 | 0 | 0 | 0,1 | 0,2 | 0,2 |
| **C** | 0,2 | 2,3 | 0,9 | 9,9 | 3,4 | 37,6 | 111 | 562 |
| **D** | 0,3 | 2,6 | 0,5 | 5,0 | 2,5 | 20 | 78,4 | >715 |

Der Vergleich der k'-Werte innerhalb und zwischen den Tabellen 4 und 5 lieferte folgende Beobachtungen und Interpretationen der Beobachtungen:

1. **Beobachtung:** Die acetylierte Rezeptor-Phase **B** (ND 03001#2) band selbst bei der niedrigsten Pufferkonzentration keines der Substrate in nennenswertem Maße (k' ≤ 1,6).
**Interpretation der Beobachtung:** Dieser Rezeptor enthält nur noch sehr wenige Aminogruppen für mögliche ionische Wechselwirkungen mit den Carboxylatgruppen der Substrate. Weder die Acetylgruppen noch die Polyvinylaminketten der Rezeptor-Phase sind in der Lage nennenswerte lipophile Wechselwirkungen einzugehen.

2. **Beobachtung:** Die Substrate mit zwei Carboxylatgruppen **(5-8)** banden in 10 mM Puffer ca. 20 bis 40 mal stärker als die entsprechenden Substrate mit nur einer Carboxylatgruppe **(1-4).** In 50 mM war die Bindung der Dicarboxylate noch 10 bis 25 mal stärker als die der Monocarboxylate.
**Interpretation der Beobachtung:** Offensichtlich bestehen ionische Wechselwirkungen zwischen Carboxylatgruppen der Substrate und den Aminogruppen der Rezeptoren. Diese Wechselwirkungen führen bei Dicarboxylaten aufgrund der Bivalenz der Wechselwirkung zu sehr viel stärkerer Bindung als bei Substraten mit nur einer Carboxylgruppe. Die Amidgruppe leistet im wässrigen Medium keinen nennenswerten Bindungsbeitrag.

3. **Beobachtung:** Mit Erhöhung der Pufferkonzentration von 10 auf 50 mM verringerten sich die Bindungsstärken, bei Monocarboxylaten um einen Faktor von etwa vier, bei Dicarboxylaten um einen Faktor von etwa zehn.
**Interpretation der Beobachtung:** Auch dieses Ergebnis erklärt sich aus ionischen Wechselwirkungen, die bei höherer Pufferkonzentration geschwächt werden. Die Schwächung resultiert offenbar aus der Konkurrenz der Puffersalze mit den Carboxylatgruppen der Substrate um die Ammoniumgruppen des Rezeptors. Bei der starken Bindung der Substrate **5-8** wirkt sich die Konkurrenz der Puffersalze stärker aus, da zwei Carboxylatgruppen davon betroffen sind.

4. **Beobachtung:** Bei sonst identischen Substraten nahmen die k'-Werte mit der Größe des organischen Restes der N-Schutzgruppe drastisch zu. Die Größe dieses Bindungszuwachses war unabhängig von der Pufferkonzentration.
**Interpretation der Beobachtung:** Damit ist gezeigt, dass neben den ionischen Wechselwirkungen zwischen den Carboxylatgruppen der Substrate und den Ammoniumgruppen der Rezeptoren zusätzlich lipophile Wechselwirkungen zwischen Substrat und Rezeptor vorhanden sind. Aus diesem Grunde wirkt sich die Bindungsverstärkung beim Übergang von kleinen zu großen organischen Resten in der N-Schutzgruppe besonders auf den Rezeptor-Phasen **C** und **D** aus, deren Rezeptorgruppen für lipophile Wechselwirkungen besonders geeignet sind.

**Fazit:** Mit den oben beschriebenen Experimenten ließ sich klar nachweisen, dass die synthetischen Rezeptoren gleichzeitig zwei oder drei bindende Wechselwirkungen mit entsprechenden Substrate eingehen können, vorausgesetzt Rezeptor und Substrat sind hinsichtlich ihrer funktionellen Gruppen entsprechend komplementär ausgestattet.

Daraus kann gefolgert werden, dass durch Design eines entsprechend zu einer Zielsubstanz komplementären Rezeptors sich Begleitsubstanzen oder Nebenprodukte leicht abtrennen lassen. Das Maß für die Durchführbarkeit der Trennung ist der Quotient aus den k'-Werten, die Selektivität *alpha*, die in nachstehender Formel angegeben ist.

**Selektivität:** alpha = $k_2'/k_1'$

Mit der Benzyl-Amino-Rezeptor-Phase **D** beispielsweise betrug *alpha* etwa 25 (263/9,7) für die chromatographische Trennung von Z-Gln **(3)** und Z-Glu (7) mit 10 mM Tris-HCl-Puffer (pH 7,5) als mobiler Phase.

Es konnte somit gezeigt werden, dass ein quantifizierbarer Zusammenhang bestand zwischen den jeweils gezielt eingebrachten Molekülresten und den Bindungsstärken.

**Beispiel 2: Bindung des Flavanons Naringenin als Substrat an Rezeptor-Phasen der Firma instAction durch mindestens bivalente Bindung**

Die Wechselwirkung zwischen Naringenin und sieben verschiedenen Rezeptor-Phasen der Firma instrAction- (Tab. 6 und 7) wurde in Acetonitril als Lösemittel gemessen. Für diese Messungen wurde die direkte Methode der Gleichgewichtsbestimmung im sog. "Rührbecherversuch" angewendet.

| Naringenin |
|---|
| 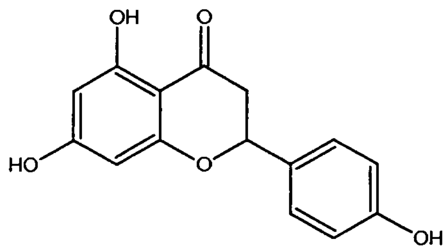 |

| Tab. 6: Verwendete Stationäre Phasen | | |
|---|---|---|
| **Rezeptor-Phase** | **Phasenaufbau** | **Aminogruppen in mmol/g** |
| **A** BV 02051 | K1000-PVA-FA-2-5-Dod | 0,54 |
| **C** ND 02048#2 | K1000-PVA-FA-2-5-Dod-MVS-100 | 0,16 |
| **D** ND 03017#3 | K1000-PVA-FA-2-5-Dod-BzlO-100 | 0,10 |
| **E** ND 03033#2 | K1000-PVA-FA-2-5-Dod-ImAc-100 | 0,53 |
| **F** ND 03049 | K1000-PVA-FA-2-5-Dod-Acrid9Car-100 | 0,29 |
| **G** ND 03050 | K1000-PVA-FA-2-5-Dod-NaphCar-100 | 0,23 |
| **H** ND 03062 | K1000-PVA-FA-2-5-Dod-iNic-100 | 0,35 |

Für die Rührbecherversuche wurden genau gewogene Mengen Rezeptor-Phase (je ca. 100 - 300 mg) in genau gemessenen Volumina Lösemittel (15 mL) suspendiert. Diesen Suspensionen wurden portionsweise genau gemessene Naringeninmengen (z. B. 1,0 mL einer 10 mM Lösung in Acetonitril) zugesetzt. Das Naringenin verteilte sich zwischen der Rezeptor-Phase und dem Lösemittel unter Einstellung eines dynamischen Gleichgewichts.

Die Lage des Gleichgewichts ließ sich dann exakt ermitteln, indem man die Naringenin-Konzentration im Lösemittel per Hochleistungsflüssigkeits-Chromatographie (HPLC) bestimmte. Hierbei erhielt man direkt die Stoffmenge des Naringenins in der flüssigen Phase (Acetonitril). Die Stoffmenge des Naringenins an der Rezeptor-Phase berechnete sich als Differenz zwischen zugefügtem Naringenin und in Lösung befindlichem Naringenin. Bei jedem Rührbecherversuch wurde das Gleichgewicht mit steigender Naringeninkonzentration im System mehrfach (6 - 12 mal) ermittelt. Die dabei eingetretenen Naringenin- und Lösemittelzugaben sowie -entnahmen wurden sorgfältig bilanziert und bei der Stoffmengenberechnung berücksichtigt.

| **Tab. 7: Derivate der stationären Phase** | | |
|---|---|---|
| **Bezeichnung** | **Kurzform** | **Struktur** |
| 4-Methylvaleriansäuregruppe | MVS | 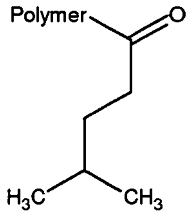 |
| Benzyloxycarbonylgruppe | BzlO | 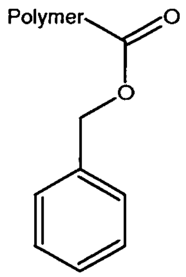 |
| 4-Imidazolylessisäuregruppe | ImAc | 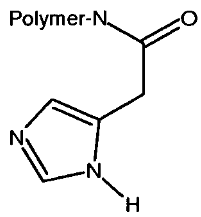 |
| Acridin-9-carbonsäuregruppe | Acrid9Car | 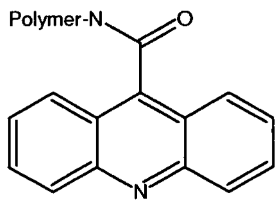 |
| 2-Naphthylcarbonsäuregruppe | Naphcar | 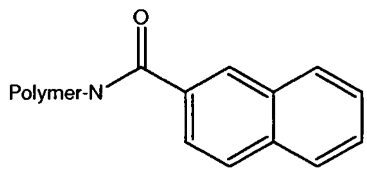 |
| Isonicotinsäuregruppe | iNic | 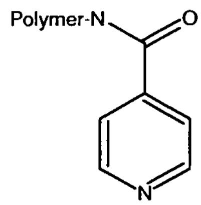 |

Bei jeder Gleichgewichtseinstellung erhielt man einen Punkt auf der Adsorptionsisothermen (Auftragung rezeptorgebundenes Naringenin [RS] gegen Naringenin in Lösung [S]). Unter Verwendung des Langmuir-Modells für Adsorptionsisothermen wurden die Gleichgewichtskonstanten für die Assoziation ($K_A$) und die maximale Beladbarkeit ($R_0$) durch nichtlineare Regression berechnet.

$$\textbf{Langmuir-Isotherme: } [RS] = [R_0] \times [S] / (1/K_A + [S])$$

Bei besonders schwachen Wechselwirkungen versagte die Methode der nichtlinearen Regression. In diesen Fällen wurden $K_A$ und $R_0$ durch lineare Regression aus dem Diagramm nach Scatchard ([RS]/[S] aufgetragen gegen [RS]) ermittelt. In der Auftragung nach Scatchard stellen sich einfache Langmuir-Isothermen als Geraden dar.

$$\textbf{Scatchard Linearisierung: } [RS]/[S] = -K_A \times [RS] + K_A \times [R_0]$$

Ein wichtiger Vorteil der Auftragung nach Scatchard ist, dass Abweichungen von der Linearität sehr leicht entdeckt werden können. Solche Abweichungen können auf Rezeptor-Phasen hindeuten, die gleichzeitig Bindestellen verschiedener Bindestärke und -anzahl aufweisen.

Die Werte für die Assoziationskonstante $K_A$ und die maximale Beladbarkeit $R_0$ sind in Tab. 8 dargestellt.

| **Tab. 8: Assoziationskonstante $K_A$ und maximale Beladbarkeit $R_0$** | | | | | |
|---|---|---|---|---|---|
| | | **Wechselwirkung** | | | |
| | | **stark** | | **schwach** | |
| **Rezeptor-Phase** | **Derivat** | $K_{A2}$ | $R_{02}$ | $K_{A1}$ | $R_{01}$ |
| **A** BV 02051 | 100% Aminogruppen | 2121 | 14,7 | 931 | 22,1 |
| **C** ND 02048#2 | MVS-100 | 1302 | 48,4 | 760 | 66,3 |
| **D** ND 03017#3 | BzlO-100 | - | - | 329 | 29,2 |
| **E** ND 03033#2 | ImAc-100 | 6194 | 4,9 | - | - |
| **F** ND 03049 | Acrid9Car-100 | 2961 | 19,5 | 65 | 243 |
| **G** ND 03050 | NaphCar-100 | 1943 | 38,5 | 379 | 91 |
| **H** ND 03062 | iNic-100 | - | - | 778 | 21,7 |

**Beobachtungen und Interpretation der Beobachtungen:** Naringenin konnte aufgrund seiner phenolischen Hydroxylgruppen polare Wechselwirkungen mit den primären Aminogruppen der Amino-Phase **A** ausbilden. Im aprotischen Lösemittel Acetonitril waren diese Wechselwirkungen gut messbar. Das Vorhandensein von starken ($K_{A2}$) und schwachen Bindestellen ($K_{A1}$) kann so gedeutet werden, dass Naringenin offenbar die Möglichkeit besitzt, mono-, bi- und trivalente polare Bindungen einzugehen, entsprechend den drei vorhandenen Phenolgruppen.

In den Rezeptor-Phasen **C, D** und **G** sind die meisten der primären Aminogruppen von Phase **A** mit lipophilen Resten derivatisiert. Hätten diese Reste keinen Anteil an der Bindung des Naringenins, so müssten die Beladbarkeiten $R_0$ dieser Phasen sinken, entsprechend dem geringeren Aminogruppengehalt. Die Gleichgewichtskonstanten sollten etwa gleich bleiben, da die Art der Wechselwirkung sich ja nicht ändern würde. Tatsächlich stiegen die Beladbarkeiten zum Teil deutlich an, z. B. von 14,7 auf 38,5 mmol/g Phase für den Naphthoyl-derivatisierten Rezeptor (Rezeptor-Phasen **A** und **G).** Dieses Resultat kann nur mit zusätzlichen Wechselwirkungen zwischen Naringenin und den Derivatisierungsgruppen erkärt werden. Diesen drei Rezeptorgruppen gemein ist die Fähigkeit, lipophile Wechselwirkungen einzugehen. Naringenin seinerseits besitzt ebenfalls lipophile Molekülteile, um an solchen Wechselwirkungen teilzunehmen.

Hieraus folgt, dass Naringenin mit den Rezeptor-Phasen **C, D** und **G** gleichzeitig polare Bindungen, nämlich mit den noch verbliebenen Aminogruppen, und lipophile Bindungen, mit den Rezeptorgruppen MVS, BzlO bzw. NaphCar, realisieren konnte. Bemerkenswert ist der Umstand, dass diese lipophilen Bindungen in einem organischen Lösemittel (Acetonitril) beobachtet werden konnten. Dies bedeutet, dass zwischen Naringenin und den lipophilen Rezeptorgruppen Kontakte stattfinden, die sich energetisch gegenüber einer Solvatation der lipophilen Gruppen mit einem organischen Lösemittel behaupten.

Die Assoziationskonstanten $K_A$ mit den Rezeptor-Phasen **C, D** und **G** setzen sich demnach aus Anteilen aus polaren und lipophilen Bindungen zusammen. Die Assoziationskonstanten sind hier durchweg geringer als bei der Amino-Phase **A.** Offenbar sind die lipophilen Bindungen schwächer als die polaren, was wiederum darauf zurück geführt werden kann, dass das verwendeten organische Lösemittel (Acetonitril) relativ polar ist.

Die Rezeptor-Phasen **E, F** und **H** enthalten Rezeptorgruppen, die sich sowohl an lipophilen als auch an polaren Bindungen beteiligen können - alle drei enthalten Aminogruppen, eingebettet in zum Teil ausgedehnten aromatischen

Strukturen. In der Tat finden sich die beiden höchsten $K_A$-Werte bei den Rezeptor-Phasen **E** und **F.** Man darf annehmen, dass hier ein kooperatives Zusammenwirken der polaren und der lipophilen Bindungsanteile besonders begünstigt war, während in den Rezeptoren **C, D** und **G** lipophile Rezeptorgruppen auf Kosten von Aminogruppen eingebaut worden waren.

**Fazit:** In diesem Beispiel wurde gezeigt, dass ein Substrat (Naringenin) in einem Lösemittel gegenüber entsprechenden Rezeptor-Phasen verschiedene Bindungen eingehen kann. Bei geeigneter Wahl der Rezeptorgruppen in der stationären Phase können gleichzeitig polare und lipophile Wechselwirkungen zur Bindung des Substrats aktiviert werden. Entsprechend können Rezeptor-Phasen hergestellt werden, die für Bindung bestimmter Substrate oder Substratgruppen optimiert sind, indem verschiedene Bindungsmöglichkeiten gleichzeitig vorhanden sind und dadurch selektive Wechselwirkungsräume entstehen.

**Beispiel 3: Bindung von strukturell verwandten Benzolderivaten als Substrate an eine Rezeptor-Phase der Firma instrAction durch mindestens bivalente Bindung**

Die Wechselwirkung zwischen strukturell verwandten Benzolderivaten und einer instrAction Rezeptor-Phase C (ND 02048#2, K1000-PVA-FA-2-5-Dod-MVS-100) wurde in einem unpolaren organischen Lösemittelgemisch gemessen. Neben den 4-Methylvaleriansäuregruppen (MVS) enthielt die Rezeptor-Phase C noch 0,16 mmol/g Aminogruppen. Als Lösemittel diente ein Gemisch aus Methyl-tert.-butylether/Heptan(1Volumenteil/3 Volumenteile). In diesem unpolaren Lösemittelgemisch waren vorwiegend polare Wechselwirkungen zu erwarten und andererseits waren alle Testsubstanzen darin gut löslich.

Die Assoziationskonstanten ($K_A$) und maximalen Beladbarkeiten ($R_0$) für die Wechselwirkungen zwischen der Rezeptor-Phase und den Testsubstanzen wurden mit sogenannten "Rührbecherversuchen" ermittelt.

Für die Rührbecherversuche wurden genau gewogene Mengen Rezeptor-Phase (je ca. 200 - 350 mg) in genau gemessenen Volumina Lösemittel (15 mL) suspendiert. Diesen Suspensionen wurden portionsweise genau gemessene Substratmengen zugesetzt. Das Testsubstrat verteilte sich zwischen der Rezeptor-Phase und dem Lösemittel unter Einstellung eines dynamischen Gleichgewichts. Die Lage des Gleichgewichts ließ sich exakt ermitteln, indem man die Substratkonzentration im Lösemittel per Hochleistungsflüssigkeits-Chromatographie (HPLC) bestimmte. Hierbei erhielt man direkt die Stoffmenge des Substrats im Lösemittel. Die Stoffmenge des Substrats an der Rezeptor-Phase berechnete sich als Differenz zwischen zugefügtem und in Lösung befindlichem Substrat. Bei jedem Rührbecherversuch wurde das Gleichgewicht mit steigender Substratkonzentration im System mehrfach (6 - 12 mal) ermittelt. Die Substrat- und Lösemittelzugaben und- entnahmen wurden sorgfältig bilanziert und bei der Stoffmengenberechnung berücksichtigt.

Bei jeder Gleichgewichtseinstellung erhielt man einen Punkt auf der Adsorptionsisothermen (Auftragung rezeptorgebundenes Substrat [RS] gegen Substrat in Lösung [S]). Unter Verwendung des Langmuir-Modells für Adsorptionsisothermen wurde die Gleichgewichtskonstante für die Assoziation ($K_A$) und die maximale Beladbarkeit ($R_0$) durch nichtlineare Regression berechnet.

**Langmuir-Isotherme:** $$[RS] = [R_0] \times [S] / (1/K_A + [S])$$

Bei besonders schwachen Wechselwirkungen versagte die Methode der nichtlinearen Regression. In diesen Fällen wurden $K_A$ und $R_0$ durch lineare Regression aus dem Diagramm nach Scatchard ([RS]/[S] aufgetragen gegen [RS]) ermittelt. In der Auftragung nach Scatchard stellen sich einfache Langmuir-Isothermen als Geraden dar.

**Scatchard Linearisierung:** $$[RS]/[S] = -K_A \times [RS] + K_A \times [R_0]$$

Die erhaltenen Wechselwirkungsparameter $K_A$ und $R_0$ sind zusammen mit den Testsubstraten in Tabelle 9 dargestellt.

| **Tab. 9: Verwendete Testsubstanzen und Bindungsresultate** | | |
|---|---|---|
| **Substratname** | **Substratstruktur** | **$K_A$ in L/mol**<br>**$R_0$ in µmol/g Phase** |
| 4-Amino-3-nitrobenzonitril | 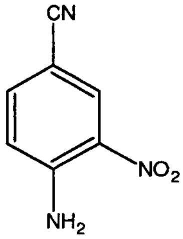 | 17700 ± 2600<br>3,5 ± 0,3 |
| 3-Nitrobenzonitril | 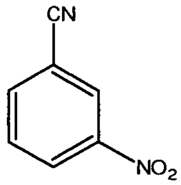 | 405 ± 109<br>12,9 ± 2,4 |
| 4-Aminobenzonitril | 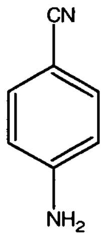 | 991 ± 59<br>19,6 ± 0,7 |
| Benzonitril | 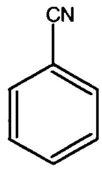 | 27 ± 14<br>nicht genau messbar |
| Nitrobenzol | 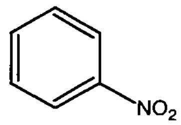 | Im verwendeten System unmessbar klein |

**Ergebnisbetrachtungen:** Aus Tabelle 9 ist ersichtlich, dass die Stärke der Wechselwirkung zwischen Testsubstanz und Rezeptor-Phase, repräsentiert durch die Assoziationskonstante $K_A$, mit der Anzahl der Substituenten am Benzolring zunahm.

Benzolderivate mit nur einem Substituenten wiesen Assoziationskonstanten von unter 40 L/mol auf, Werte, die im beschriebenen Messaufbau an der Erfassungsgrenze lagen.

Ein zweiter Substituent am Benzolring brachte eine weitere Wechselwirkungsmöglichkeit ins Testmolekül ein. Die beiden schwachen Wechselwirkungen kooperierten und ergaben Assoziationskonstanten für disubstituierte Benzolderivate, die sich näherungsweise als das Produkt der Assoziationskonstanten der monosubstituierten Benzole darstellten. Es wurden demnach $K_A$ Werte von 400 bis 1000 L/mol erhalten.

Der dritte Substituent am Benzolring multiplizierte die Assoziationskonstante des disubstituierten Benzols mit seinem eigenen, relativ schwachen Wechselwirkungspotential ($K_A \sim 20 - 40$ L/mol), und man erhielt für das trisubstituierte Benzol eine Assoziationskonstante von 17722 L/mol.

Für 4-Amino-3-nitrobenzonitril ist das Scatchard-Diagramm in Abb. 1 dargestellt.

Abb. 1: Scatchard-Diagramm für verschiedene Substratkonzentrationen [S] von 4-Amino-3-nitrobenzonitril

Darin bedeuten:

a: Bereich trivalenter Wechselwirkungen

[S] = 0,0044 - 0,043 mM
$K_{A3}$ = 17722 L/mol
$R_{03}$ = 3,5 μmol/g Phase

b: Übergangsbereich trivalenter und bivalenter Bindungen
[S] = 0,086 - 0,30 mM
$K_{A2}$ = 2350 L/mol
$R_{02}$ = 16 μmol/g Phase

c: Bereich bivalenter Wechselwirkungen
[S] = 0,40 - 0,98 mM
$K_{A1}$ = 855 L/mol
$R_{01}$ = 33 μmol/g Phase

Aus den Langmuir-Isothermen erhielt man nicht nur die Stärke der Wechselwirkung in Form der Assoziationskonstante $K_A$, sondern auch die Anzahl der Wechselwirkungsstellen als maximale Beladbarkeit $R_0$. Die maximale Beladbarkeit für die trivalente Wechselwirkung war ca. fünfmal geringer als $R_0$ für die bivalente Wechselwirkung. Dies ist unmittelbar verständlich, da man annehmen kann, dass in der synthetischen Rezeptor-Phase weniger Bindestellen für drei gleichzeitige Wechselwirkungen vorhanden sind als für zwei oder gar nur eine Wechselwirkung. 4-Amino-3-nitrobenzonitril konnte zusätzlich zu den trivalenten Bindestellen auch bi- und sogar monovalente Bindestellen besetzen; selbstverständlich mit den diesen Bindestellen entsprechenden geringeren Bindestärken ($K_A$) und höheren maximalen Beladbarkeiten ($R_0$).

In Abbildung 2 ist dieser Sachverhalt illustriert: Bestimmte man die Parameter $K_A$ und $R_0$ mit sehr kleinen Substratkonzentrationen, so beobachtete man vorwiegend die starke, trivalente Wechselwirkung ($K_{A3}$ und $R_{03}$). Die schwächeren mono- und bivalenten Bindestellen wurden aus solch verdünnten Lösungen noch nicht nennenswert besetzt. Bestimmte man $K_A$ und $R_0$ mit höheren Substratkonzentrationen, erhielt man die Wechselwirkungsgrößen der schwächeren und zahlreicheren bivalenten Bindestellen (z. B $K_{A1}$ und $R_{01}$). Starke Bindestellen waren unter diesen Substratkonzentrationen bereits gesättigt und leisteten nur noch einen konstanten Beitrag zur Adsorptionsisotherme. Monovalente Wechselwirkungen sind in Abb. 2 nicht dargestellt.

Generell beweist ein nach links ansteigender, gekrümmter Verlauf der Isothermen im Scatchard-Diagramm das gleichzeitige Vorhandensein von verschieden starken Bindestellen.

**Fazit:** Mit den dargestellten, experimentellen Ergebnissen ließ sich zeigen, dass die Rezeptor-Phase C (Struktur K1000-PVA-FA-2-5-Dod-MVS-100) sowohl starke trivalente als auch schwächere, mono- und bivalente Wechselwirkungen mit 3-Amino-4-nitrobenzonitril eingehen kann.

Substraten mit einer geringeren Anzahl von Substituenten gegenüber verhält sich die gleiche Rezeptor-Phase entsprechend, d. h. die maximale Bindestärke richtete sich nach der Anzahl der Substituenten im Substratmolekül.

Die Stärke der Bindung konnte darüber hinaus durch die substituentenabhängige Veränderung der permanenten und induzierten Dipole des Substratmoleküls beeinflusst werden.

**Beispiel 4: Bindung von Steroiden als Substrate an Rezeptor-Phasen der Firma instrAction durch mindestens bivalente Bindung**

Die Bindung (Retention) von Estradiol und von Testosteron an eine lediglich Aminogruppen enthaltende Rezeptor-Phase A (SBV 01044 VD/4 in Säule PV 02007) und an eine mit verzweigten Alkygruppen (4-Methylvaleriansäure) zu 27 % derivatisierte Phase C (ND 02001/1 in Säule PV 02001) wurde mit Gradienten-HPLC bestimmt.

Für die Gradienten-HPLC wurden folgende Bedingungen verwendet:

Neutrale Eluentien:

**Eluens A:** 1 Volumenteil Dimethyformamid + 9 Volumenteile Wasser
**Eluens B:** Dimethylformamid

Saure Eluentien:

**Eluens A:** 10 mM Trifluoressigsäure (TFA) in 1 Volumenteil Dimethyformamid-+ 9 Volumenteile Wasser

**Eluens B:** 10 mM Trifluoressigsäure in Dimethylformamid

**Gradientenprofil:** Für 5 Minuten konstant Eluens A mit einer Flußrate von 0,2 mL/min, dann Zumischung von B mit 2 %/min bei 0,6 mL/min bis zur vollständigen Substanzelution.

Im Gradienten eluiert die jeweilige Substanz dann, wenn die Gibbs-Energie für den Lösungsvorgang in der mobilen Phase die Rezeptor-Substrat-Bindungsenergie gerade übertrifft. Die Gibbs-Energie $\Delta$**G** der Rezeptor-Lösungsmittelwechselwirkung geht ebenfalls in die Energie-Bilanz ein: Beim Vorgang der Substratablösung (Desorption) wegen der höheren Anzahl adsorbierter kleiner Lösungsmittelmoleküle in der Regel mit einer Entropieabnahme $\Delta$**S** sowie mit einer mäßig negativen Wechselwirkungsenthalpie $\Delta$**H.**

Bei einer zweckmäßig aufgebauten Rezeptorphase ist während der Substratbindung (Adsorption) die Wechselwirkungsenthalpie $\Delta$**H** der Lösungsmitteladsorption erheblich weniger negativ als der Beitrag der mehrvalenten Wechselwirkungsenthalpie $\Delta$**H** zwischen Rezeptor und Substrat.

Da die betrachteten Substanzen in Wasser schlecht und in DMF gut löslich waren, war der zur Elution notwendige DMF-Gehalt der mobilen Phase ein grobes, jedoch einfach zu ermittelndes Maß, um die Bindungsstärke mehrerer Substrate gegenüber einem Rezeptor schnell zu vergleichen.

Es wurde erwartet, dass sowohl Estradiol als auch Testosteron mit den Alkylgruppen der Rezeptor-Phasen lipophile Wechselwirkungen eingehen, Estradiol sollte außerdem zu einer salzartigen Phenol-Amin Bindung fähig sein. Die in Rezeptor-Phase C (ND 02001/1 in Säule PV 02001) vorliegende 4-Methylvaleriansäuregruppe sollte außerdem den lipophilen Bindungsanteil im Vergleich zur Aminophase A erheblich verstärken.

Es wurde prognostiziert, dass Estradiol im Gegensatz zu Testosteron eine bivalente Bindung eingehen kann mit einem ionischen und einem lipophilen Anteil. In diesem Fall sollte Estradiol im verwendeten Lösungsmittelgradienten von der Rezeptor-Phase **C** erheblich später eluieren als Testosteron. Bei der Phase **A** waren hingegen insgesamt deutlich kürzere Retentionszeiten zu erwarten sowie geringere Unterschiede im Elutionsverhalten von Testosteron und Estradiol.

In Tabelle 10 ist der DMF-Anteil der mobilen Phase angegeben, der erforderlich war, um die Rezeptor-Substrat-Bindung zu brechen.

| **Tab. 10: Gradientenelution von Estradiol und Testosteron** | | | | |
|---|---|---|---|---|
| | **Wasser-DMF-Gradient** | | **10 mM TFA Wasser-DMF-Gradient** | |
| Substrat | Aminophase **A** PV 02007 | Rezeptor-Phase **C** PV 02001 | Aminophase **A** PV 02007 | Rezeptor-Phase **C** PV 02001 |
| Estradiol | 13,1 % | 46,7 % | 11,3 % | 36,6 % |
| Testosteron | 10,0 % | 18,5 % | 10,0 % | 27,3 % |

**1. Beobachtung:** Die Ergebnisse zeigten, dass Estradiol bereits auf der Aminophase A (PV 02007) stärker band als Testosteron, was auf die zusätzliche ionische Wechselwirkung zurückgeführt werden konnte. Auf der alkylierten Phase C (PV 02001) eluierte Estradiol erst bei 46,7 % DMF, was gegenüber der Grundphase eine Zunahme um 33,6 Volumenanteile darstellte. Angesichts der Elutionskraft von DMF entsprach dieses Ergebnis einer drastischen Bindungszunahme. Die Bindung von Testosteron nahm hingegen nur moderat auf 18,5 % DMF zu.

**2. Beobachtung:** Wie zu erwarten war, nahm die Bindung von Estradiol ab, wenn seine ionische Wechselwirkungsmöglichkeit durch Protonierung der Aminogruppen an der stationären Phase weitgehend ausgeschaltet wird, indem man der mobilen Phase 10 mM Trifluoressigsäure zufügte.

Die Bindung von Testosteron wurde hingegen im sauren Medium gegenüber der Phase C mäßig verstärkt und bliebt gegenüber der Phase A unverändert. Für beide Substrate war denkbar, dass die wegen der Trifluoressigsäure im Eluens entstandenen Ammoniumgruppen des Rezeptors zusätzliche Wechselwirkungen eingehen, die dem Amin nicht zur Verfügung stehen.

Insgesamt fiel auf, dass die Bindungsverstärkung an der Rezeptor-Phase erheblich größer ausfiel, wenn zwei unterschiedliche nicht-kovalente Wechselwirkungsarten genutzt wurden. Die Bindungsverstärkung durch Vergrößerung lediglich der lipophilen Kontaktfläche der aliphatischen Molekülteile war weniger ausgeprägt.

Diese Befunde wurden weiter gestützt durch Vergleich der Retention charakteristischer Strukturelemente des Estradiol-Moleküls. Mit solchen molekularen Sonden ließen sich schnell umfangreiche HPLC-Tests durchführen. So band 2-Naphthol an Phasen vom Typ C erheblich stärker als Naphthalin und dieses wiederum besser als 1,2,3,4-Tetrahydronaphthol. Aus diesem Verhalten ließ sich wiederum der erwartete ionische Bindungsbeitrag ableiten, während eine polare Bindung der alkoholischen OH-Gruppen im wässrigen Lösungsmittel erwartungsgemäß nicht erfolgte.

**Fazit:** Die bivalente Bindung von phenolischen Steroiden auf alkyl- und aminogruppenhaltigen Phasen wie C (PV 02001) war für ihre Abtrennung von nicht aromatischen Steroiden vorteilhaft. Dabei wurden unter isokratischen Trennbedingungen $\alpha$-Werte (Trennselektivitäten) bis 10 erreicht.

Auf dem schwach hydrophoben Ionenaustauscher **A** (PV 02007) war diese Trennung hingegen nicht mit zufriedenstellender Auflösung möglich.

Das dargestellte Prinzip lässt sich verallgemeinern zur Trennung von phenolischen Substanzen von neutralen oder basischen Aliphaten, aber auch Aromaten. Darüber hinaus können auch mehrwertige Phenole gut getrennt werden.

**Beipiel 5: Bindung von Lactamen als Substrate an Rezeptor-Phasen der Firma instrAction durch mindestens bivalente Bindung**

Die Bindung von Methyl-phenylhydantoin (MPH) 1, Diphenylhydantoin (DPH) 2 und Methyl-phenylsuccinimid 3 aus Chloroform zu einer Reihe von 80 % Aminogruppen und 14 % Benzylgruppen (Vernetzungsgrad 5 %) enthaltenden Rezeptor-Phasen (z. B. PV 99047, PV 00010) wurde mittels Frontalanalyse bestimmt.

Hierzu wurde die in HPLC Säulen (40 x 4 mm) gepackte Rezeptor-Phase mit Substratlösungen steigender Konzentration gespült bis zum jeweiligen Sättigungsgleichgewicht. Aus der Flussrate, der Zeit bis zum Substanzdurchbruch und der Substratkonzentration lassen sich, bei bekannt konstanter Substratkonzentration **[S] = [$S_o$],** die jeweiligen Konzentrationen an gebundenem Substrat **[RS]** berechnen. Aus den für 10-12 Substratkonzentrationen gemessenen Durchbruchskurven konnten über die Adsorptionsisothermen bzw. die Scatchard-Diagramme die Bindungskonstanten **$K_A$** und die Sättigungskonzentration **[$R_o$]** bestimmt werden, wodurch sich die Bereiche bivalenter und monovalenter Bindung ermitteln ließen.

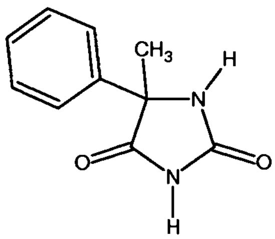

1

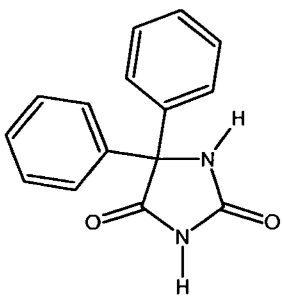

2

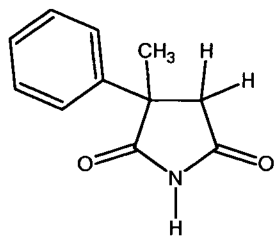

3

Durch die Lösungsmittelwahl wurde sichergestellt, dass im Wesentlichen polare Wechselwirkungen erfasst werden, insbesondere Wasserstoffbrückenbindungen.

Typische aufgefundene Werte sind unter a) bis c) angegeben.

a) Bindung von MPH an Poly(benzyl-N-allyl-carbamat) auf Kieselgel, 6 Schichten, vernetzt (PAA-OBzl14-2Dod, PV 99047):

Bereich bivalenter Bindung:

$K_A$ = 12703 $M^{-1}$
$\Delta G$ = 5,50 kcal/mol
$R_o$ = 12,0 $\mu$mol/g

Bereich monovalenter Bindung:

$K_A$ = 221 $M^{-1}$
$\Delta G$ = 3,14 kcal/mol
$R_o$ = 301,4 $\mu$mol/g

b) Bindung von DPH an Poly(benzyl-N-allyl-carbamat) auf Kieselgel, 6 Schichten, vernetzt (PAA-OBzl14-2Dod, PV 00010):

Bereich bivalenter Bindung:

$K_A$ = 19880 $M^{-1}$
$\Delta G$ = 5,76 kcal/mol
$R_o$ = 4,6 µmol/g

Bereich monovalenter Bindung:

$K_A$ = 201 $M^{-1}$
$\Delta G$ = 3,09 kcal/mol
$R_o$ = 226,7 µmol/g

c) Bindung von MPS an Poly(benzyl-N-allyl-carbamat) auf Kieselgel, 3 Schichten, vernetzt (PAA-OBzl14-2Dod, PV 99047):

Bereich monovalenter Bindung:

$K_A$ = 75 - 78 $M^{-1}$
$R_o$ = 96,5 - 97,4 µmol/g

**1. Beobachtung:** Für die beiden Hydantoine 1 und 2 (MPH und DPH) wurden in umfangreichen Messreihen für mehrere Varianten der Rezeptor-Phasen (z. B. PV 99047, PV 00010) bivalente Bindungskonstanten $\mathbf{K_A}$ zwischen 6000 und 23000 $M^{-1}$ ermittelt bei Sättigungsstoffmengen $\mathbf{R_o}$ zwischen 3 µmol/g Phase und 12,6 µmol/g Phase. Dies deutete darauf hin, dass zwei Wasserstoffbrücken zum Amin ausgebildet werden konnten. Die monovalente Bindungskonstante lag zwischen 109 und 221 $M^{-1}$ ($\mathbf{R_o}$ 239 - 301 µmol/g). Für das Succinimidderivat hingegen wurden lediglich monovalente Bindungskonstanten von 75 bis 78 $M^{-1}$ gefunden mit einem Sättigungswert $\mathbf{R_o}$ von 96 µmol/g. Dies kann damit gedeutet werden, dass ein Succinimid nur eine Wasserstoffbrücke ausbilden kann, und deshalb nur zur monovalenten Bindung befähigt ist.

**2. Beobachtung:** Die Bindungskonstante für eine bivalente Bindung entspricht recht gut dem Produkt der Werte der kombinierten monovalenten Bindungskonstanten. Die zugehörigen monovalenten Gibbs-Energien $\Delta\mathbf{G}$ addieren sich näherungsweise. Für eine einzelne Wasserstoffbrückenbindung einer Fünfring-Lactamgruppe wurden in Chloroform bei 25 °C Gibbs-Energien $\Delta\mathbf{G}$ zwischen 2,5 und 3,14 kcal/mol bestimmt, für die bivalenten H-Brücken 5,06 bis 5,88 kcal/mol. Diese Werte übertreffen die anhand der Literatur für das Lösungsmittel Chloroform erwarteten Daten (MPH: $K_A$ = 6014 $M^{-1}$, $\Delta G$ = 5,06 kcal/mol, $R_o$ = 3,2 µmol/g; DPH: $K_A$ = 7171 $M^{-1}$, $\Delta G$ = 5,16 kcal/mol, $R_o$ = 6,9 µmol/g und $K_A$ = 145 $M^{-1}$, $\Delta G$ = 2,90 kcal/mol, $R_o$ = 264,0 µmol/g).

**Fazit:** Damit konnte gezeigt werden, dass eine bivalente Bindungsverstärkung auch dann auftritt, wenn auf der Substratseite und auf der Rezeptorseite jeweils zwei gleichartige komplementäre Reste (binding-site residues) miteinander in Wechselwirkung treten, ähnlich wie bei Chelateffekten. Im genannten Fall waren dies die Amidgruppen des Substrates und die Amingruppen des Rezeptors. Dabei verhielten sich die Gibbs-Energien näherungsweise additiv, die Bindungskonstanten multiplikativ. Nach diesem Prinzip lassen sich insbesondere Rezeptor-Phasen entwickeln, die sich für die Trennung von homologen Substanzen oder von Substanzen mit unterschiedlicher Wertigkeit hinsichtlich der funktionellen Gruppen (z. B. ein- bis sechswertige Alkohole wie etwa Zucker) eignen.

**Beispiel 6: Bindung einiger C-blockierter Aminosäuren als Substrate an Sorbentien auf Basis Polyvinylamin/Kieselgel als Sorbentien durch mindestens bivalente Bindung**

Es wurden die Retentionseigenschaften von 18 verschiedenen Aminosäurederivaten (Substrate in Tabelle 11) bei der Chromatographie auf sieben verschiedenen stationären Phasen (synthetische Rezeptoren) untersucht.

Bei den Aminosäurederivaten **(1-18)** handelte es sich um Ester des Alanins, Leucins, Prolins, Lysins, Histidins, Phenylalanins, Tyrosins und des Tryptophans. Die Ester wurden gewählt, um unerwünschte Wechselwirkungen der ionisierbaren Carboxylatfunktionen auszuschließen. Von den Methylestern erwarteten wir keinen nennenswerten Wechselwirkungsbeitrag, ganz im Gegensatz zu den Benzylestern.

| Tab. 11: Aminosäurederivate als Substrate | | |
|---|---|---|
| **Substrat** | **Name** | **Struktur** |
| **1** | H-Ala-OMe | 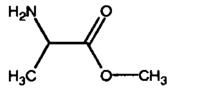 |
| **2** | H-Ala-OBzl | 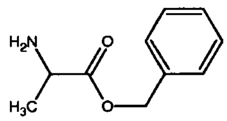 |
| **3** | H-Leu-OMe | 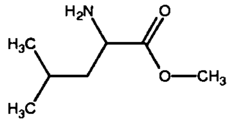 |
| **4** | H-Leu-OBzl | 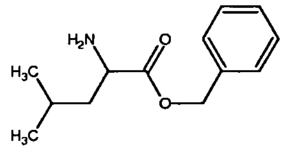 |
| **5** | H-Pro-OMe | 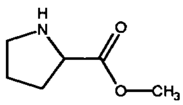 |
| **6** | H-Pro-OBzl | 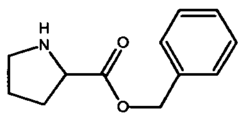 |
| **7** | Z-Lys-OMe | 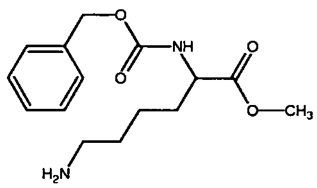 |
| **8** | H-Lys(Z)-OMe | 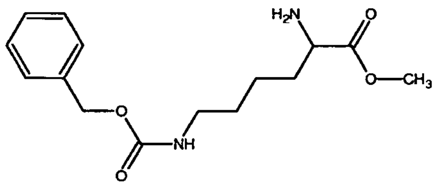 |
| **9** | Boc-Lys-OMe | 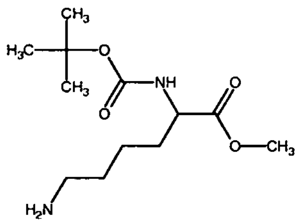 |
| **10** | H-Lys(Boc)-OMe | 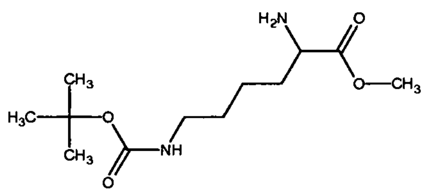 |

(fortgesetzt)

| Tab. 11: Aminosäurederivate als Substrate | | |
|---|---|---|
| **Substrat** | **Name** | **Struktur** |
| **11** | H-His-OMe | 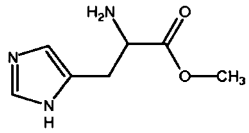 |
| **12** | Bzl-His-OMe | 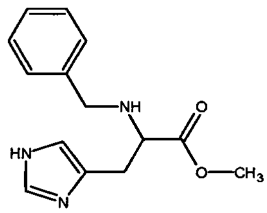 |
| **13** | H-Phe-OMe | 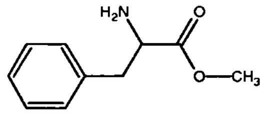 |
| **14** | H-Phe-OBzl | 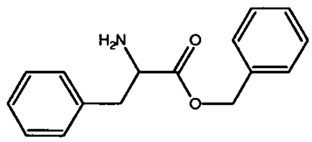 |
| **15** | H-Tyr-OMe | 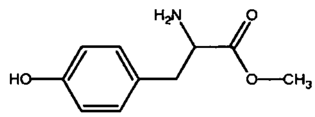 |
| **16** | H-Tyr-OBzl | 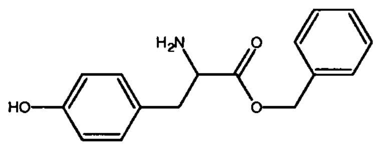 |
| **17** | H-Trp-OMe | 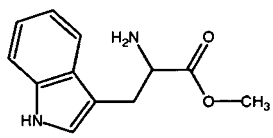 |
| **18** | H-Trp-OBzl | 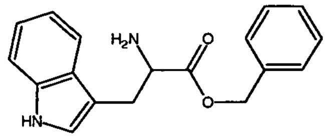 |

Bei den verwendeten Rezeptor-Phasen handelte es sich um Polyvinylaminbeschichtetes, sphärisches Kieselgel mit 20 µm Partikelgröße und 1000 Å Porendurchmesser. Beim Beschichtungsvorgang entstand zunächst die Amino-Phase **A.** Die derivatisierten Rezeptor-Phasen **B** bis **K** wurden durch Festphasensynthese aus der Amino-Phase **A** nach bekannten Methoden hergestellt. Die Phasen sind in Tab. 12 zusammengestellt.

| Tab. 12: Strukturen der verwendeten Rezeptor-Phasen | | |
|---|---|---|
| **Phasenbezeichnung** | **Phasenaufbau** | **Phasenstruktur** |
| **A**<br>BV 03002 | K1000-PVA-FA-2-5-Dod<br>Amino-Phase | 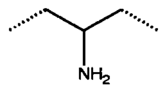 |
| **B**<br>ND 03001#2 | K1000-PVA-FA-2-5-Dod-Ac-100<br>Acetyl-Phase | 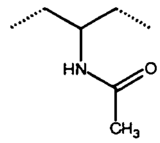 |
| **C**<br>ND 03105 | K1000-PVA-FA-2-10-Dod-MVS-100<br>4-Methylvaleryl-Phase | 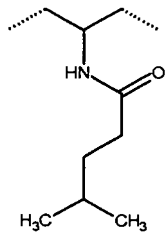 |
| **D**<br>ND 03017#3 | K1000-PVA-FA-2-5-Dod-BzlO-100<br>Benzyloxycarbonyl-Phase | 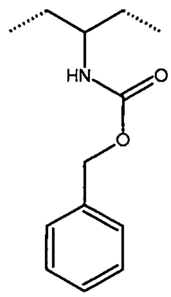 |
| **I**<br>ND 02061#2 | K1000-PVA-FA-2-5-Dod-BSr-100<br>Bernsteinsäure-Phase | 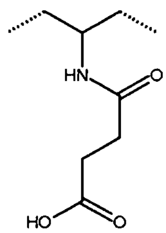 |
| **J**<br>ND 03096 | K1000-PVA-FA-2-5-Dod-MVS-50-BSr-50<br>Phase mit 4-Methylvaleryl- und Bernsteinsäuregruppen | 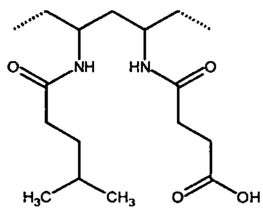 |
| **K**<br>ND 03088 | K1000-PVA-FA-2-5-Dod-BzlO-50-BSr-50<br>Phase mit Benzyloxycarbonyl- und Bernsteinsäuregruppen | 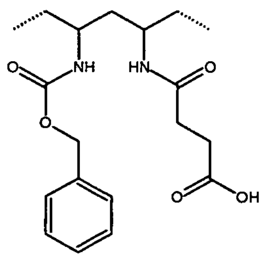 |

Als mobile Phase für die chromatographischen Untersuchungen wurde wässriger 10 mM Tris-HCl-Puffer von pH 7,5 verwendet.

Als Maß für die Stärke der Wechselwirkung zwischen Substrat und Rezeptor in den jeweiligen Pufferlösungen

wurde die anlagenunabhängige relative Elutionsgrösse k' (Kapazitätsfaktor) verwendet. Sie berechnet sich aus der Differenz aus Elutionsvolumen am Peakmaximum und Säulentotvolumen, dividiert durch das Säulentotvolumen.

$$k' = \frac{Elutionsvolumen - Säulentotvolumen}{Säulentotvolumen}$$

Die k'-Werte der Substrate in 10 mM Tris-HCl-Puffer sind in Tabelle 13 zusammengestellt.

| **Tab. 13: k'-Werte der Substrate in 10 mM Tris-HCl-Puffer** | | | | | | | | |
|---|---|---|---|---|---|---|---|---|
| Substrat | | **k'-Wert auf Rezeptor-Phase** | | | | | | |
| | | **A** | **B** | **C** | **D** | **I** | **J** | **K** |
| **1** | H-Ala-OMe | 0,0 | 0,0 | 0,0 | 0,2 | 13,7 | 8,7 | 11,6 |
| **2** | H-Ala-OBzl | 0,0 | 0,0 | 0,2 | 2,1 | 17,4 | 13,4 | 23,9 |
| **3** | H-Leu-OMe | 0,0 | 0,0 | 0,0 | 0,3 | 12,5 | 7,1 | 10,4 |
| **4** | H-Leu-OBzl | 0,0 | 0,1 | 6,3 | 10,0 | 13,1 | 18,6 | 41,7 |
| **5** | H-Pro-OMe | 0,0 | 0,0 | 0,0 | 0,4 | - | 11,9 | 15,9 |
| **6** | H-Pro-OBzl | 0,0 | 0,1 | 0,2 | 3,6 | 21,7 | 16,6 | 34,4 |
| **7** | Z-Lys-OMe | 0,0 | 0,1 | 0,0 | 2,4 | 19,7 | 20,9 | 61,5 |
| **8** | H-Lys(Z)-OMe | 0,0 | 0,1 | 0,6 | 6,5 | 12,0 | 11,5 | 30,2 |
| **9** | Boc-Lys-OMe | 0,0 | 0,0 | 0,0 | 0,4 | 14,3 | 11,9 | 20,2 |
| **10** | H-Lys(Boc)-OMe | 0,0 | 0,0 | 0,1 | 0,5 | 9,0 | 5,3 | 9,8 |
| **11** | H-His-OMe | 0,0 | 0,0 | 0,0 | 0,3 | 18,2 | 5,7 | 13,8 |
| **12** | Bzl-His-OMe | 0,0 | 0,0 | 0,5 | 1,2 | 4,5 | 1,9 | 3,7 |
| **13** | H-Phe-OMe | 0,0 | 0,0 | 0,1 | 0,2 | 6,5 | 1,7 | 3,2 |
| **14** | H-Phe-OBzl | 0,1 | 0,1 | 12,6 | 39,0 | 7,3 | 12,3 | 39,4 |
| **15** | H-Tyr-OMe | 0,0 | 0,2 | 0,7 | 1,0 | 8,7 | 4,8 | 6,5 |
| **16** | H-Tyr-OBzl | 0,1 | 0,3 | 16,7 | 20,3 | 9,4 | 16,3 | 16,5 |
| **17** | H-Trp-OMe | 0,5 | 0,2 | 1,0 | 4,4 | 12,5 | 10,7 | 17,7 |
| **18** | H-Trp-OBzl | 0,8 | 0,3 | 49,6 | 55,4 | 16,6 | 49,5 | 186,4 |

**1. Beobachtung:** In Beispiel 1 wurden k'-Werte von Aminosäurederivaten mit Carboxylatgruppen auf Amino-Phasen untersucht. Die Monocarboxylate Ac-Gln **1** und Boc-Gln **2** aus Beispiel 1 erzielten auf einer Amino-Phase (BV 02042) k'-Werte von 9,5 und 8,8. Im vorliegenden Beispiel 6 erhielt man für einfache Monoamine wie H-Ala-OMe **1** und H-Leu-OMe **3** auf der Carboxylat-Phase **I** k'-Werte von 13,7 und 12,5.

**Interpretation der Beobachtung:** Beim Vertauschen der Wechselwirkungsgruppen in Substrat und Rezeptorphase ändern sich die k'-Werte nur wenig. Dies war zu erwarten, da die Stärke der Bindung von der Richtung der Bindung unabhängig sein sollten. Für den planvollen Einsatz von Wechselwirkungsgruppen ist es wichtig, dass eine vergleichbare Bindung stattfindet, unabhängig welche der Gruppen sich fixiert im Rezeptor oder mobil in Substrat befindet.

**2. Beobachtung:** Auf der Amino-Phase **A** und der Acetamido-Phase **B** trat praktisch keine Retention der Substrate auf.

**Interpretation der Beobachtung:** Die Rezeptorphasen **A** und **B** enthalten keine Rezeptorgruppen, mit denen im gewählten Puffer eine nennenswerte Wechselwirkung zu den Substraten möglich wäre. Entsprechend lagen die k'-Werte um Null. Diese Phasen lassen sich als Nullpunkte auf einer relativen Wechselwirkungsskala verwenden. Der lipophile Einfluss des Polymergerüstes kann in der Bindungsbilanz vernachlässigt werden.

**3. Beobachtung:** Alle Substrate zeigten deutlich Retention auf der Carboxylat-Phase **I.** Die k'-Werte liegen zwischen 4,5 und 21,7.

**Interpretation der Beobachtung:** Alle untersuchten Substrate enthalten mindestens eine Aminogruppe. Diese Aminogruppe ist bei pH 7,5 weitgehend protoniert und kann starke ionische Wechselwirkungen mit den Carboxylat-Anionen der Phase eingehen.

**4. Beobachtung:** Die Rezeptorphasen **C** und **D** zeigten geringe Retention mit Substraten, die eine einzelne lipophile Teilstruktur enthielten, z. B. **2, 6, 7, 8, 15** oder **17.** Starke Retention (k'-Werte > 8) fanden sich mit Substraten, die mindestens zwei größere lipophile Molekülteile besaßen, wie **4, 14, 16** und **18.** Dabei war die Bindung zur aromatischen Rezeptorphase **D** in jedem Fall größer als zur Alkyl-Rezeptorphase.

**Interpretation der Beobachtung:** Die Rezeptorphasen **C** und **D** können nur lipophile Wechselwirkungen eingehen. Diese Bindungen sind verglichen mit ionischen Wechselwirkungen relativ schwach. Monovalente lipophile Wechselwirkungen liegen im gewählten Puffer daher oft an der Nachweisgrenze. Substrate mit zwei ausgedehnten lipophilen Resten weisen in Folge der lipophilen Kontaktfläche eine erhöhte Retention auf.

**5. Beobachtung:** Der größte k'-Wert für das jeweilige Substrat fand sich in den meisten Fällen auf der Rezeptor-Phase **K.**

**Interpretation der Beobachtung:** Die Rezeptorphase **K** enthält zu etwa gleichen molaren Anteilen Carboxylat- und Benzyloxycarbonylgruppen, d. h. Rezeptorgruppen für ionische und für lipophile Wechselwirkungen. Da die Gesamtzahl der Wechselwirkungsgruppen etwa jener der sortenreinen Rezeptorphasen **C, D** oder **I** entspricht, sollte man auf der gemischten Rezeptorphase **K** einen k'-Wert erwarten, der zwischen den k'-Werten der Phasen **D** und **I** liegt. Die gefundenen, hohen k'-Werte auf der gemischten Rezeptorphase zeigen, dass in diesen Fällen ionische und lipophile Bindungen gleichzeitig stattfinden und damit ein gemischter, bivalenter Bindemodus vorliegt.

Wegen der Stärke von $\pi$-$\pi$-Kontakten zwischen Aromaten ist die Bindung aller Substrate, die einen aromatischen Rest aufweisen zur benzylguppenhaltigen Phase K stärker als zur Phase J mit verzweigtem Alkylrest.

**Fazit:** Mit den oben beschriebenen Experimenten ließ sich klar nachweisen, dass man Wechselwirkungen zwischen einem Substrat und einer Rezeptorphase durch geeignete Wahl sortenreiner Rezeptorgruppen gezielt ein- und ausschalten konnte. Zur Regulierung von Affinität und Selektivität können zusätzlich die Lösungsmittelzusammensetzung, die Ionenstärke und der pH-Wert variiert werden.

Besitzt ein Substrat zwei lipophile Molekülteile, so kann es bivalent mit der Rezeptorphase wechselwirken, was zu einer deutlichen Verstärkung der Bindung führt. In diesem Fall handelte es sich um den Fall einer bivalenten Wechselwirkung gleichen Typs.

Es konnte auch gezeigt werden, dass bivalente Wechselwirkungen verschiedenen Typs (ionisch und lipophil) möglich sind, wenn sowohl die Rezeptorphase als auch das Substrat entsprechend komplementäre Gruppen enthalten. Auch hierbei kommt es zu einer selektiven Bindungsverstärkung.

Durch Design eines entsprechend zu einer Zielsubstanz komplementären Rezeptors lassen sich Begleitsubstanzen oder Nebenprodukte leicht abtrennen. Das Maß für die Durchführbarkeit der Trennung ist der Quotient aus den k'-Werten, die Selektivität *alpha.*

$$\textbf{Selektivität:}\ \mathrm{alpha} = k_2'/k_1'$$

Mit der Benzyl-Rezeptor-Phase **D** beispielsweise wäre eine chromatographische Trennung von Boc-Lys-OMe **(9)** und H-Lys(Boc)-OMe **(10)** kaum möglich. Auf der Carboxylat-Rezeptorphase **I** ergab sich ein alpha-Wert von 1,59. Die gemischten Rezeptorphasen **J** und **K** zeigten bereits alpha-Werte von 2,25 und 2,06. Dies bedeutete, dass die chromatographische Trennbarkeit eines Gemischs durch geeignetes Design der Rezeptorphase signifikant verbessert wird.

**Patentansprüche**

1. Verfahren zur selektiven bivalenten Bindung eines synthetischen oder natürlichen Substrats mit mindestens zwei unterschiedlichen zur Bindung befähigten Gruppen an ein Sorbens, welches einen festen Träger und mindestens zwei unterschiedliche zur Bindung mit dem synthetischen oder natürlichen Substrat befähigte Gruppen umfasst, wobei das Verfahren die Stufen (i) bis (iv) umfasst:

   (i) Ermittlung von mindestens zwei zur Bindung mit einem Sorbens befähigten Gruppen aus dem synthetischen oder natürlichen Substrat,
   (ia) Bindung eines underivatisierten Polymers über nichtkovalente Wechselwirkungen an den Träger,
   (ii) Einführen jeweils mindestens zweier unterschiedlicher zur Bindung mit dem synthetischen oder natürlichen Substrat befähigten Gruppen über mindestens zwei gleiche oder unterschiedliche funktionelle Gruppen des

Polymers in dieses Polymer unter Bildung mindestens eines Sorbens, wobei es sich bei den Gruppen um Gruppen handelt, die komplementär zu den in Stufe (i) ermittelten Gruppen sind, wobei ein mit mindestens zwei unterschiedlichen zur Bindung befähigten Gruppen derivatisiertes Polymer entsteht, und die jeweils mindestens zwei unterschiedlichen Gruppen zur Bindung befähigten Gruppen kovalent gebunden am Polymer vorliegen, wobei

(a) die Gruppen derart ermittelt werden, dass die Beiträge der Gibbs-Energien der einzelnen Gruppen für die nicht-kovalente Bindung mit dem Substrat einen negativen Wert der Gibbs-Energie $\Delta G$ ergeben, derart, dass eine Bindungsverstärkung eintritt, die sich in einer verbesserten Trennselektivität $\alpha$, mit der das selektiv zu bindende Substrat mit den mindestens zwei unterschiedlichen zur Bindung an das mindestens eine Sorbens befähigter Gruppen unter Verwendung des mindestens einen Sorbens aus einem Substratgemisch abgetrennt wird, größer als 1,4 auswirkt
und wobei

(iii) Inkontaktbringen des Substrats mit dem Sorbens der Stufe (ii);
(iv) Prüfung der Bindungsstärke des Substrats an das Sorbens der Stufe (iii).

2. Verfahren nach Anspruch 1 **dadurch gekennzeichnet, dass** die Ermittlung in Stufe (i)das Zerlegen des synthetischen oder natürlichen Substrats in mindestens zwei Bausteine mit mindestens zwei zur Bindung mit einem Sorbens befähigten Gruppen umfasst.

3. Verfahren nach Anspruch 2, **dadurch gekennzeichnet, dass** ein Baustein mindestens zwei unterschiedliche zur Bindung befähigte Gruppen besitzt.

4. Verfahren nach einem der vorhergehenden Ansprüche, **dadurch gekennzeichnet, dass** die Auswahl mindestens zweier zur Bindung mit einem Sorbens befähigten Gruppen aus dem synthetischen oder natürlichen Substrat in Stufe (i) zu zwei Bausteinen mit jeweils mindestens einer zur Bindung mit dem Sorbens befähigten Gruppe führt und in Stufe (ii) ein Sorbens erhalten wird; oder die Auswahl mindestens zweier zur Bindung mit einem Sorbens befähigten Gruppen aus dem synthetischen oder natürlichen Substrat in Stufe (i) zu drei Bausteinen mit jeweils mindestens einer zur Bindung mit dem Sorbens befähigten Gruppe führt und in Stufe (ii) mindestens drei Sorbentien erhalten werden; oder die Auswahl mindestens zweier zur Bindung mit einem Sorbens befähigten Gruppen aus dem synthetischen oder natürlichen Substrat in Stufe (i) zu vier Bausteinen mit jeweils mindestens einer zur Bindung mit dem Sorbens befähigten Gruppe führt und in Stufe (ii) mindestens sechs Sorbentien erhalten werden.

5. Verfahren nach einem der vorhergehenden Ansprüche, **dadurch gekennzeichnet, dass** die mindestens zwei unterschiedlichen zur Bindung befähigten Gruppen des mindestens einen Sorbens ausgewählt werden unter Gruppen, die Bestandteil von Aminosäuren, Zuckern, Nukleotiden; Nukleosiden, Pyrimidin- und Purinbasen sind.

6. Verfahren nach einem der vorhergehenden Ansprüche, **dadurch gekennzeichnet, dass** die mindestens zwei unterschiedlichen zur Bindung befähigten Gruppen des Substrats ausgewählt werden unter Gruppen, die Bestandteil von Aminosäuren, Zuckern, Nukleotiden, Nukleosiden, Pyrimidin- und Purinbasen sind.

7. Verfahren nach einem der Ansprüche 1 bis 6, **dadurch gekennzeichnet, dass** in Stufe (ii) die mindestens zwei unterschiedlichen zur Bindung mit dem Substrat befähigten Gruppen über ein Reagenz auf einen polymerbeschichteten Träger aufgebracht werden, das ausgewählt wird aus der Gruppe umfassend Aktivierungsreagenzien, Silanisierungsreagenzien und Spacer, oder Gemische aus zwei oder mehreren dieser Reagenzien.

8. Verfahren nach Anspruch 7, **dadurch gekennzeichnet, dass** in Stufe (ii) die mindestens zwei unterschiedlichen zur Bindung mit dem Substrat befähigten Gruppen bestehend aus Phenyl-, Hydroxyphenyl-, Carboxyl-, Amin-, Amid-, Hydroxyl-, Indol-, Imidazol- und Guanidin-Rest.

9. Verfahren nach einem der vorhergehenden Ansprüche, **dadurch gekennzeichnet, dass** es zusätzlich die Stufe (v) umfasst:

(v) Isolierung des Substrats.

10. Verfahren nach einem der vorhergehenden Ansprüche, **dadurch gekennzeichnet, dass** es zusätzlich die Stufe (vi) umfasst:

(vi) Charakterisierung und Identifizierung des Substrats.

11. Verfahren nach einem der vorhergehenden Ansprüche, **dadurch gekennzeichnet, dass** das Substrat einen oder mehrere natürliche Wirkstoffe ausgewählt aus der Gruppe umfassend Aminosäuren, Oligopeptide, Nukleotide, Proteine, Glukoproteine, Antigene, Antikörper, Kohlenhydrate, Enzyme, Co-Enzyme, Hormone, Alkaloide, Steroide, Viren, Mikroorgansimen, Inhaltsstoffe pflanzlicher und tierischer Gewebe, Zellen, Zellfragmente, Zellkompartimente, Zellaufschlüsse, Lectine, Flavyliumverbindung, Flavone und Isoflavone, oder einen oder mehrere synthetische Wirkstoffe ausgewählt aus der Gruppe von Substanzen mit Wirkung auf das Nervensystem, mit Wirkung auf das Hormonsystem, mit Wirkung auf Mediatoren, mit Wirkung auf das Herz-Kreislauf-System, mit Wirkung auf den Respirationstrakt, mit Wirkung auf den Magen-Darm-Trakt, mit Wirkung auf die Niere und ableitende Harnwege, mit Wirkung auf das Auge, mit Wirkung auf die Haut, Substanzen zur Prophylaxe und Therapie von Infektionskrankheiten, mit Wirkung auf maligne Tumore, mit Wirkung auf das Immunsystem und immunologisch wirksame Stoffe, sowie Insektizide, Herbizide, Pestizide und Fungizide umfasst.

## Claims

1. Method for the selective bivalent binding of a natural or synthetic substrate, having at least two different groups capable of binding, to a sorbent, which comprises a solid carrier and at least two different groups capable of binding to the natural or synthetic substrate, wherein the method comprises the steps (i) to (iv):

   (i) determining at least two groups capable of binding a sorbent from the natural or synthetic substrate,
   (ia) binding an underivatised polymer via non-covalent interactions to the carrier,
   (ii) respectively introducing at least two different groups capable of binding the natural or synthetic substrate via at least two identical or different functional groups of the polymer into the polymer, thereby forming at least one sorbent, whereby the groups are groups that are complementary to the groups determined in step (i), whereby a derivatised polymer having at least two different groups capable of binding is formed, and the respective at least two different groups capable of binding are covalently bonded to the polymer,
   wherein

      (a) the groups are determined such that the contributions of the Gibbs energies of the individual groups for the non-covalent bond with the substrate results in a negative value of the Gibbs energy $\Delta G$, so that a binding strengthening occurs, which results in an improved separation selectivity $\alpha$, by way of which the substrate to be selectively bound having the at least two different groups capable of binding to the at least one sorbent is separated off from a substrate mixture by way of using the at least one sorbent, of more than 1.4 and wherein

   (iii) contacting the substrate with the sorbent of step (ii) ;
   (iv) testing the binding strength of the substrate to the sorbent of step (iii).

2. Method according to claim 1, **characterized in that** the determination in step (i) comprises the dissection of the natural or synthetic substrate into at least two components having at least two groups capable of binding a sorbent.

3. Method according to claim 2, **characterized in that** one component has at least two different groups capable of binding.

4. Method according to any one of the preceding claims, **characterized in that** the selection of at least two groups capable of binding a sorbent from the natural or synthetic substrate in step (i) yields two components each having at least one group capable of binding the sorbent, and in step (ii) one sorbent is obtained; or the selection of at least two groups capable of binding a sorbent from the natural or synthetic substrate in step (i) yields three components each having at least one group capable of binding the sorbent, and in step (ii) at least three sorbents are obtained; or the selection of at least two groups capable of binding a sorbent from the natural or synthetic substrate in step (i) yields four components each having at least one group capable of binding the sorbent, and in step (ii) at least six sorbents are obtained.

5. Method according to any one of the preceding claims, **characterized in that** the at least two different groups capable of binding of the at least one sorbent are selected among groups which are part of amino acids, sugars, nucleotides, nucleosides, pyrimidine bases and purine bases.

6. Method according to any one of the preceding claims, **characterized in that** the at least two different groups capable of binding of the substrate are selected among groups which are part of amino acids, sugars, nucleotides, nucleosides, pyrimidine bases and purine bases.

7. Method according to any one of claims 1 to 6, **characterized in that** in step (ii) the at least two different groups capable of binding the substrate are applied onto a polymer-coated carrier via a reagent which is selected from the group comprising activating reagents, silanization reagents and spacer, or mixtures of two or more of said reagents.

8. Method according to claim 7, **characterized in that** in step (ii) the at least two different groups capable of binding the substrate consist of phenyl, hydroxyphenyl, carboxyl, amine, amide, hydroxyl, indole, imidazole and guanidine residue.

9. Method according to any one of the preceding claims, **characterized in that** it additionally comprises the step (v):

(v) isolating the substrate.

10. Method according to any one of the preceding claims, **characterized in that** it additionally comprises the step (vi) :

(vi) characterizing and identifying the substrate.

11. Method according to any one of the preceding claims, **characterized in that** the substrate comprises one or more natural agents selected from the group comprising amino acids, oligopeptides, nucleotides, proteins, glycoproteins, antigens, antibodies, carbohydrates, enzymes, co-enzymes, hormones, alkaloids, steroids, viruses, microorganisms, substances contained in vegetable and animal tissue, cells, cell fragments, cell compartments, cell disruptions, lectins, flavylium compounds, flavones, and isoflavones, or one or more synthetic agents selected from the group of substances having influence on the nervous system, having influence on the hormone system, having influence on mediators, having influence on the cardio-vascular system, having influence on the respiratory tract, having influence on the gastrointestinal tract, having influence on the kidney and the lower urinary tract, having influence on the eye, having influence on the skin, substances for the prophylaxis and therapy of infection diseases, having influence on malignant tumors, having influence on the immune system and substances having immunological influence, as well as insecticides, herbicides, pesticides and fungicides.

**Revendications**

1. Procédé de liaison sélective bivalente d'un substrat synthétique ou naturel, avec au moins deux groupes différents capables de liaison, à un sorbant, lequel comprend au moins un support fixe et au moins deux groupes différents capables de se lier au substrat synthétique ou naturel, ou le procédé comprend les étapes (i) à (iv):

(i) détermination d'au moins deux groupes capables de liaison à un sorbant à partir du substrat synthétique ou naturel,
(ii) liaison d'un polymère non dérivé par moyen d'interactions non covalentes au support,
(iii) application respectivement d'au moins deux groupes différents capables de liaison au substrat naturel ou synthétique par au moins deux groupes fonctionnels différents ou égaux du polymère dans le polymère de manière à former au moins un sorbant, dans lequel les groupes sont complémentaires aux groupes déterminés dans l'étape (i), dans lequel un polymère dérivé se forme avec au moins deux groupes capables de liaison, et les au moins deux groupes respectifs différents capables de liaison sont reliés de façon covalente au polymère, dans lequel

(a) les groupes sont déterminés de telle sorte que les contributions des énergies de Gibbs des groupes individuels pour la liaison non-covalente avec le substrat donnent une valeur négative de l'énergie de Gibbs $\Delta G$, de sorte qu'un renforcement de liaison se produit, qui résulte dans une sélectivité de séparation $\alpha$ améliorée plus grand que 1,4, avec laquelle le substrat à lier sélectivement avec les au moins deux groupes différents capables de liaison avec le au moins un sorbant est séparé d'un mélange de substrat par application d'au moins un sorbant

et dans lequel
(iii) mise en contact du substrat avec le sorbant de l'étape (ii);

(iv) épreuve de la force de liaison du substrat au sorbant de l'étape (iii).

2. Procédé selon la revendication 1, **caractérisé en ce que** la détermination dans l'étape (i) comprend la séparation du substrat naturel ou synthétique dans au moins deux composants avec au moins deux groupes capables de liaison avec un sorbant.

3. Procédé selon la revendication 2, **caractérisé en ce qu'**un composant comprend au moins deux groupes différents capables de liaison.

4. Procédé selon l'une des revendications précédentes, **caractérisé en ce que** la sélection d'au moins deux groupes capables de liaison avec un sorbant sur le substrat synthétique ou naturel dans l'étape (i) mène à deux composants ayant chacun au moins un group capable de liaison avec le sorbant, et dans l'étape (ii) un sorbant est obtenu; ou la sélection d'au moins deux groupes capables de liaison avec un sorbant sur le substrat synthétique ou naturel dans l'étape (i) mène à trois composants ayant chacun au moins un group capable de liaison avec le sorbant, et dans l'étape (ii) aux moins trois sorbants sont obtenus; ou la sélection d'au moins deux groupes capables de liaison avec un sorbant sur le substrat synthétique ou naturel dans l'étape (i) mène à quatre composants ayant chacun au moins un group capable de liaison avec le sorbant, et dans l'étape (ii) aux moins six sorbants sont obtenus;

5. Procédé selon l'une des revendications précédentes, **caractérisé en ce que** les au moins deux groupes différents capables de liaison du au moins un sorbant sont choisis parmi les groupes qui sont des composants d'acides aminés, sucres, nucléotides; nucléosides, bases pyrimidine et bases purine.

6. Procédé selon l'une des revendications précédentes, **caractérisé en ce que** les au moins deux groupes différents capables de liaison du substrat sont choisis parmi les groupes qui sont des composants d'aminoacides, sucres, nucléotides; nucléosides, bases pyrimidine et bases purine.

7. Procédé selon l'une des revendications 1 à 6, **caractérisé en ce que** dans l'étape (ii) le au moins deux groupes différents capables de liaison avec le substrat par l'intermédiaire d'un groupe réactif sont appliquées sur un support couvert par polymère, qui est choisi dans le groupe comprenant réactifs d'activation, réactifs de silanisation et spacers, ou des mélanges de deux ou plusieurs de ces réactifs.

8. Procédé selon la revendication 7, **caractérisé en ce que** dans l'étape (ii) au moins deux groupes différents capable de liaison avec le substrat sont composés de résidu de phényle, hydroxyphényle, carboxyle, amine, amide, hydroxyle, indole, imidazole et guanidine.

9. Procédé selon l'une des revendications précédentes, **caractérisé en ce qu'**il comprend en outre l'étape (v):

(v) l'isolation du substrat.

10. Procédé selon l'une des revendications précédentes, **caractérisé en ce qu'**il comprend en outre l'étape (vi):

(vi) la caractérisation et l'identification du substrat.

11. Procédé selon l'une quelconque des revendications précédentes, **caractérisé en ce que** le substrat comprend une ou plusieurs substances actives naturelles choisis parmi le groupe comprennent aminoacides, oligopeptides, nucléotides, protéines, glycoprotéines, antigènes, anticorps, carbohydrates, enzymes, coenzymes, hormones, alcaloïdes, stéroïdes, virus, micro-organismes, ingrédients de tissus végétaux et animaux, cellules, fragments cellulaires, compartiments cellulaires, fractions cellulaires, lectines, liaison de flavylium, flavones et isoflavones, ou une ou plusieurs substances actives synthétiques choisies dans le groupe des substances agissant sur le système nerveux, avec des effets sur le système endocrinien, avec effet sur les médiateurs, agissant sur le système cardio-vasculaire, avec des effets sur les voies respiratoires, avec effet sur le tractus gastro-intestinal, avec effet sur le rein et des voies urinaires, avec effet sur l'oeil, avec effet sur la peau, des agents pour la prophylaxie et le traitement des infections, avec effet sur les tumeurs malignes, avec effet sur le système immunitaire et les substances immunologiquement actives, ainsi que des insecticides, herbicides, pesticides et fongicides.

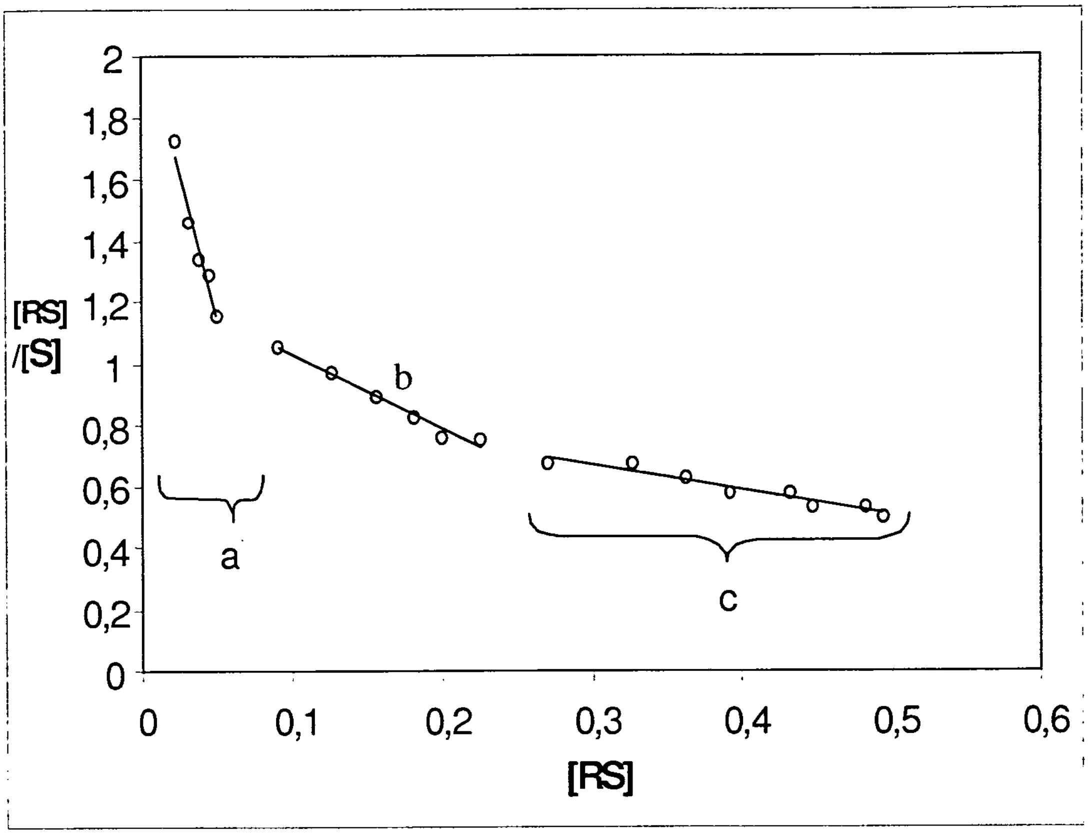


Abb. 1

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 0032649 A
- WO 0032648 A
- WO 0138009 A
- WO 0078825 A
- WO 0013016 A
- WO 9319844 A
- US 20020155509 A1

### In der Beschreibung aufgeführte Nicht-Patentliteratur

- **M. WHITESIDES et al.** *Angew. Chem.,* 1998, vol. 110, 2908-2953
- Column Watch. *LC*GC Europe,* Dezember 2002, 780-786